# EUROPEAN PATENT APPLICATION

(11) **EP 4 361 272 A1**
(43) Date of publication of application: **01.05.2024**
(21) Application number: 22828398.2
(22) Date of filing: 20.06.2022
(51) Int. Cl.: C12N 15/12, C07K 14/705, C12N 1/15, C12N 1/19, C12N 1/21, C12N 5/10, C12N 15/09, C12N 15/63, C12Q 1/02

(54) **OLFACTORY RECEPTOR EXPRESSING METHOD AND RESPONSE MEASUREMENT METHOD**

(30) Priority: 22.06.2021 JP 2021103675; 15.02.2022 JP 2022021606
(71) Applicant: Kao Corporation, Tokyo 103-8210 (JP)
(72) Inventor: YOSHIKAWA, Keiichi, Haga-gun, Tochigi 321-3497 (JP)
(74) Representative: Vossius & Partner Patentanwälte Rechtsanwälte mbB
(86) International application number: PCT/JP2022/024613
(87) International publication number: WO 2022/270481

(57) **Abstract**

Provided is an approach which can express an olfactory receptor and function. The present invention provides a method for expressing an olfactory receptor polypeptide, comprising expressing, in a cell, an olfactory receptor polypeptide consisting of an amino acid sequence obtained by, in an amino acid sequence of an olfactory receptor of interest, altering at least one amino acid residue different from that in a consensus amino acid sequence to the amino acid residue at a position corresponding thereto in the consensus amino acid sequence, wherein the consensus amino acid sequence is an amino acid sequence obtained by alignment of the amino acid sequence of the olfactory receptor of interest and amino acid sequences of particular olfactory receptors.

## Description

### Field of the Invention

The present invention relates to an olfactory receptor expressing method and a response measurement method.

### Background of the Invention

Odor of candidate substance has heretofore been evaluated in accordance with sensory tests by specialists in fragrance development for favorable aroma or bad odor elimination. However, problems of the sensory tests are large variations and low throughputs, for example, or necessary training of specialists who can properly evaluate odor. Accordingly, fragrance development methods using olfactory receptors as an index serving as an objective index which well reflects a human sense of smell have been reported in recent years.

In mammals such as humans, odorants are recognized by olfactory receptors on olfactory neurons which reside in the olfactory epithelium spreading over the deepest part in the nasal cavity. Odor molecules taken up into the nasal cavity act on and activate olfactory receptors. Signals from olfactory neurons caused by the activated olfactory receptors are transduced to the central nervous system so that odor is perceived. In a report of 2014, 396 functional genes were predicted as genes encoding human olfactory receptors (Non Patent Literature 1). The quality of odor perceived by us from a particular odorant is presumably determined depending on which combination of the approximately 400 olfactory receptors is activated by the odorant.

A method is disclosed which can acquire more favorable aroma by identifying an olfactory receptor which selectively responds to particular aroma among the olfactory receptors, and designing a fragrance using the receptor as an index (Patent Literature 1). Meanwhile, provided that an olfactory receptor which recognizes bad odor is identified, a fragrance working as a receptor antagonist which suppresses the bad odor recognition of the olfactory receptor can be used as a fragrance effective for odor elimination (Patent Literature 2). According to disclosure, use of a fragrance which activates an olfactory receptor important for bad odor recognition but presents no bad odor can suppress the bad odor recognition through cross-adaptation, thereby solving bad odor problems (Patent Literature 3). The identification of such olfactory receptors that recognize odorants enables industrially useful approaches of developing preferred fragrances with high efficiency.

Since responses of many olfactory receptors are selective for odorants, it is vital to first find an olfactory receptor having selectivity for target odor. However, unfortunately, it has not been easy so far to find an olfactory receptor sensitive to particular odor. An existing receptor analysis method merely succeeds in functional analysis on only about 12% of all human receptors (Non Patent Literature 2). This situation means that, for example, an approach using an antagonist fragrance which eliminates particular bad odor can merely identify and control only a limited number of olfactory receptors among the olfactory receptors involved in the bad odor recognition.

This problem has been discussed for nearly 30 years since 1991 when olfactory receptor genes were discovered. Even if cultured cells express an olfactory receptor of interest to be analyzed, the olfactory receptor protein, albeit synthesized, remains inside the cells without being translocated to the cell surface. Hence, an odorant of interest, even when administered from outside the cells, does not reach the receptor, so that the binding therebetween cannot be tested. This is presumably because olfactory neurons of the nose have molecules essential for the translocation of olfactory receptors to the cell surface (membrane expression) whereas the molecules are absent in cultured cells derived from cells other than the olfactory neurons of the nose. An RTP (receptor-transporting protein) was identified as one of such essential molecules in 2004, and the number of olfactory receptors possible to be analyzed has reached the current one (Non Patent Literature 3). However, the identification of a potential essential molecule other than RTP has been frustrated. Furthermore, cultured cells derived from olfactory neurons have not yet been established. Therefore, the analysis and functional understanding of olfactory receptors are behind the curve, and a success rate remains at only about 12% even after nearly 30 years.

In the meantime, mouse olfactory receptors which are expressed on the cell surface and mouse olfactory receptors which are not expressed thereon have been comparatively analyzed (Non Patent Literature 4). As a result, it has been found that olfactory receptors which are not membrane-expressed might have low conformational stability. Importantly, the result has shown that the amino acid properties are statistically significantly different at some sites in primary amino acid sequences of proteins between olfactory receptors capable of membrane expression and olfactory receptors incapable thereof; and the amino acids at the sites have high commonality in all of approximately 1,000 mouse olfactory receptors. This has specifically suggested that olfactory receptors which are not membrane-expressed, might cause a mutation at a position where a common amino acid is supposed to be used in a polypeptide to thereby result in a different amino acid, and thus lack conformational stability, so that the judgement of no translocation to the cell membranes is made in cultured cells. In light of this suggestion, a "consensus design technique" of introducing an amino acid having high commonality among olfactory receptors is considered to enable acquirement of an olfactory receptor of interest as a stable protein or to be efficiently expressed on the cell membranes.

Under this concept, Patent Literature 4 discloses a method for efficiently analyzing human olfactory receptors. Approximately 400 human olfactory receptors are phylogenetically classified into 18 groups. As for each group, the analysis employs one type of consensus olfactory receptor designed by adopting an amino acid sequence having the highest commonality (paralog consensus) in an olfactory receptor group constituting the group. However, this method cannot evaluate the respective functions of approximately 400 olfactory receptors of humans. Meanwhile, in Non Patent Literature 2, in order to understand functions of a particular human olfactory receptor, the consensus design of the receptor has been performed by using commonality among evolutionary homologous genes, not commonality in the group, as an index. Specifically, as for three types of human olfactory receptors, OR6Y1, OR6B2, and OR56A4, consensus olfactory receptors OR6Y1, OR6B2, and OR56A4 have been prepared by introducing an amino acid having high commonality among homologous genes of 10 types of mammals (gorillas, bonobos, chimpanzees, Sumatran orangutans, rhesus macaques, drills, common marmosets, gray mouse lemurs, rats, and mice) into each human olfactory receptor. As a result, among the three olfactory receptors, OR6Y1 has attained response measurement to an odorant using cultured cells according to the disclosure. This suggests that response analysis is attained at a probability of 1/3 by introducing a common amino acid among evolutionary homologous genes in order to analyze a particular human olfactory receptor.

Nonetheless, even if this method is applied, human olfactory receptors which can be analyzed presumably remain at about 1/3 of approximately 400 types. This low efficiency is also supported by previous findings from studies. The consensus design method has been carried out from long ago in order to design industrially useful enzymes as more stable one. However, Non Patent Literature 5, which gives a review regarding this technique, points out that the probability of obtaining an improving effect by introducing consensus design to an amino acid sequence at a location is about 50% whereas the remaining 40% has the risk of changing a protein for the worse; thus the consensus design method is difficult to exploit. There exists an idea that the application of the consensus design method is not proper for research aimed at predicting inherent functions of olfactory receptors. Specifically, an approach of introducing amino acid substitution might change a ligand binding site of the original olfactory receptor and consequently cause inherent odor responsiveness to be no longer observed. Thus, the consensus design is expected to have two risks, a low success rate and change in inherent functions of an olfactory receptor of interest, and therefore, has not yet been tested for its effectiveness for a wide range of olfactory receptors in any case.

(Patent Literature 1) JP-A-2018-074944
(Patent Literature 2) JP-A-2020-10629
(Patent Literature 3) JP-A-2016-224039
(Patent Literature 4) WO 2020/006108
(Non Patent Literature 1) Niimura Y et al., Genome Research 24, 1485-1496 (2014)
(Non Patent Literature 2) Trimmer C et al., PNAS 116: 9475-9480 (2019)
(Non Patent Literature 3) Saitou H et al., Cell. 119: 679-91 (2004)
(Non Patent Literature 4) Ikegami K et al., PNAS 117: 2957-2967 (2020)
(Non Patent Literature 5) Porebski TB et al., Protein Engineering, Design & Selection 29: 245-251 (2016)

### Summary of the Invention

The present invention provides the following 1) to 12) .
1) A method for expressing an olfactory receptor polypeptide, comprising
   expressing, in a cell, an olfactory receptor polypeptide consisting of an amino acid sequence obtained by, in an amino acid sequence of an olfactory receptor of interest, altering at least one amino acid residue different from that in a consensus amino acid sequence to an amino acid residue at a position corresponding thereto in the consensus amino acid sequence, wherein
   the consensus amino acid sequence is an amino acid sequence derived by alignment of the amino acid sequence of the olfactory receptor of interest and amino acid sequences of any of the following olfactory receptors (a) to (d):
      (a) at least 11 olfactory receptors selected from the group consisting of olfactory receptors encoded by orthologs of the olfactory receptor of interest in the same order as that of an organism species from which the olfactory receptor of interest is derived;
      (b) at least 11 olfactory receptors selected from the group consisting of the olfactory receptors encoded by orthologs of the olfactory receptor of interest in the same order as that of an organism species from which the olfactory receptor of interest is derived and olfactory receptors encoded by paralogs of the olfactory receptor of interest, the 11 olfactory receptors including at least one of the olfactory receptors encoded by the paralogs of the olfactory receptor of interest;
      (c) at least 11 olfactory receptors selected from the group consisting of olfactory receptors encoded by orthologs of the olfactory receptor of interest in vertebrates, the 11 olfactory receptors including at least one olfactory receptor encoded by a vertebrate ortholog in an order different from the order of an organism species from which the olfactory receptor of interest is derived; and
      (d) at least 11 olfactory receptors selected from the group consisting of olfactory receptors encoded by orthologs of the olfactory receptor of interest in vertebrates and olfactory receptors encoded by orthologs in a vertebrate with a paralog having the highest homology to the olfactory receptor of interest among paralogs of the olfactory receptor of interest having 11 or more vertebrate orthologs, the 11 olfactory receptors including at least one olfactory receptor encoded by a vertebrate ortholog in an order different from the order of an organism species from which the olfactory receptor of interest is derived, and
         an olfactory receptor encoded by the paralog.
2) A method for expressing an olfactory receptor polypeptide, comprising
   expressing, in a cell, an olfactory receptor polypeptide consisting of an amino acid sequence obtained by, in an amino acid sequence of an olfactory receptor of interest, altering at least one amino acid residue different from that in a consensus amino acid sequence to an amino acid residue at a position corresponding thereto in the consensus amino acid sequence, wherein
   the olfactory receptor polypeptide consists of an amino acid sequence obtained by, in the amino acid sequence of SEQ ID NO: 96 of human olfactory receptor OR7E24, altering at least one amino acid residue different from that in the consensus amino acid sequence of SEQ ID NO: 210 to an amino acid residue at a position corresponding thereto in the consensus amino acid sequence,
   the olfactory receptor polypeptide consists of an amino acid sequence obtained by, in the amino acid sequence of SEQ ID NO: 484 of human olfactory receptor OR9K2, altering at least one amino acid residue different from that in the consensus amino acid sequence of SEQ ID NO: 642 to an amino acid residue at a position corresponding thereto in the consensus amino acid sequence, or
   the olfactory receptor polypeptide consists of an amino acid sequence obtained by, in the amino acid sequence of SEQ ID NO: 850 of human olfactory receptor OR5I1, altering at least one amino acid residue different from that in the consensus amino acid sequence of SEQ ID NO: 900 to an amino acid residue at a position corresponding thereto in the consensus amino acid sequence.
3) A method for measuring a response of an olfactory receptor of interest, comprising
   measuring a response of an olfactory receptor polypeptide expressed by the method according to 1) or 2) .
4) A method for searching for a ligand for an olfactory receptor of interest, comprising:
   measuring a response of an olfactory receptor polypeptide expressed by the method according to 1) or 2) in the presence of a test substance; and
   selecting a test substance to which the olfactory receptor polypeptide has responded.
5) A method for evaluating and/or selecting a suppressor of odor of a ligand for an olfactory receptor of interest, comprising:
   adding a test substance and the ligand for an olfactory receptor of interest to an olfactory receptor polypeptide expressed by the method according to 1) or 2); and
   measuring a response of the olfactory receptor polypeptide to the ligand.
6) A method for evaluating and/or selecting a suppressor of odor of a ligand for an olfactory receptor of interest, comprising:
   adding a test substance to an olfactory receptor polypeptide expressed by the method according to 1) or 2); and
   measuring a response of the olfactory receptor polypeptide to the test substance.
7) A method for evaluating and/or selecting an enhancer of odor of a ligand for an olfactory receptor of interest, comprising:
   adding a test substance and the ligand for an olfactory receptor of interest to an olfactory receptor polypeptide expressed by the method according to 1) or 2); and
   measuring a response of the olfactory receptor polypeptide to the ligand.
8) An altered olfactory receptor polypeptide consisting of
   an amino acid sequence obtained by, in an amino acid sequence of an olfactory receptor of interest, altering at least one amino acid residue different from that in a consensus amino acid sequence to an amino acid residue at a position corresponding thereto in the consensus amino acid sequence, wherein
   the consensus amino acid sequence is an amino acid sequence obtained by alignment of the amino acid sequence of the olfactory receptor of interest and amino acid sequences of any of the following olfactory receptors (a) to (d):
      (a) at least 11 olfactory receptors selected from the group consisting of olfactory receptors encoded by orthologs of the olfactory receptor of interest in the same order as that of an organism species from which the olfactory receptor of interest is derived;
      (b) at least 11 olfactory receptors selected from the group consisting of the olfactory receptors encoded by orthologs of the olfactory receptor of interest in the same order as that of an organism species from which the olfactory receptor of interest is derived and olfactory receptors encoded by paralogs of the olfactory receptor of interest, the 11 olfactory receptors including at least one of the olfactory receptors encoded by the paralogs of the olfactory receptor of interest;
      (c) at least 11 olfactory receptors selected from the group consisting of olfactory receptors encoded by orthologs of the olfactory receptor of interest in vertebrates, the 11 olfactory receptors including at least one olfactory receptor encoded by a vertebrate ortholog in an order different from the order of an organism species from which the olfactory receptor of interest is derived; and
      (d) at least 11 olfactory receptors selected from the group consisting of olfactory receptors encoded by orthologs of the olfactory receptor of interest in vertebrates and olfactory receptors encoded by orthologs in a vertebrate with a paralog having the highest homology to the olfactory receptor of interest among paralogs of the olfactory receptor of interest having 11 or more vertebrate orthologs, the 11 olfactory receptors including at least one olfactory receptor encoded by a vertebrate ortholog in an order different from the order of an organism species from which the olfactory receptor of interest is derived, and
   an olfactory receptor encoded by the paralog.
9) An altered olfactory receptor polypeptide
   consisting of an amino acid sequence obtained by, in amino acid sequence of SEQ ID NO: 96 of human olfactory receptor OR7E24, altering at least one amino acid residue different from that in the consensus amino acid sequence of SEQ ID NO: 210 to an amino acid residue at a position corresponding thereto in the consensus amino acid sequence,
   consisting of an amino acid sequence obtained by, in the amino acid sequence of SEQ ID NO: 484 of human olfactory receptor OR9K2, altering at least one amino acid residue different from that in the consensus amino acid sequence of SEQ ID NO: 642 to an amino acid residue at a position corresponding thereto in the consensus amino acid sequence, or
   consisting of an amino acid sequence obtained by, in the amino acid sequence of SEQ ID NO: 850 of human olfactory receptor OR5I1, altering at least one amino acid residue different from that the consensus amino acid sequence of SEQ ID NO: 900 to an amino acid residue at a position corresponding thereto in the consensus amino acid sequence.
10) A polynucleotide encoding the altered olfactory receptor polypeptide according to 8) or 9).
11) A vector or a DNA fragment comprising the polynucleotide according to 10).
12) A transformed cell comprising the vector or the DNA fragment according to 11).

### Brief Description of Drawings

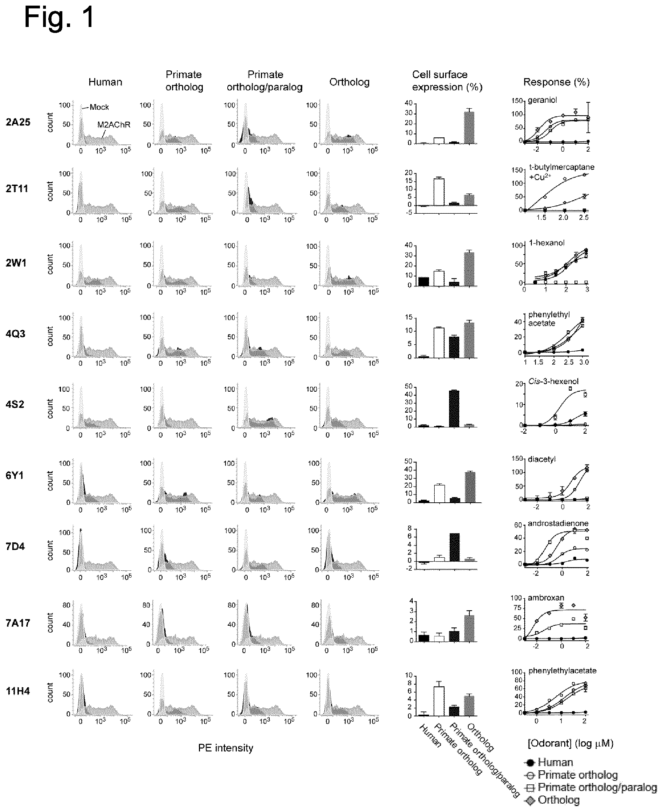

[Figure 1] Figure 1 shows a cell membrane expression level and odor responsiveness of each designed consensus olfactory receptor. Four histograms and one bar graph from the left depict a receptor protein level on HEK293 cell membranes determined by flow cytometry. Cells expressing no receptor (Mock) and cells expressing a receptor M2AcR with efficient membrane expression were analyzed as controls. Results of measuring ligand responsiveness of each receptor by luciferase assay are shown on the right. The error bar represents SEM (n = 3) .

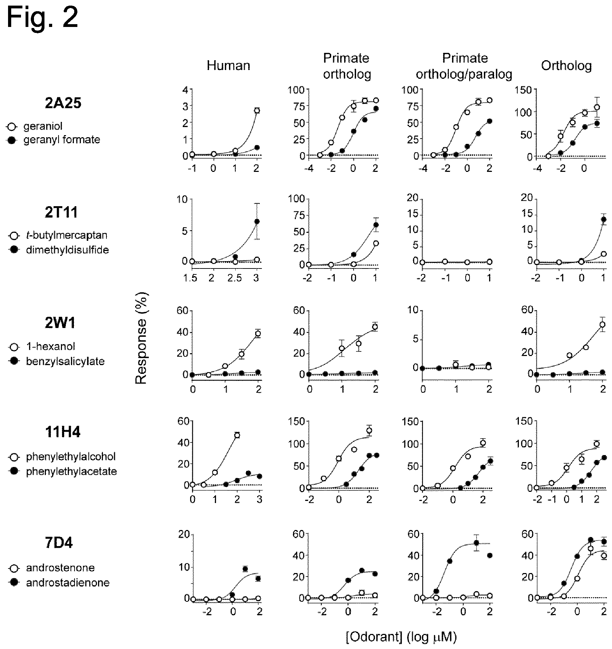

[Figure 2] Figure 2 shows ligand selectivity of each designed consensus olfactory receptor. Results of luciferase assay are shown. The error bar represents SEM (n = 3) .

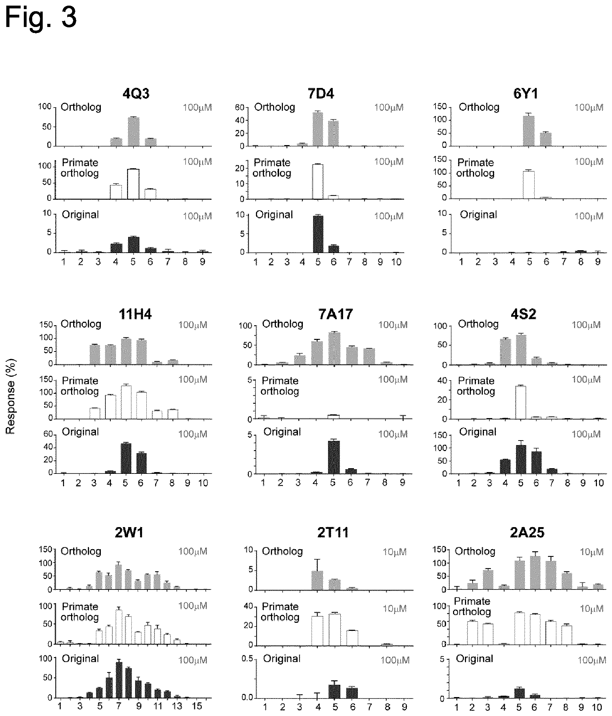

[Figure 3] Figure 3 shows ligand selectivity of each designed consensus olfactory receptor. Results of luciferase assay are shown. The error bar represents SEM (n = 3). The numbers shown in the lower part respectively correspond to odorant numbers shown in Table 8 given below.

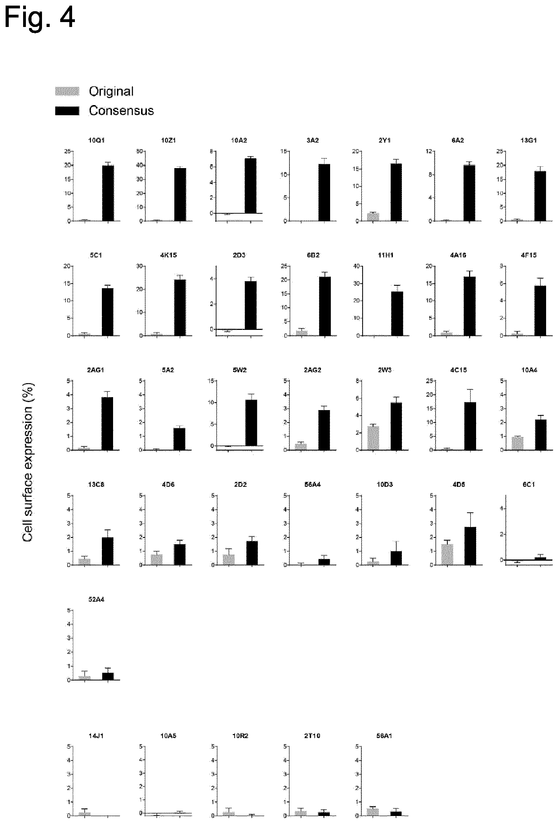

[Figure 4] Figure 4 shows a cell membrane expression level of each designed consensus olfactory receptor. Each graph depicts a receptor protein level on HEK293 cell membranes determined by flow cytometry. The comparison between the original human olfactory receptor and a receptor to which mammalian consensus design was applied is illustrated as to each of 34 receptors. The error bar represents SEM (n = 3 or 4).

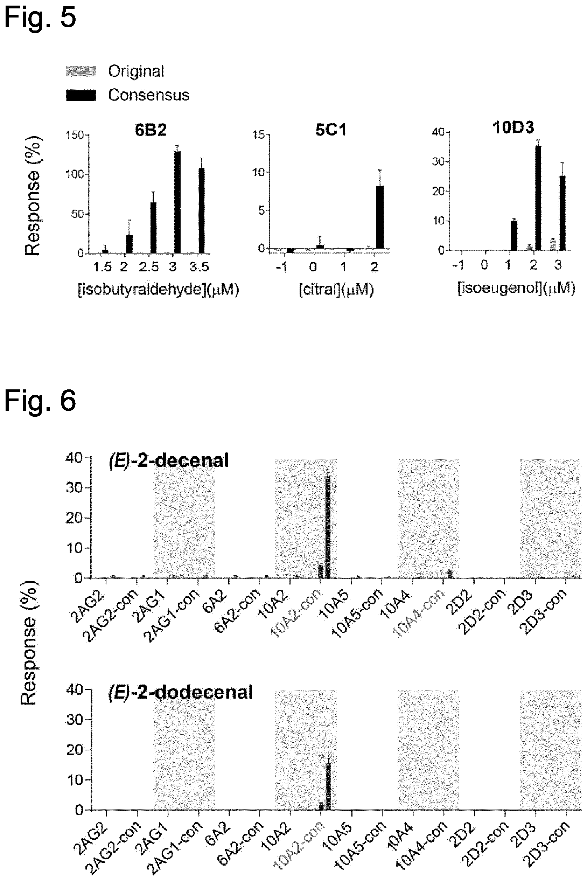

[Figure 5] Figure 5 shows luciferase assay of each olfactory receptor. Varied concentrations of odorants were administered to 3 types of human olfactory receptors and their consensus olfactory receptors, and responses were measured. The error bar represents SEM (n = 3).
[Figure 6] Figure 6 shows luciferase assay of each olfactory receptor. Varied concentrations of odorants were administered to 8 types of human olfactory receptors and their consensus olfactory receptors, and responses were measured. The error bar represents SEM (n = 3).

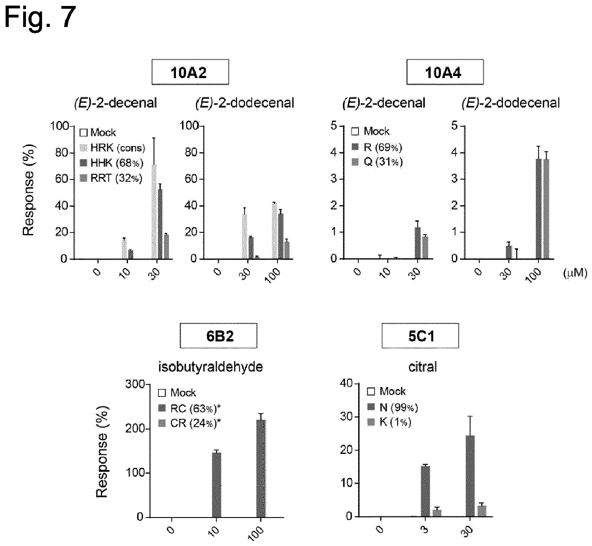

[Figure 7] Figure 7 shows luciferase assay of each olfactory receptor. In HEK293 cells, each of 4 types of consensus olfactory receptors harboring amino acid substitution reported to cause difference among individuals was expressed. Varied concentrations of odorants were administered thereto, and responses were measured. The error bar represents SEM (n = 3) .

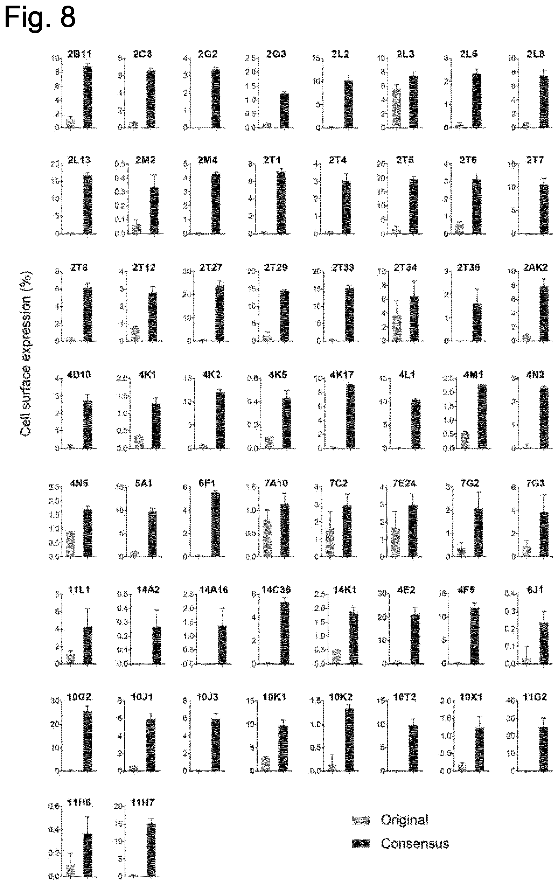

[Figure 8] Figure 8 shows a cell membrane expression level of each designed consensus olfactory receptor. Each graph depicts a receptor protein level on HEK293 cell membranes determined by flow cytometry. The comparison between the original human olfactory receptor and a receptor to which mammalian consensus design was applied is illustrated as to each receptor. The error bar represents SEM (n = 3).

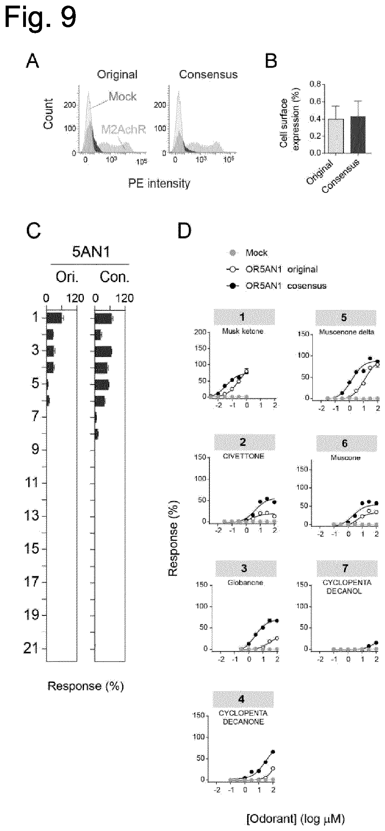

[Figure 9] Figures 9A and 9B show cell membrane expression levels of OR5AN1 and consensus OR5AN1. Figure 9A is a histogram depicting the distribution of PE fluorescent signals of a cell population expressing each receptor. Cells expressing no receptor (Mock) and cells expressing a receptor M2AchR with efficient membrane expression were analyzed as controls. Figure 9B shows results of quantifying the membrane expression levels in Figure 9A. The error bar represents SEM (n = 3). Figure 9C shows responses of OR5AN1 (Ori.) and consensus OR5AN1 (Con.) to various odorants (100 µM). The error bar represents SEM (n = 3). The numbers on the ordinate correspond to odorant numbers shown in Table 19 given below. Figure 9D shows dose dependence for 7 odorants. A mean and SEM from three replicas from one experiment are shown.

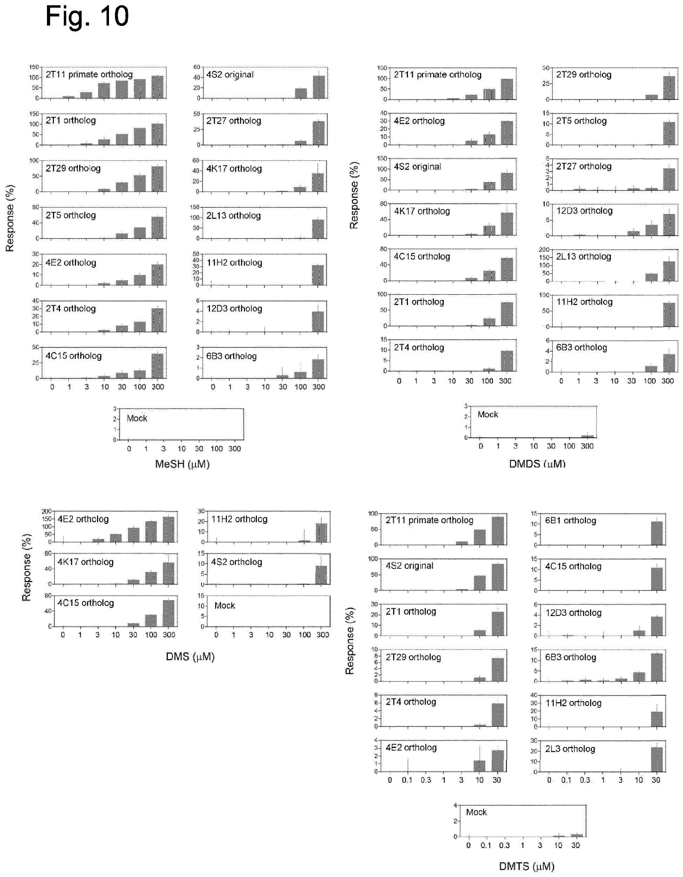

[Figure 10] Figure 10 shows luciferase assay of each olfactory receptor. Each bar graph depicts response intensity when varied concentrations of methyl mercaptan (MeSH), dimethyl sulfide (DMS), dimethyl disulfide (DMDS), or dimethyl trisulfide (DMTS) were administered to each consensus olfactory receptor and the original olfactory receptor. The error bar represents SEM (from three replicas in one experiment).

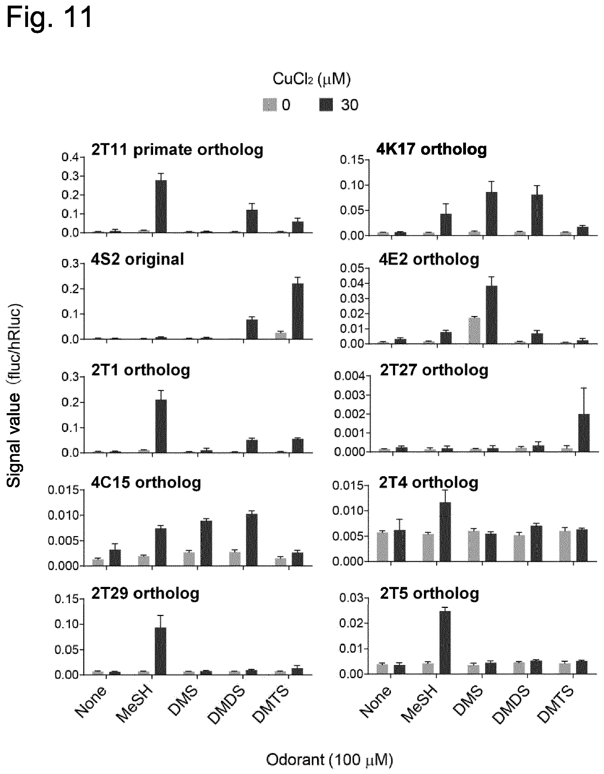

[Figure 11] Figure 11 shows luciferase assay of each olfactory receptor. Each bar graph depicts a signal value when 100 µM MeSH, DMS, DMDS, or DMTS was administered to each consensus olfactory receptor and the original olfactory receptor in the presence or absence of a copper ion. The error bar represents SEM (from three replicas in one experiment).

### Detailed Description of the Invention

In the present specification, the "olfactory receptor polypeptide" refers to an olfactory receptor or a polypeptide functionally equivalent thereto. The polypeptide functionally equivalent to the olfactory receptor refers to a polypeptide which can be expressed on cell membranes, as in the olfactory receptor, is activated through the binding of an odor molecule, and when activated, coupled to Gαs in cells to activate adenylate cyclase, thereby having a function of increasing intracellular cAMP levels (Nat. Neurosci., 2004, 5: 263-278).

In the present specification, the term "functionally expressing" the olfactory receptor polypeptide in cells means that the expressed olfactory receptor polypeptide functions as an odorant receptor in the cells.

In the present specification, the "agonist" refers to a substance which binds to and activates a receptor. In the present specification, the "antagonist" refers to a substance which binds to a receptor but does not activate the receptor, or suppresses a response of the receptor to an agonist.

In the present specification, the "olfactory receptor agonism" refers to binding to a receptor to activate the receptor.

In the present specification, the "odor cross-adaptation (or olfactory cross-adaptation)" regarding target odor refers to a phenomenon in which olfactory sensitivity to a causative substance of target odor is reduced or changed by receiving in advance odor of a substance different from the causative substance of the target odor and acclimatizing to the odor. The present inventor and so on previously revealed that the "odor cross-adaptation" is a phenomenon based on olfactory receptor agonism (WO 2016/194788). Specifically, in the "odor cross-adaptation", an olfactory receptor for a causative substance of target odor responds to a causative substance of different odor prior to the responding to the causative substance of the target odor, and is subsequently desensitized so that the olfactory receptor merely low responds to the causative substance of the target odor even when later exposed thereto, resulting in reduction in the intensity or degeneration of the target odor to be recognized by individuals. In the present specification, the mechanism of the odor cross-adaptation caused by such a behavior of an olfactory receptor is also referred to as the "odor cross-adaptation ascribable to olfactory receptor agonism".

In the present specification, the "suppression ascribable to olfactory receptor antagonism" regarding target odor means that an antagonist suppresses a response of an olfactory receptor to a substance having the target odor, resulting in the suppression of the target odor to be recognized by individuals.

In the present specification, the identity between nucleotide sequences or amino acid sequences is calculated by the Lipman-Pearson method (Science, 1985, 227: 1435-41). Specifically, the identity is calculated by conducting analysis using homology analysis (Search Homology) program of genetic information processing software Genetyx-Win (Ver. 5.1.1; Software Development K.K.) with Unit size to compare (ktup) set to 2.

In the present specification, the "amino acid residue" means any of 20 amino acid residues constituting a protein: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V).

In the present specification, the alteration of an amino acid is also represented by [original amino acid, position, altered amino acid] in accordance with the authorized single-letter amino acid abbreviation of IUPAC. For example, the alteration of histidine at position 43 to arginine is represented by "H43R".

In the present specification, the "position corresponding" on an amino acid sequence can be determined by arranging (aligning) a sequence of interest and a reference sequence (in the present invention, an amino acid sequence of the original olfactory receptor) so as to give the largest homology. The alignment of amino acid sequences can be carried out using an algorithm known in the art, and procedures thereof are known to those skilled in the art. The alignment can be performed, for example, by using Clustal W multiple alignment program (Thompson, J.D. et al., 1994, Nucleic Acids Res. 22: 4673-4680) at default setting. Alternatively, Clustal W2 or Clustal omega, a revised version of Clustal W, may be used. Clustal W, Clustal W2, and Clustal omega are available, for example, on the website of Clustal run by University College Dublin [www.clustal.org] or European Bioinformatics Institute (EBI) [www.ebi.ac.uk/index.html] or the website of DNA Data Bank of Japan (DDBJ) run by National Institute of Genetics [www.ddbj.nig.ac.jp/searches-j.html]. A position in the sequence of interest aligned with an arbitrary position in the reference sequence by the alignment mentioned above is regarded as the "position corresponding" to the arbitrary position.

Those skilled in the art can finely adjust and optimize the alignment of the amino acid sequences thus obtained. Such optimized alignment is preferably determined in consideration of similarity between the amino acid sequences, the frequency of a gap to be inserted, and the like. In this context, the similarity between the amino acid sequences refers to, when the two amino acid sequences are aligned, the ratio (%) of the number of positions where the same or similar amino acid residues exist in both the sequences relative to the number of full-length amino acid residues. The similar amino acid residues mean amino acid residues having properties similar to each other in terms of polarity or charge so as to cause so-called conservative substitution, among 20 amino acids constituting a protein. Groups of such similar amino acid residues are well known to those skilled in the art. Examples thereof include, but are not limited to, arginine and lysine or glutamine; glutamic acid and aspartic acid or glutamine; serine and threonine or alanine; glutamine and asparagine or arginine; and leucine and isoleucine.

In addition, the alignment of the amino acid sequences thus obtained can be finely adjusted and optimized, for example, with reference to highly conserved amino acids or amino acid motifs among olfactory receptors. Examples of the highly conserved amino acids among olfactory receptors which can be used for this purpose include the most highly conservative amino acids among amino acids constituting transmembrane regions in each olfactory receptor. An additional example thereof includes cysteine which forms an intramolecular disulfide bond. Examples of the highly conserved amino acid motifs among olfactory receptors include LHTPMY positioned in the first intracellular loop, MAYDRYVAIC positioned from the latter half of the third transmembrane region to the former half of the second intracellular loop, SY positioned in the latter half of the fifth transmembrane region, KAFSTCASH positioned in the former half of the sixth transmembrane region, and PMLNPFIY positioned in the seventh transmembrane region.

In the present specification, the "operable linkage" of a control region such as a promoter to a gene refers to the linkage of the gene and the control region such that the gene can be expressed under the control of the control region. Procedures of the "operable linkage" of the gene and the control region are well known to those skilled in the art.

In the present specification, the terms "upstream" and "downstream" regarding a gene refer to upstream and downstream in the transcriptional directions of the gene. For example, the term "gene located downstream of a promoter" means that the gene resides on a 3' side of the promoter in a DNA sense strand, and the term "upstream of a gene" means a region on a 5' side of the gene in a DNA sense strand.

In the present specification, the "homolog" refers to homologous genes derived from a common ancestor. The "ortholog", also called "orthologue", refers to a homolog which has diverged after a specification event. The orthologs reside in different organism species and have the same or similar functions. However, in general, olfactory receptor genes undergo duplication and deletion at high frequencies in each organism species, and have many pseudogenes. Therefore, it is allegedly difficult to strictly determine an orthologous relationship. As one example, the ortholog used in the present invention can be an olfactory receptor gene which involves the same name as that a gene of an olfactory receptor of interest and which is of an organism species different from the organism species from which the olfactory receptor of interest is derived, among homologous genes of the gene of the olfactory receptor of interest. When the nomenclature of an olfactory receptor of an organism species is different from the nomenclature in the organism species from which the olfactory receptor of interest is derived, the ortholog may be an olfactory receptor gene having high homology, preferably an olfactory receptor gene having the highest homology, to the gene of the olfactory receptor of interest in the organism species. Alternatively, the ortholog may be an olfactory receptor gene known to be an ortholog of the gene of the olfactory receptor of interest in the organism species. As another example, the ortholog used in the present invention can be an olfactory receptor gene suggested to have the possibility that the gene has diverged after a specification event by phylogenetic tree analysis among the orthologs as above. On the other hand, the "paralog" refers to a homolog resulting from gene duplication. The paralogs reside in the same organism species. The paralogs may have the same or similar functions or may have different functions. As one example, the paralog used in the present invention can be an olfactory receptor gene having high homology, preferably an olfactory receptor gene having the highest homology, to the gene of the olfactory receptor of interest, among olfactory receptor genes of the organism species from which the olfactory receptor of interest is derived.

Ligands remain to be elucidated for many olfactory receptors. There is a demand for the elucidation of ligands for these olfactory receptors or odor responsiveness thereof.

The present inventor conducted diligent studies on an approach which can express on a cultured cell membrane and function an olfactory receptor in light of many olfactory receptors that cannot be functionally analyzed due to insufficient membrane expression in cultured cells. As a result, the present inventor found that consensus design of an olfactory receptor different from a conventional method can improve the membrane expression of an olfactory receptor in cultured cells in comparison with the original olfactory receptor; the consensus design can improve the odor responsiveness of an olfactory receptor; the consensus design can improve the odor responsiveness of an olfactory receptor even if the membrane expression of the olfactory receptor in cultured cells is not improved in comparison with the original olfactory receptor; and the olfactory receptor obtained by the consensus design can well maintain the ligand selectivity of the original olfactory receptor before the consensus design; and analysis results brought about by the olfactory receptor obtained by the consensus design well reflect a human sense of smell.

The present invention provides an approach which can express on a cell membrane and function an olfactory receptor which well maintains the ligand selectivity of the original olfactory receptor. Use of the olfactory receptor enables examination of functions or properties of the original olfactory receptor for which neither a ligand nor odor responsiveness has been confirmed so far. Thus, the approach of the present invention is effective for explaining and predicting how an odorant acts on a human sense of smell and what odor perception the odorant produces.

As shown in Examples mentioned later, the present inventor altered an amino acid sequence of a human olfactory receptor on the basis of a consensus amino acid sequence derived from the amino acid sequence of the human olfactory receptor and amino acid sequences of olfactory receptors encoded by particular orthologs or particular orthologs and paralogs of the human olfactory receptor, and expressed the obtained olfactory receptor polypeptide in cells. As a result, the present inventor found that the membrane expression of the olfactory receptor polypeptide in the cells can be increased in comparison with the human olfactory receptor before the alteration (Figure 1). As a result of altering a mouse olfactory receptor in the same manner as in the case of the human olfactory receptor, and expressing the obtained olfactory receptor polypeptide in cells, the present inventor found that the membrane expression of the olfactory receptor polypeptide in the cells can be increased in comparison with the mouse olfactory receptor before the alteration. Specifically, the olfactory receptor polypeptide is improved in expression stability in comparison with the olfactory receptor before the alteration. In the present specification, the olfactory receptor before the alteration is also referred to as the "original olfactory receptor"; the alteration of an amino acid sequence of an olfactory receptor on the basis of a consensus amino acid sequence is also referred to as the "consensus design"; and the olfactory receptor obtained by the consensus design is also referred to as the "consensus olfactory receptor" or the "altered olfactory receptor polypeptide" in some cases.

As shown in Examples mentioned later, the present inventor also found that when the membrane expression of the consensus olfactory receptor in cells is increased in comparison with the original human olfactory receptor, its responsiveness is also improved; when the membrane expression of the consensus olfactory receptor is increased even slightly, its responsiveness is sufficiently improved (Figure 1); and further importantly, the consensus olfactory receptor well maintains the ligand selectivity of the original human olfactory receptor (Figures 2 and 3). The present inventor also performed consensus design as to 34 types of human olfactory receptors whose responsiveness to a ligand candidate substance had not been demonstrated in preceding studies although the ligand candidate substance had been identified. As a result, the present inventor found that the membrane expression of the olfactory receptors as many as approximately 85% is increased (Figure 4). The present inventor further performed the consensus design of a human olfactory receptor never before possible to functionally analyze, and thereby confirmed that olfactory receptors predicted as candidates of an olfactory receptor which responds to an odorant actually exhibit responsiveness to the odorant (Figures 5 and 6). These olfactory receptors include receptors reported to fail response measurement in Non Patent Literature 2, though consensus design has been attempted. The present inventor further confirmed that receptor responsiveness consistent with different sensory evaluation results found in a human population can be observed by applying consensus design to a human olfactory receptor never before possible to functionally analyze, and then introducing thereto an amino acid sequence found to have difference among individuals in the human population (Figure 7). Moreover, the present inventor revealed that the consensus design of the present invention is effective for a larger number of olfactory receptors (Figure 8 and Tables 12, 14, 15, and 17) .

As shown in Examples mentioned later, the present inventor further found that even when the membrane expression of the consensus olfactory receptor in cells is equivalent and is not increased in comparison with the original human olfactory receptor, the consensus olfactory receptor well maintains the ligand selectivity of the original human olfactory receptor and is improved in responsiveness (Figure 9). The improved responsiveness, despite no increase in membrane expression level, suggests the possibility that the consensus design of an olfactory receptor enhances the efficiency of shift of the olfactory receptor to an active structure or the efficiency of coupling to G protein in cells.

Accordingly, the consensus design of an olfactory receptor is useful for expressing in cells and function an olfactory receptor which well maintains its ligand selectivity therein, particularly, an olfactory receptor never before possible to functionally analyze due to insufficient membrane expression in cultured cells. Thus, in one aspect, the present invention provides a method for expressing an olfactory receptor polypeptide. The expression method of the present invention enables improved expression (e.g., increased expression and stabilized expression) of an olfactory receptor. Therefore, the method is preferably a method for improving expression of an olfactory receptor polypeptide. The method includes expressing, in a cell, an olfactory receptor polypeptide consisting of an amino acid sequence obtained by, in an amino acid sequence of an olfactory receptor of interest, altering at least one amino acid residue different from that in a consensus amino acid sequence to an amino acid residue at a position corresponding thereto in the consensus amino acid sequence, wherein
the consensus amino acid sequence is an amino acid sequence derived by alignment of the amino acid sequence of the olfactory receptor of interest and amino acid sequences of any of the following olfactory receptors (a) to (d):
(a) at least 11 olfactory receptors selected from the group consisting of olfactory receptors encoded by orthologs of the olfactory receptor of interest in the same order as that of an organism species from which the olfactory receptor of interest is derived;
(b) at least 11 olfactory receptors selected from the group consisting of the olfactory receptors encoded by orthologs of the olfactory receptor of interest in the same order as that of an organism species from which the olfactory receptor of interest is derived and olfactory receptors encoded by paralogs of the olfactory receptor of interest, the 11 olfactory receptors including at least one of the olfactory receptors encoded by the paralog of the olfactory receptor of interest;
(c) at least 11 olfactory receptors selected from the group consisting of olfactory receptors encoded by orthologs of the olfactory receptor of interest in vertebrates, the 11 olfactory receptors including at least one olfactory receptor encoded by a vertebrate ortholog in an order different from the order of an organism species from which the olfactory receptor of interest is derived; and
(d) at least 11 olfactory receptors selected from the group consisting of olfactory receptors encoded by orthologs of the olfactory receptor of interest in vertebrates and olfactory receptors encoded by orthologs in a vertebrate with a paralog having the highest homology to the olfactory receptor of interest among paralogs of the olfactory receptor of interest having 11 or more vertebrate orthologs, the 11 olfactory receptors including at least one olfactory receptor encoded by a vertebrate orthologs in an order different from the order of an organism species from which the olfactory receptor of interest is derived, and
an olfactory receptor encoded by the paralog.

In the expression method of the present invention, the olfactory receptor of interest is not particularly limited and is preferably an olfactory receptor for which improvement in responsiveness in cells, or improvement in cell membrane expression and responsiveness is desired. The olfactory receptor of interest may be an olfactory receptor of any organism species and is preferably a mammalian olfactory receptor, more preferably a human olfactory receptor. An olfactory receptor of any family, subfamily, or member may be used. Olfactory receptor families differ depending on a species. Examples of human olfactory receptor families include OR1, OR2, OR3, OR4, OR5, OR6, OR7, OR8, OR9, OR10, OR11, OR12, OR13, OR14, OR51, OR52, OR55, and OR56. The olfactory receptor of interest may be an olfactory receptor that can or cannot be functionally analyzed using cultured cells by a conventional technique. The method of the present invention is more suitably applied to an olfactory receptor that cannot be functionally analyzed using cultured cells by a conventional technique.

In the expression method of the present invention, the olfactory receptors (a) are olfactory receptors encoded by orthologs of the olfactory receptor of interest in the same order as that of an organism species from which the olfactory receptor of interest is derived. The orthologs are not particularly limited and are preferably orthologs having higher homology to the gene of the olfactory receptor of interest.

In a preferred example of the present embodiment, the orthologs of the olfactory receptor of interest in the same order as that of an organism species from which the olfactory receptor of interest is derived in (a) are genes involving the same name as that of the gene of the olfactory receptor of interest among olfactory receptor genes of organism species belonging to the order. The orthologs can be selected by, for example, the following procedures: database search is performed with NCBI BLAST or the like by setting the amino acid sequence of the olfactory receptor of interest to a query sequence and an organism name to be searched to the same order as that of an organism species from which the olfactory receptor of interest is derived. The genes involving the same name as that of the gene of the olfactory receptor of interest are selected from the resulting homologous gene group (e.g., top 500 genes, preferably top 250 genes, more preferably top 100 genes, further more preferably top 50 genes).

In a more preferred example of the present embodiment, the orthologs of the olfactory receptor of interest in the same order as that of an organism species from which the olfactory receptor of interest is derived in (a) are olfactory receptor genes of organism species belonging to the order and are genes suggested to have the possibility that the genes diverged after a specification event by phylogenetic tree analysis among the genes involving the same name as that of the gene of the olfactory receptor of interest. The orthologs can be selected by, for example, the following procedures: database search is performed with NCBI BLAST or the like by setting the amino acid sequence of the olfactory receptor of interest to a query sequence and an organism name to be searched to the same order as that of an organism species from which the olfactory receptor of interest is derived. A phylogenetic tree is prepared by an approach known in the art using the resulting homologous gene group (e.g., top 500 genes, preferably top 250 genes, more preferably top 100 genes, further more preferably top 50 genes). A clade is identified which includes as many genes involving the same name as that of the gene of the olfactory receptor of interest as possible and as few other genes as possible. Then, genes included in the identified clade are selected.

When a plurality of genes derived from the same organism species are selected as orthologs, only one gene having the highest homology to the gene of the olfactory receptor of interest may be selected. When the nomenclature of an olfactory receptor of an organism species is different from the nomenclature in the organism species from which the olfactory receptor of interest is derived, a gene having high homology, preferably a gene having the highest homology, to the gene of the olfactory receptor of interest in the organism species may be selected as an ortholog. Alternatively, a gene known to be an ortholog of the gene of the olfactory receptor of interest in the organism species may be selected.

The number of types of the olfactory receptors (a) is at least 11 types, preferably at least 15 types, in terms of the number of receptors. On the other hand, the upper limit of the number of types is the number of all types of orthologs in organism species in the same order as that of an organism species from which the olfactory receptor of interest is derived. The number of types is preferably 500 or less types, more preferably 250 or less types, further more preferably 100 or less types, further more preferably 50 or less types, in terms of the number of receptors. The number of types of the olfactory receptors (a) can be, for example, from 11 types to all types of orthologs in organism species in the same order as that of an organism species from which the olfactory receptor of interest is derived, from 11 to 500 types, from 11 to 250 types, from 11 to 100 types, from 11 to 50 types, or from 15 to 50 types, in terms of the number of receptors.

In the expression method of the present invention, the olfactory receptors (b) are olfactory receptors encoded by orthologs of the olfactory receptor of interest in the same order as that of an organism species from which the olfactory receptor of interest is derived and olfactory receptors encoded by paralogs of the olfactory receptor of interest. The orthologs are not particularly limited and are preferably orthologs having higher homology to the gene of the olfactory receptor of interest. The paralogs are not particularly limited and are preferably paralogs having high homology, more preferably paralogs having the highest homology, to the gene of the olfactory receptor of interest.

In a preferred example of the present embodiment, the orthologs of the olfactory receptor of interest in the same order as that of an organism species from which the olfactory receptor of interest is derived in (b) are genes involving the same name as that of the gene of the olfactory receptor of interest among olfactory receptor genes of organism species belonging to the order. The orthologs can be selected in the same manner as in the case of (a) described above.

In a more preferred example of the present embodiment, the orthologs of the olfactory receptor of interest in the same order as that of an organism species from which the olfactory receptor of interest is derived in (b) are olfactory receptor genes of organism species belonging to the order and are genes suggested to have the possibility that the genes diverged after a specification event by phylogenetic tree analysis among the genes involving the same name as that of the gene of the olfactory receptor of interest. The orthologs can be selected in the same manner as in the case of (a) described above.

In a preferred example of the present embodiment, the paralogs of the olfactory receptor of interest in (b) are genes having high homology to the gene of the olfactory receptor of interest among olfactory receptor genes of the organism species from which the olfactory receptor of interest is derived. The paralogs can be selected from a homologous gene group (e.g., top 500 genes, preferably top 250 genes, more preferably top 100 genes, further more preferably top 50 genes) obtained as a result of performing database search with NCBI BLAST or the like by setting the amino acid sequence of the olfactory receptor of interest to a query sequence and an organism name to be searched to the organism species from which the olfactory receptor of interest is derived.

Alternatively, the orthologs of the olfactory receptor of interest in the same order as that of an organism species from which the olfactory receptor of interest is derived and the paralogs of the olfactory receptor of interest in (b) can also be selected by, for example, the following procedures: database search is performed with NCBI BLAST or the like by setting the amino acid sequence of the olfactory receptor of interest to a query sequence and an organism name to be searched to the same order as that of an organism species from which the olfactory receptor of interest is derived. A phylogenetic tree is prepared by an approach known in the art using the resulting homologous gene group (e.g., top 500 genes, preferably top 250 genes, more preferably top 100 genes, further more preferably top 50 genes). A clade is identified which includes as many genes involving the same name as that of the gene of the olfactory receptor of interest as possible and as few other genes as possible. A clade is identified which is adjacent to the identified clade and includes a gene, having the highest homology to the gene of the olfactory receptor of interest, in the organism species from which the olfactory receptor of interest is derived. Genes included in the identified clade group are selected. In this respect, when the clade group includes a gene ranked below top 50 of the homologous gene group of organism species in the same order as that of an organism species from which the olfactory receptor of interest is derived, it is preferred to exclude the gene.

The number of types of the olfactory receptors (b) is at least 11 types, preferably at least 25 types, more preferably at least 35 types, in terms of the number of receptors. On the other hand, the upper limit of the number of types is the number of all types of orthologs in organism species in the same order as that of an organism species from which the olfactory receptor of interest is derived and paralogs of the olfactory receptor of interest. The number of types is preferably 500 or less types, more preferably 250 or less types, further more preferably 100 or less types, further more preferably 50 or less types, in terms of the number of receptors. The number of types of the olfactory receptors (b) can be, for example, from 11 types to all types of orthologs in organism species in the same order as that of an organism species from which the olfactory receptor of interest is derived and paralogs of the olfactory receptor of interest, from 11 to 500 types, from 11 to 250 types, from 11 to 100 types, from 11 to 50 types, from 25 to 50 types, or from 35 to 50 types, in terms of the number of receptors. Among them, the number of types of olfactory receptors encoded by paralogs of the olfactory receptor of interest is at least one type in terms of the number of receptors. On the other hand, the upper limit of the number of types is the number of all types of paralogs of the olfactory receptor of interest. The number of types is preferably 50 or less types, more preferably 10 or less types, in terms of the number of receptors. The number of types of olfactory receptors encoded by paralogs of the olfactory receptor of interest can be, for example, from one type to all types of paralogs of the olfactory receptor of interest, from 1 to 50 types, or from 1 to 10 types, in terms of the number of receptors. The olfactory receptors encoded by paralogs are more preferably one member having the highest homology to the gene of the olfactory receptor of interest.

When the olfactory receptor of interest is, for example, a human olfactory receptor, the "same order as that of an organism species from which the olfactory receptor of interest is derived" in (a) or (b) refers to the order *Primates* of the class *Mammalia* of the phylum *Vertebrata.* Organism species belonging to the order *Primates* are called primates and are known as approximately 220 now-existing species. Examples of the primates include, but are not limited to, humans, chimpanzees, bonobos, gorillas, Sumatran orangutans, northern white-cheeked gibbons, drills, gelada baboons, rhesus macaques, olive baboons, sooty mangabeys, green monkeys, ashy red colobuses, Angola colobuses, Garnett's greater galagoes, gray mouse lemurs, Coquerel's sifakas, and Philippine tarsiers. When the olfactory receptor of interest is a human olfactory receptor, the "genes involving the same name as that of the gene of the olfactory receptor of interest" refer to genes involving the same family name, subfamily name, and member name as those of the gene of the human olfactory receptor of interest.

In the expression method of the present invention, the olfactory receptors (c) are olfactory receptors encoded by orthologs of the olfactory receptor of interest in vertebrates and include at least one olfactory receptor encoded by a vertebrate ortholog in an order different from the order of an organism species from which the olfactory receptor of interest is derived. The olfactory receptors encoded by orthologs of the olfactory receptor of interest in vertebrates are preferably olfactory receptors selected from the group consisting of olfactory receptors encoded by orthologs of the olfactory receptor of interest in mammals, Aves, Reptilia, Amphibia, and fish, more preferably olfactory receptors selected from the group consisting of olfactory receptors encoded by orthologs of the olfactory receptor of interest in mammals, Aves, Reptilia, and Amphibia, further more preferably olfactory receptors encoded by orthologs of the olfactory receptor of interest in mammals. The order different from that of an organism species from which the olfactory receptor of interest is derived may be orders other than the order to which the organism species belongs, and a classification system higher than the order may be the same as or different from that of the organism species. The orthologs are not particularly limited and are preferably orthologs having higher homology to the gene of the olfactory receptor of interest. In this context, the mammals refer to organism species belonging to the class *Mammalia* of the phylum *Vertebrata* and are known as approximately 5500 now-existing species also including the primates described above. Examples of the mammals other than the primates described above include, but are not limited to, mice, rats, rabbits, cats, dogs, foxes, raccoon dogs, weasels, tigers, cheetahs, bears, sea lions, earless seals, sea bears, horses, rhinos, camels, pigs, hogs, bovines, goats, sheep, deer, giraffes, hippopotamuses, elephants, pangolins, moles, and bats. The Aves refers to organism species belonging to the class *Aves* of the phylum *Vertebrata.* Examples of the Aves include, but are not limited to, chickens, dabblers, ducks, gooses, turkeys, ostriches, pheasants, doves, parrots, canary-birds, society finches, hummingbirds, manakins, quails, and flycatchers. The Reptilia refers to organism species belonging to the class *Reptilia* of the phylum *Vertebrata.* Examples of the Reptilia include, but are not limited to, tortoises, lizards, gators, iguanas, chameleons, geckos, and snakes. The Amphibia refers to organism species belonging to the class *Amphibia* of the phylum *Vertebrata.* Examples of the Amphibia include, but are not limited to, frogs, newts, and salamanders. The fish collectively refers to organism species belonging to the class *Myxini,* the order *Petromyzontiformes,* the class *Chondrichthyes,* and the class *Osteichthyes* of the phylum *Vertebrata.* Examples of the fish include, but are not limited to, hagfishes, lampreys, sharks, rays, tunas, bonitos, salmons, trout, cods, porgies, flounders, amberjacks, horse mackerels, and mackerels.

In a preferred example of the present embodiment, the orthologs of the olfactory receptor of interest in vertebrates in (c) are genes involving the same name as that of the gene of the olfactory receptor of interest among olfactory receptor genes of organism species belonging to the vertebrates. The orthologs can be selected by, for example, the following procedures: database search is performed with NCBI BLAST or the like by setting the amino acid sequence of the olfactory receptor of interest to a query sequence. The genes involving the same name as that of the gene of the olfactory receptor of interest are selected from the resulting homologous gene group (e.g., top 500 genes, preferably top 250 genes, more preferably top 100 genes, further more preferably top 50 genes).

When a plurality of genes derived from the same organism species are selected as orthologs, only one gene having the highest homology to the gene of the olfactory receptor of interest may be selected. For example, when the olfactory receptor of interest is a human olfactory receptor and a plurality of genes of a non-human organism species are selected as orthologs, a gene of the organism species having the highest homology to the gene of the human olfactory receptor of interest may be selected. When the nomenclature of an olfactory receptor of an organism species is different from the nomenclature in the organism species from which the olfactory receptor of interest is derived, a gene having high homology, preferably a gene having the highest homology, to the gene of the olfactory receptor of interest in the organism species may be selected as an ortholog. Alternatively, a gene known to be an ortholog of the gene of the olfactory receptor of interest in the organism species may be selected.

The number of types of the olfactory receptors (c) is at least 11 types, preferably at least 15 types, more preferably at least 30 types, in terms of the number of receptors. On the other hand, the upper limit of the number of types is the number of all types of orthologs of the olfactory receptor of interest in vertebrates. The number of types is preferably 500 or less types, more preferably 400 or less types, further more preferably 300 or less types, in terms of the number of receptors. The number of types of the olfactory receptors (c) can be, for example, from 11 types to all types of orthologs of the olfactory receptor of interest in vertebrates, from 11 to 500 types, from 11 to 400 types, from 11 to 300 types, from 15 to 300 types, or from 30 to 300 types, in terms of the number of receptors. Among them, the number of types of olfactory receptors encoded by vertebrate orthologs of orders different from that of an organism species from which the olfactory receptor of interest is derived is at least one type, preferably 5 types, more preferably 10 types, in terms of the number of receptors. On the other hand, the upper limit of the number of types of is the number of all types of vertebrate orthologs of orders different from that of an organism species from which the olfactory receptor of interest is derived. The number of types is preferably 250 or less types, more preferably 100 or less types, further more preferably 50 or less types. The number of types of olfactory receptors encoded by vertebrate orthologs of orders different from that of an organism species from which the olfactory receptor of interest is derived can be, for example, from one type to all types of vertebrate orthologs of orders different from that of an organism species from which the olfactory receptor of interest is derived, from 1 to 250 types, from 5 to 250 types, from 10 to 250 types, from 10 to 100 types, or from 10 to 50 types, in terms of the number of receptors.

In the expression method of the present invention, the olfactory receptors (d) are olfactory receptors selected from the group consisting of olfactory receptors encoded by orthologs of the olfactory receptor of interest in vertebrates, and olfactory receptors encoded by orthologs in a vertebrate with a paralog having the highest homology to the olfactory receptor of interest among paralogs of the olfactory receptor of interest having 11 or more vertebrate orthologs, and are olfactory receptors including at least one olfactory receptor encoded by a vertebrate ortholog in an order different from the order of an organism species from which the olfactory receptor of interest is derived, and an olfactory receptor encoded by the paralog. The olfactory receptors encoded by orthologs of the olfactory receptor of interest in vertebrates are preferably olfactory receptors selected from the group consisting of olfactory receptors encoded by orthologs of the olfactory receptor of interest in mammals, Aves, Reptilia, Amphibia, and fish, more preferably olfactory receptors selected from the group consisting of olfactory receptors encoded by orthologs of the olfactory receptor of interest in mammals, Aves, Reptilia, and Amphibia, further more preferably olfactory receptors encoded by orthologs of the olfactory receptor of interest in mammals. The orders different from that of an organism species from which the olfactory receptor of interest is derived may be orders other than the order to which the organism species belongs, and a classification system higher than the order may be the same as or different from that of the organism species. The orthologs are not particularly limited and are preferably orthologs having higher homology to the gene of the olfactory receptor of interest. The orthologs in a vertebrate with a paralog are not particularly limited and are preferably orthologs having higher homology to the paralog. In this context, examples of the mammals, the Aves, the Reptilia, the Amphibia, and the fish can include the same organism species as in the case of (c) described above.

In a preferred example of the present embodiment, the orthologs of the olfactory receptor of interest in vertebrates in (d) are genes involving the same name as that of the gene of the olfactory receptor of interest among olfactory receptor genes of organism species belonging to the vertebrates. The orthologs can be selected in the same manner as in the case of (c) described above. If any ortholog of the olfactory receptor of interest cannot be selected in vertebrates, the olfactory receptors (d) can be olfactory receptors encoded by orthologs in a vertebrate with a paralog having the highest homology to the olfactory receptor of interest among paralogs of the olfactory receptor of interest having 11 or more vertebrate orthologs, and be olfactory receptors including at least one olfactory receptor encoded by a vertebrate ortholog in an order different from the order of an organism species from which the olfactory receptor of interest is derived, and an olfactory receptor encoded by the paralog. The orthologs in a vertebrate with a paralog having the highest homology to the olfactory receptor of interest among paralogs of the olfactory receptor of interest having 11 or more vertebrate orthologs in (d) are preferably olfactory receptor genes of organism species belonging to the vertebrate, are genes involving the same name as that of the paralog having the highest homology to the gene of the olfactory receptor of interest among paralogs of the olfactory receptor of interest having 11 or more vertebrate orthologs, and are selected from the group consisting of paralogs having high homology to the gene of the olfactory receptor of interest. The orthologs can be selected by, for example, the following procedures: database search is performed with NCBI BLAST or the like by setting the amino acid sequence of the olfactory receptor of interest to a query sequence. The paralog having the highest homology to the gene of the olfactory receptor of interest is selected from the resulting homologous gene group (e.g., top 500 genes, preferably top 250 genes, more preferably top 100 genes, further more preferably top 50 genes). Subsequently, genes involving the same name as that of the paralog are selected. When the selected genes are less than 11 genes, a paralog having the second highest homology to the gene of the olfactory receptor of interest is selected and genes involving the same name as that of the paralog are selected in the same manner as above. Finally, this procedure can be repeated until 11 or more genes are selected.

When a plurality of genes derived from the same organism species are selected as orthologs, only one gene having the highest homology to the gene of the olfactory receptor of interest may be selected. For example, when the olfactory receptor of interest is a human olfactory receptor and a plurality of genes of a non-human organism species are selected as orthologs, a gene of the organism species having the highest homology to the gene of the human olfactory receptor of interest may be selected. When the nomenclature of an olfactory receptor of an organism species is different from the nomenclature in the organism species from which the olfactory receptor of interest is derived, a gene having high homology, preferably a gene having the highest homology, to the gene of the olfactory receptor of interest in the organism species may be selected as an ortholog. Alternatively, a gene known to be an ortholog of the gene of the olfactory receptor of interest in the organism species may be selected.

The number of types of the olfactory receptors (d) is at least 11 types, preferably at least 30 types, more preferably at least 60 types, in terms of the number of receptors. On the other hand, the upper limit of the number of types is the number of all types of orthologs of the olfactory receptor of interest in vertebrates, and orthologs in a vertebrate with a paralog having the highest homology to the olfactory receptor of interest among paralogs of the olfactory receptor of interest having 11 or more vertebrate orthologs. The number of types is preferably 500 or less types, more preferably 400 or less types, further more preferably 300 or less types, in terms of the number of receptors. The number of types of the olfactory receptors (d) can be, for example, from 11 types to all types of orthologs of the olfactory receptor of interest in vertebrates, and orthologs in a vertebrate with a paralog having the highest homology to the olfactory receptor of interest among paralogs of the olfactory receptor of interest having 11 or more vertebrate orthologs, from 11 to 500 types, from 11 to 400 types, from 11 to 300 types, from 30 to 300 types, or from 60 to 300 types, in terms of the number of receptors. Among them, the number of types of olfactory receptors encoded by vertebrate orthologs in orders different from that of an organism species from which the olfactory receptor of interest is derived is at least one type, preferably 5 types, more preferably 10 types, in terms of the number of receptors. On the other hand, the upper limit of the number of types is the number of all types of vertebrate orthologs in orders different from that of an organism species from which the olfactory receptor of interest is derived. The number of types is preferably 250 or less types, more preferably 100 or less types, further more preferably 50 or less types. The number of types of olfactory receptors encoded by vertebrate orthologs in orders different from that of an organism species from which the olfactory receptor of interest is derived can be, for example, from one type to all types of vertebrate orthologs in orders different from that of an organism species from which the olfactory receptor of interest is derived, from 1 to 250 types, from 5 to 250 types, from 10 to 250 types, from 10 to 100 types, or from 10 to 50 types, in terms of the number of receptors.

Among the olfactory receptors (a) to (d), the olfactory receptors (c) are preferably used in consensus design because consensus based on olfactory receptor genes of more species can be reflected in the consensus design of the olfactory receptor of interest. If the olfactory receptors (c) cannot be used in the consensus design of the olfactory receptor of interest, in other words, if only less than 11 types of orthologs of the olfactory receptor of interest are identified, the olfactory receptors (d) can be suitably used in the consensus design.

In the expression method of the present invention, the "consensus amino acid sequence" is an amino acid sequence derived by alignment of the amino acid sequence of the olfactory receptor of interest and the amino acid sequences of any of the olfactory receptors (a) to (d). Specifically, the "consensus amino acid sequence" is an amino acid sequence consisting of consensus residues identified in accordance with the following criteria (i) to (iii) from the alignment of the amino acid sequence of the olfactory receptor of interest and the amino acid sequences of any of the olfactory receptors (a) to (d):
(i) at each amino acid position of the alignment,
   (i-i) when there exists one amino acid residue which is different from the amino acid residue of the olfactory receptor of interest and has a frequency of appearance of 50% or more, the amino acid residue is identified as a consensus residue,
   (i-ii) when there exist two amino acid residues having a frequency of appearance of 50%, the amino acid residues of the olfactory receptor of interest are identified as a consensus residue,
   (i-iii) when there exists an amino acid residue in the olfactory receptor of interest and there exists no amino acid residue having a frequency of appearance of 40% or more, the absence of a consensus residue is identified,
   (i-iv) when there exists no amino acid residue in the olfactory receptor of interest and there exists an amino acid residue having a frequency of appearance of 60% or more, an amino acid residue having the highest frequency of appearance is identified as a consensus residue, and when there exist two or more amino acid residues having the highest frequency of appearance, an amino acid residue having the smallest molecular weight among the amino acid residues is identified as a consensus residue, and
   (i-v) if none of the above (i-i) to (i-iv) is appropriate, the amino acid residue of the olfactory receptor of interest is identified as a consensus residue;
(ii) when the consensus residues are identified in accordance with the criterion (i) and when a consensus residue nearest to the N terminus is a consensus residue at a position corresponding to the N terminus of the olfactory receptor of interest or a C-terminal side thereof and is not a methionine residue, a consensus residue on an N-terminal side of a consensus residue consisting of a methionine residue positioned nearest to the N terminus is changed to the absence of a consensus residue; and
(iii) when the consensus residues are identified in accordance with the criterion (i) and when a consensus residue nearest to the N terminus is a consensus residue at a position corresponding to an N-terminal side of the N terminus of the olfactory receptor of interest and is not a methionine residue, an amino acid residue having the highest frequency of appearance is identified as a consensus residue by tracing back one by one amino acid positions on an N-terminal side of the position of the consensus residue of the alignment until a methionine residue appears, and when there exist two or more amino acid residues having the highest frequency of appearance, an amino acid residue having the smallest molecular weight among the amino acid residues is identified as a consensus residue.

In this context, the "frequency of appearance" refers to a percentage of the number of particular amino acid residues appearing at each amino acid position in the alignment of amino acid sequences with respect to the number of amino acid sequences subjected to the alignment. The alignment of amino acid sequences can be carried out with an algorithm known in the art.

The criterion (i), (i-i) is a criterion in the case where there exists an amino acid residue in the olfactory receptor of interest at an amino acid position in the alignment of the amino acid sequence of the olfactory receptor of interest and the amino acid sequences of any of the olfactory receptors (a) to (d). In this respect, provided that there exists one amino acid residue which is different from the amino acid residue of the olfactory receptor of interest and has a frequency of appearance of 50% or more, the amino acid residue having a frequency of appearance of 50% or more is identified as a consensus residue at the position. On the other hand, provided that all amino acid residues other than the amino acid residue of the olfactory receptor of interest have a frequency of appearance of less than 50%, the amino acid residue of the olfactory receptor of interest is identified as a consensus residue at the position in accordance with the criterion (i-v).

The criterion (i), (i-ii) is a criterion in the case where there exists an amino acid residue in the olfactory receptor of interest at an amino acid position in the alignment of the amino acid sequence of the olfactory receptor of interest and the amino acid sequences of any of the olfactory receptors (a) to (d). In this respect, provided that there exist two amino acid residues having a frequency of appearance of 50%, one of the two amino acid residues is inevitably the amino acid residue of the olfactory receptor of interest and the amino acid residue of the olfactory receptor of interest is identified as a consensus residue at the position.

The criterion (i), (i-iii) is a criterion in the case where there exists an amino acid residue in the olfactory receptor of interest at an amino acid position in the alignment of the amino acid sequence of the olfactory receptor of interest and the amino acid sequences of any of the olfactory receptors (a) to (d). In this respect, provided that there exists no amino acid residue having a frequency of appearance of 40% or more, the absence of a consensus residue is identified at the position. On the other hand, provided that the frequency of appearance in the absence of an amino acid residue is less than 40%, a consensus residue at the position is identified in accordance with the criterion (i-i) when the criterion (i-i) is appropriate, while the amino acid residue of the olfactory receptor of interest is identified as a consensus residue at the position in accordance with the criterion (i-v) when the criterion (i-i) is not appropriate.

The criterion (i), (i-iv) is a criterion in the case where there exists no amino acid residue in the olfactory receptor of interest at an amino acid position in the alignment of the amino acid sequence of the olfactory receptor of interest and the amino acid sequences of any of the olfactory receptors (a) to (d). In this respect, provided that there exists an amino acid residue having a frequency of appearance of 60% or more, an amino acid residue having the highest frequency of appearance is identified as a consensus residue at the position, and when there exist two or more amino acid residues having the highest frequency of appearance, an amino acid residue having the smallest molecular weight among the amino acids having the highest frequency of appearance is identified as a consensus residue. For example, provided that one amino acid residue has the highest frequency of appearance, the one amino acid residue is identified as a consensus residue at the position. Provided that two or more amino acid residues have the highest frequency of appearance, an amino acid residue having the smallest molecular weight thereamong can be identified as a consensus residue at the position. If the change extends the full-length of the consensus amino acid sequence by 10% or more on an N-terminal side from the full-length of the amino acid sequence of the olfactory receptor of interest, a consensus residue at a position corresponding to the N terminus of the olfactory receptor of interest may be set to a methionine residue and a consensus residue on an N-terminal side of the methionine residue may be changed to the absence, in the consensus amino acid sequence from the viewpoint of structural maintenance of the olfactory receptor. Specifically, an N-terminal structure of the olfactory receptor of interest may be maintained as it is. On the other hand, provided that the frequency of appearance in the presence of an amino acid residue is less than 60%, the amino acid residue of the olfactory receptor of interest is identified as a consensus residue at the position in accordance with the criterion (i-v). Specifically, the absence of an amino acid residue is identified at the position.

If none of the above (i), (i-i) to (i-iv) is appropriate for an amino acid position in the alignment of the amino acid sequence of the olfactory receptor of interest and the amino acid sequences of any of the olfactory receptors (a) to (d), the amino acid residue of the olfactory receptor of interest is identified as a consensus residue at the position. In this respect, provided that there exists no amino acid residue in the olfactory receptor of interest, the absence of an amino acid residue can be identified at the position of the consensus amino acid sequence.

The criterion (ii) is a criterion in the case where when the consensus residue is identified at each amino acid position in the alignment of the amino acid sequence of the olfactory receptor of interest and the amino acid sequences of any of the olfactory receptors (a) to (d) in accordance with the criterion (i), a consensus residue positioned nearest to the N terminus among the consensus residues is a consensus residue at a position corresponding to the N terminus of the olfactory receptor of interest or a C-terminal side thereof and is not a methionine residue. In this respect, a consensus residue on an N-terminal side of a consensus residue consisting of a methionine residue positioned nearest to the N terminus among the consensus residues is changed to the absence of a consensus residue such that an N-terminal consensus residue is a methionine residue, in other words, such that a translation initiation amino acid of the olfactory receptor polypeptide is a methionine residue. For example, the consensus residues are checked one by one from the N terminus, and the absence of a consensus residue is identified at a position having no methionine residue. This operation can be repeated until a methionine residue appears for the first time. If the change shortens the full-length of the consensus amino acid sequence by 10% or more from the full-length of the amino acid sequence of the olfactory receptor of interest, a consensus residue nearest to the N terminus among the consensus residues before the change may be changed to a consensus residue consisting of a methionine residue from the viewpoint of structural maintenance of the helix of the olfactory receptor. In this respect, provided that the consensus residue nearest to the N terminus among the consensus residues before the change is asparagine, serine, or threonine involved in sugar chain modification and/or membrane translocation, an amino acid residue of the olfactory receptor of interest on an N-terminal side of a position corresponding to the consensus residue may be used as a consensus residue without changing the consensus residue nearest to the N terminus among the consensus residues before the change from the viewpoint of structural maintenance of the olfactory receptor. Specifically, an N-terminal structure of the olfactory receptor of interest may be maintained as it is.

The criterion (iii) is a criterion in the case where when the consensus residue is identified at each amino acid position in the alignment of the amino acid sequence of the olfactory receptor of interest and the amino acid sequences of any of the olfactory receptors (a) to (d) in accordance with the criterion (i), a consensus residue nearest to the N terminus is a consensus residue at a position corresponding to an N-terminal side of the N terminus of the olfactory receptor of interest and is not a methionine residue. In this respect, the alignment is checked by tracing back one by one amino acid positions on an N-terminal side of the position of a consensus residue nearest to the N terminus until a methionine residue appears such that an N-terminal consensus residue is a methionine residue, in other words, such that a translation initiation amino acid of the olfactory receptor polypeptide is a methionine residue. An amino acid residue having the highest frequency of appearance is identified as a consensus residue. When there exist two or more amino acid residues having the highest frequency of appearance, an amino acid residue having the smallest molecular weight among the amino acid residues is identified as a consensus residue.

An amino acid sequence consisting of the consensus residues thus identified is the consensus amino acid sequence. The olfactory receptor polypeptide used in the present invention is an altered olfactory receptor polypeptide consisting of an amino acid sequence obtained by, in an amino acid sequence of an olfactory receptor of interest, altering at least one amino acid residue different from that in a consensus amino acid sequence to an amino acid residue at a position corresponding thereto in the consensus amino acid sequence. In this context, the term "altering" conceptually includes all of substitution, deletion, addition, and insertion. The difference between the amino acid sequence of the olfactory receptor of interest and the consensus amino acid sequence can be found, for example, by aligning the amino acid sequences and comparing both the amino acid sequences using an algorithm known in the art. For example, provided that an amino acid residue at an amino acid position in the amino acid sequence of the olfactory receptor of interest is an amino acid residue different from an amino acid residue at a position corresponding thereto in the consensus amino acid sequence when the amino acid sequence of the olfactory receptor of interest is compared with the consensus amino acid sequence, the amino acid residue of the olfactory receptor of interest can be substituted by the amino acid residue of the consensus amino acid sequence. Alternatively, provided that an amino acid residue at a position corresponding to an amino acid position in the amino acid sequence of the olfactory receptor of interest is absent in the consensus amino acid sequence when the amino acid sequence of the olfactory receptor of interest is compared with the consensus amino acid sequence, an amino acid residue at the amino acid position of the olfactory receptor of interest can be deleted. Alternatively, provided that an amino acid residue at a position corresponding to a position having no amino acid residue in the amino acid sequence of the olfactory receptor of interest is present in the consensus amino acid sequence when the amino acid sequence of the olfactory receptor of interest is compared with the consensus amino acid sequence, the amino acid residue of the consensus amino acid sequence can be inserted to the amino acid position of the olfactory receptor of interest. Provided that the consensus amino acid sequence is longer at the N terminus than the amino acid sequence of the olfactory receptor of interest when the amino acid sequence of the olfactory receptor of interest is compared with the consensus amino acid sequence, an N-terminal moiety present only in the consensus amino acid sequence can be added to the N terminus of the amino acid sequence of the olfactory receptor of interest. Alternatively, provided that the consensus amino acid sequence is longer at the C terminus than the amino acid sequence of the olfactory receptor of interest when the amino acid sequence of the olfactory receptor of interest is compared with the consensus amino acid sequence, a C-terminal moiety present only in the consensus amino acid sequence can be added to the C terminus of the amino acid sequence of the olfactory receptor of interest.

When the consensus amino acid sequence is a sequence derived from the olfactory receptor of interest and the olfactory receptors (a), the number of amino acid residues to be altered in the amino acid sequence of the olfactory receptor of interest is at least 1, preferably at least 3, more preferably at least 5. Further more preferably, all amino acid residues different from those of the consensus amino acid sequence in the amino acid sequence of the olfactory receptor of interest are altered to the amino acid residues of the consensus amino acid sequence at positions corresponding thereto (i.e., the amino acid sequence of the olfactory receptor of interest is altered to the consensus amino acid sequence). When the consensus amino acid sequence is a sequence derived from the olfactory receptor of interest and the olfactory receptors (b), the number of amino acid residues to be altered in the amino acid sequence of the olfactory receptor of interest is at least 1, preferably at least 3, more preferably at least 5. Further more preferably, all amino acid residues different from those in the consensus amino acid sequence in the amino acid sequence of the olfactory receptor of interest are altered to the amino acid residues at positions corresponding thereto in the consensus amino acid sequence (i.e., the amino acid sequence of the olfactory receptor of interest is altered to the consensus amino acid sequence). When the consensus amino acid sequence is a sequence derived from the olfactory receptor of interest and the olfactory receptors (c), the number of amino acid residues to be altered in the amino acid sequence of the olfactory receptor of interest is at least 1, preferably at least 5, more preferably at least 10. Further more preferably, all amino acid residues different from those in the consensus amino acid sequence in the amino acid sequence of the olfactory receptor of interest are altered to the amino acid residues at positions corresponding thereto in the consensus amino acid sequence (i.e., the amino acid sequence of the olfactory receptor of interest is altered to the consensus amino acid sequence). When the consensus amino acid sequence is a sequence derived from the olfactory receptor of interest and the olfactory receptors (d), the number of amino acid residues to be altered in the amino acid sequence of the olfactory receptor of interest is at least 1, preferably at least 5, more preferably at least 10. Further more preferably, all amino acid residues different from those in the consensus amino acid sequence in the amino acid sequence of the olfactory receptor of interest are altered to the amino acid residues at positions corresponding thereto in the consensus amino acid sequence (i.e., the amino acid sequence of the olfactory receptor of interest is altered to the consensus amino acid sequence).

Alternatively, the number of amino acid residues to be altered in the amino acid sequence of the olfactory receptor of interest can be the number of preferably at least 10%, more preferably at least 30%, further more preferably at least 50%, further more preferably at least 70%, further more preferably at least 90%, further more preferably 100% of the number of amino acid residues different from those in the consensus amino acid sequence (i.e., the amino acid sequence of the olfactory receptor of interest is altered to the consensus amino acid sequence).

Provided that the consensus amino acid sequence is longer at the N terminus than the amino acid sequence of the olfactory receptor of interest, it is preferred to perform the alteration by integrally adding an N-terminal moiety present only in the consensus amino acid sequence to the N terminus of the amino acid sequence of the olfactory receptor of interest, irrespective of the number of amino acid residues. Provided that the consensus amino acid sequence is shorter at the N terminus than the amino acid sequence of the olfactory receptor of interest, it is preferred to perform the alteration by integrally deleting an N-terminal moiety present only in the amino acid sequence of the olfactory receptor of interest from the N terminus of the amino acid sequence of the olfactory receptor of interest, irrespective of the number of amino acid residues. Provided that the consensus amino acid sequence is longer at the C terminus than the amino acid sequence of the olfactory receptor of interest, it is preferred to perform the alteration by integrally adding a C-terminal moiety present only in the consensus amino acid sequence to the C terminus of the amino acid sequence of the olfactory receptor of interest, irrespective of the number of amino acid residues. Provided that the consensus amino acid sequence is shorter at the C terminus than the amino acid sequence of the olfactory receptor of interest, it is preferred to perform the alteration by integrally deleting a C-terminal moiety present only in the amino acid sequence of the olfactory receptor of interest from the C terminus of the amino acid sequence of the olfactory receptor of interest, irrespective of the number of amino acid residues.

As the olfactory receptor polypeptide used in the present invention, a polypeptide containing substitution, deletion, addition, or insertion of one to several (e.g., 1 to 10, 1 to 5, or 1 to 3) amino acid residues at amino acid positions other than the amino acid position to be altered on the basis of the consensus amino acid sequence described above is also included in the present invention without impairing its functions.

In a preferred example of the present embodiment, the olfactory receptor polypeptide consists of an amino acid sequence obtained by, in an amino acid sequence of sequence identification number (2) of an olfactory receptor (1) in Tables 1-1 to 1-5 given below, altering at least one amino acid residue different from that in a consensus amino acid sequence of sequence identification number (3) to an amino acid residue at a position corresponding thereto in the consensus amino acid sequence. More preferably, the olfactory receptor polypeptide consists of a consensus amino acid sequence of sequence identification number (3) in Tables 1-1 to 1-5 given below. Further more preferably, the olfactory receptor polypeptide consists of an amino acid sequence of any of SEQ ID NOs: 99 to 104, 109 to 118, 121, 123, 125 to 130, 133, 136, 138, 140 to 146, 149 to 210, 485 to 642, and 851 to 900. The olfactory receptor polypeptide consisting of an amino acid sequence of any of SEQ ID NOs: 97 to 107, 109 to 121, 123, 125 to 133, 135 to 210, 485 to 639, 641, 642, and 851 to 900 is highly improved in membrane expression in comparison with the original olfactory receptor. In Tables 1-1 to 1-5, the olfactory receptors (1) of Nos. 1 to 270 and 272 to 322 are human olfactory receptors, and the olfactory receptor (1) of No. 271 is a mouse olfactory receptor. Accession No. represents GenBank Accession No.

**[Table 1-1]**

| No. | (1) Olfactory receptor | Accession No. | (2) SEQ ID NO of amino acid sequence | (3) SEQ ID NO of consensus amino acid sequence | No. | (1) Olfactory receptor | Accession No. | (2) SEQ ID NO of amino acid sequence | (3) SEQ ID NO of consensus amino acid sequence |
|---|---|---|---|---|---|---|---|---|---|
| 1 | OR2A25 | NP_001004488.1 | 1 | 97 | 31 | OR5W2 | NP_001001960.1 | 21 | 127 |
| 2 | OR2A25 | NP_001004488.1 | 1 | 98 | 32 | OR6A2 | NP_003687.2 | 22 | 128 |
| 3 | OR2A25 | NP_001004488.1 | 1 | 99 | 33 | OR6B2 | NP_001005853.1 | 23 | 129 |
| 4 | OR2AG1 | NP_001004489.1 | 2 | 100 | 34 | OR6C1 | NP_001005182.1 | 24 | 130 |
| 5 | OR2AG2 | NP_001004490.1 | 3 | 101 | 35 | OR6Y1 | NP_001005189.1 | 25 | 131 |
| 6 | OR2D2 | ALI87480.1 | 4 | 102 | 36 | OR6Y1 | NP_001005189.1 | 25 | 132 |
| 7 | OR2D3 | NP_001004684.1 | 5 | 103 | 37 | OR6Y1 | NP_001005189.1 | 25 | 133 |
| 8 | OR2T11 | NP_001001964.1 | 6 | 104 | 38 | OR7A17 | NP_112163.1 | 26 | 134 |
| 9 | OR2T11 | NP_001001964.1 | 6 | 105 | 39 | OR7A17 | NP_112163.1 | 26 | 135 |
| 10 | OR2T11 | NP_001001964.1 | 6 | 106 | 40 | OR7A17 | NP_112163.1 | 26 | 136 |
| 11 | OR2W1 | NP_112165.1 | 7 | 107 | 41 | OR7D4 | NP_001005191.1 | 27 | 137 |
| 12 | OR2W1 | NP_112165.1 | 7 | 108 | 42 | OR7D4 | NP_001005191.1 | 27 | 138 |
| 13 | OR2W1 | NP_112165.1 | 7 | 109 | 43 | OR7D4 | NP_001005191.1 | 27 | 139 |
| 14 | OR2W3 | NP_001001957.2 | 8 | 110 | 44 | OR10A2 | NP_001004460.1 | 28 | 140 |
| 15 | OR2Y1 | NP_001001657.1 | 9 | 111 | 45 | OR10A4 | ALI87721.1 | 29 | 141 |
| 16 | OR3A2 | NP_002542.3 | 10 | 112 | 46 | OR10D3 | NP_001342142.1 | 30 | 142 |
| 17 | OR4A16 | NP_001005274.1 | 11 | 113 | 47 | OR10Q1 | NP_001004471.1 | 31 | 143 |
| 18 | OR4C15 | NP_001001920.2 | 12 | 114 | 48 | OR10Z1 | NP_001004478.1 | 32 | 144 |
| 19 | OR4D5 | NP_001001965.1 | 13 | 115 | 49 | OR11H1 | NP_001005239 | 33 | 145 |
| 20 | OR4D6 | NP_001004708.1 | 14 | 116 | 50 | OR11H4 | NP_001004479.1 | 34 | 146 |
| 21 | OR4F15 | NP_001001674.1 | 15 | 117 | 51 | OR11H4 | NP_001004479.1 | 34 | 147 |
| 22 | OR4K15 | NP_001005486.1 | 16 | 118 | 52 | OR11H4 | NP_001004479.1 | 34 | 148 |
| 23 | OR4Q3 | NP_751944.1 | 17 | 119 | 53 | OR13C8 | HP_001004483.1 | 35 | 149 |
| 24 | OR4Q3 | NP_751944.1 | 17 | 120 | 54 | OR13G1 | NP_001005487.1 | 36 | 150 |
| 25 | OR4Q3 | NP_751944.1 | 17 | 121 | 55 | OR52A4 | AAI40753.1 | 37 | 151 |
| 26 | OR4S2 | NP_001004059.2 | 18 | 122 | 56 | OR56A4 | NP_001005179.3 | 38 | 152 |
| 27 | OR4S2 | NP_001004059.2 | 18 | 123 | | | | | |
| 28 | OR4S2 | NP_001004059.2 | 18 | 124 | | | | | |
| 29 | OR5A2 | NP_001001954.1 | 19 | 125 | | | | | |
| 30 | OR5C1 | NP_001001923.1 | 20 | 126 | | | | | |

**[Table 1-2]**

| No. | (1) Olfactory receptor | Accession No. | (2) SEQ ID NO of amino acid sequence | (3) SEQ ID NO of consensus amino acid sequence | No. | (1) Olfactory receptor | Accession No. | (2) SEQ ID NO of amino acid sequence | (3) SEQ ID NO of consensus amino acid sequence |
|---|---|---|---|---|---|---|---|---|---|
| 57 | OR2B11 | NP_001004492.1 | 39 | 153 | 87 | OR4K17 | NP_001004715.3 | 69 | 183 |
| 58 | OR2C3 | NP_932340.4 | 40 | 154 | 88 | OR4L1 | NP_ 001004717.1 | 70 | 184 |
| 59 | OR2G2 | NP_001001915.1 | 41 | 155 | 89 | OR4M1 | NP_001005500.1 | 71 | 185 |
| 60 | OR2G3 | NP_001001914.1 | 42 | 156 | 90 | OR4N2 | NP_ 001004723.1 | 72 | 186 |
| 61 | OR2L2 | NP_001004686.1 | 43 | 157 | 91 | OR4N5 | NP_ 001004724.1 | 73 | 187 |
| 62 | OR2L3 | NP_001004687.1 | 44 | 158 | 92 | OR5A1 | NP_001004728 | 74 | 188 |
| 63 | OR2L5 | NP_001245213.1 | 45 | 159 | 93 | OR6F1 | NP_001005286.1 | 75 | 189 |
| 64 | OR2L8 | NP_001001963.1 | 46 | 160 | 94 | OR6J1 | NP_001335162.1 | 76 | 190 |
| 65 | OR2L13 | NP_001291464.1 | 47 | 161 | 95 | OR7A10 | NP_001005190.1 | 77 | 191 |
| 66 | OR2M2 | NP_001004688.1 | 48 | 162 | 96 | OR7C2 | NP_036509.1 | 78 | 192 |
| 67 | OR2M4 | NP_059974.1 | 49 | 163 | 97 | OR7G2 | NP_001005193.2 | 79 | 193 |
| 68 | OR2T1 | NP_112166.2 | 50 | 164 | 98 | OR7G3 | NP_001001958.1 | 80 | 194 |
| 69 | OR2T4 | NP_001004696.2 | 51 | 165 | 99 | OR10G2 | NP_001005466.2 | 81 | 195 |
| 70 | OR2T5 | NP_001004697.1 | 52 | 166 | 100 | OR10J1 | NP_036483.3 | 82 | 196 |
| 71 | OR2T6 | NP_001005471.1 | 53 | 167 | 101 | OR10J3 | NP_001004467.1 | 83 | 197 |
| 72 | OR2T7 | NP_001372981.1 | 54 | 168 | 102 | OR10K1 | NP_001004473.1 | 84 | 198 |
| 73 | OR2T8 | NP_001005522.1 | 55 | 169 | 103 | OR10K2 | NP_001004476.1 | 85 | 199 |
| 74 | OR2T12 | NP_001004692.1 | 56 | 170 | 104 | OR10T2 | NP_001004475.1 | 86 | 200 |
| 75 | OR2T27 | NP_001001824.1 | 57 | 171 | 105 | OR10X1 | NP_001004477.1 | 87 | 201 |
| 76 | OR2T29 | NP_001004694.2 | 58 | 172 | 106 | OR11G2 | NP_001372962.1 | 88 | 202 |
| 77 | OR2T33 | NP_001004695.1 | 59 | 173 | 107 | OR11H6 | NP_001004480.1 | 89 | 203 |
| 78 | OR2T34 | NP_001001821.1 | 60 | 174 | 108 | OR11H7 | NP_001335202.1 | 90 | 204 |
| 79 | OR2T35 | NP_001001827.1 | 61 | 175 | 109 | OR11L1 | NP_001001959.1 | 91 | 205 |
| 80 | OR2AK2 | NP_001004491.1 | 62 | 176 | 110 | OR14A2 | NP_001342221.1 | 92 | 206 |
| 81 | OR4D10 | NP_001004705.1 | 63 | 177 | 111 | OR14A16 | NP_001001966.1 | 93 | 207 |
| 82 | OR4E2 | NP_001001912.2 | 64 | 178 | 112 | OR14C36 | NP_001001918.1 | 94 | 208 |
| 83 | OR4F5 | NP_001005484.2 | 65 | 179 | 113 | OR14K1 | NP_001004732.2 | 95 | 209 |
| 84 | OR4K1 | XP_011535455.1 | 66 | 180 | 114 | OR7E24 | NP_001373037.1 | 96 | 210 |
| 85 | OR4K2 | NP_001005501.1 | 67 | 181 | | | | | |
| 86 | OR4K5 | NP_001005483.1 | 68 | 182 | | | | | |

**[Table 1-3]**

| No. | (1) Olfactory receptor | Accession No. | (2) SEQ ID NO of amino acid sequence | (3) SEQ ID NO of consensus amino acid sequence | No. | (1) Olfactory receptor | Accession No. | (2 )SEQ ID NO of amino acid sequence | (3) SEQ ID NO of consensus amino acid sequence |
|---|---|---|---|---|---|---|---|---|---|
| 115 | OR1B1 | NP_001004450.2 | 327 | 485 | 155 | OR3A3 | NP_036505.3 | 367 | 525 |
| 116 | OR1D2 | NP_002539.2 | 328 | 486 | 156 | OR4A5 | NP_001005272.3 | 368 | 526 |
| 117 | OR1D4 | NP_003543.3 | 329 | 487 | 157 | OR4A15 | NP_001005275.1 | 369 | 527 |
| 118 | OR1E2 | NP_003545.1 | 330 | 488 | 158 | OR4A47 | NP_001005512.2 | 370 | 528 |
| 119 | OR1F1 | NP_036492.1 | 331 | 489 | 159 | OR4B1 | NP_001005470.1 | 371 | 529 |
| 120 | OR1G1 | NP_003546.1 | 332 | 490 | 160 | OR4C3 | NP_001004702.2 | 372 | 530 |
| 121 | OR1I1 | NP_001004713.1 | 333 | 491 | 161 | OR4C6 | NP_001004704.1 | 373 | 531 |
| 122 | OR1J1 | NP_001004451.1 | 334 | 492 | 162 | OR4C11 | NP_001004700.2 | 374 | 532 |
| 123 | OR1J4 | NP_001004452.1 | 335 | 493 | 163 | OR4C12 | NP_001005270.3 | 375 | 533 |
| 124 | OR1K1 | NP_543135.1 | 336 | 494 | 164 | OR4C16 | NP_001004701.2 | 376 | 534 |
| 125 | OR1L1 | NP_001005236.3 | 337 | 495 | 165 | OR4C46 | NP_001004703.1 | 377 | 535 |
| 126 | OR1L3 | NP_001005234.1 | 338 | 496 | 166 | OR4D1 | NP_036506.1 | 378 | 536 |
| 127 | OR1L4 | NP_001005235.1 | 339 | 497 | 167 | OR4D2 | NP_001004707.1 | 379 | 537 |
| 128 | OR1L6 | NP_001004453.2 | 340 | 498 | 168 | OR4D9 | NP_001004711.1 | 380 | 538 |
| 129 | OR1L8 | NP_001004454.1 | 341 | 499 | 169 | OR4D11 | NP_ 001004706.1 | 381 | 539 |
| 130 | OR1M1 | NP_001004456.1 | 342 | 500 | 170 | OR4F17 | MP_001005240.1 | 382 | 540 |
| 131 | OR1Q1 | NP_036496.1 | 343 | 501 | 171 | OR4F21 | NP_001005504.1 | 383 | 541 |
| 132 | OR1S1 | NP_001004458.1 | 344 | 502 | 172 | OR4P4 | NP_001004124.1 | 384 | 542 |
| 133 | OR1S2 | NP_001004459.1 | 345 | 503 | 173 | OR4X1 | NP_001004726.1 | 385 | 543 |
| 134 | OR2A1 | NP_001005287.1 | 346 | 504 | 174 | OR4X2 | NP_001004727.1 | 386 | 544 |
| 135 | OR2A5 | NP_036497.1 | 347 | 505 | 175 | OR5B2 | NP_001005566.1 | 387 | 545 |
| 136 | OR2A7 | NP_001005328.1 | 348 | 506 | 176 | OR5B12 | NP_001004733.1 | 388 | 546 |
| 137 | OR2A14 | NP_001001659.1 | 349 | 507 | 177 | OR5B17 | NP_001005489.1 | 389 | 547 |
| 138 | OR2B2 | NP_149046.2 | 350 | 508 | 178 | OR5H1 | NP_001005338.1 | 390 | 548 |
| 139 | OR2B3 | NP_001005226.1 | 351 | 509 | 179 | OR5H2 | NP_001005482.2 | 391 | 549 |
| 140 | OR2B6 | NP_036499.1 | 352 | 510 | 180 | OR5H6 | NP_001005479.2 | 392 | 550 |
| 141 | OR2C1 | NP_036500.2 | 353 | 511 | 181 | OR5H14 | NP_001005514.1 | 393 | 551 |
| 142 | OR2F1 | NP_036501.2 | 354 | 512 | 182 | OR5K3 | NP_001005516.1 | 394 | 552 |
| 143 | OR2F2 | NP_001004685.1 | 355 | 513 | 183 | OR5AK2 | NP_001005323.1 | 395 | 553 |
| 144 | OR2H1 | NP_001304943.1 | 356 | 514 | 184 | OR5AR1 | NP_001004730.1 | 396 | 554 |
| 145 | OR2H2 | NP_009091.3 | 357 | 515 | 185 | OR5AS1 | NP_001001921.1 | 397 | 555 |
| 146 | OR2K2 | NP_995581.1 | 358 | 516 | 186 | OR6B1 | NP_001005281.1 | 398 | 556 |
| 147 | OR2S2 | NP_063950.2 | 359 | 517 | 187 | OR6B3 | NP_775486.1 | 399 | 557 |
| 148 | OR2V1 | NP_001245212.1 | 360 | 518 | 188 | OR6C2 | NP_473446.1 | 400 | 558 |
| 149 | OR2V2 | NP_996763.1 | 361 | 519 | 189 | OR6C3 | NP_473445.1 | 401 | 559 |
| 150 | OR2Z1 | NP_001004699.1 | 362 | 520 | 190 | OR6C4 | NP_001372904.1 | 402 | 560 |
| 151 | OR2AE1 | NP_001005276.1 | 363 | 521 | 191 | OR6C6 | NP_001005493.1 | 403 | 561 |
| 152 | OR2AP1 | NP_001245214.1 | 364 | 522 | 192 | OR6C65 | NP_001005518.1 | 404 | 562 |
| 153 | OR2AT4 | NP_001005285.1 | 365 | 523 | 193 | OR6C68 | NP_001005519.2 | 405 | 563 |
| 154 | OR3A1 | NP_002541.2 | 366 | 524 | 194 | OR6C70 | NP_001005499.1 | 406 | 564 |

**[Table 1-4]**

| No. | (1) Olfactory receptor | Accession No. | (2) SEQ ID NO of amino acid sequence | (3) SEQ ID NO of consensus amino acid sequence | No. | (1) Olfactory receptor | Accession No. | (2) SEQ ID NO of amino acid sequence | (3) SEQ ID NO of consensus amino acid sequence |
|---|---|---|---|---|---|---|---|---|---|
| 195 | OR6C74 | NP_001005490.1 | 407 | 565 | 234 | OR51B5 | NP_001005567.2 | 446 | 604 |
| 196 | OR6C75 | NP_001005497.1 | 408 | 566 | 235 | OR51B6 | NP_001004750.1 | 447 | 605 |
| 197 | OR6C76 | NP_001005183.1 | 409 | 567 | 236 | OR51D1 | NP_001004751.1 | 448 | 606 |
| 198 | OR6K3 | NP_001005327.2 | 410 | 568 | 237 | OR51E2 | NP_110401.1 | 449 | 607 |
| 199 | OR6K6 | NP_001005184.1 | 411 | 569 | 238 | OR51F1 | NP_001004752.2 | 450 | 608 |
| 200 | OR6N2 | NP_001005278.1 | 412 | 570 | 239 | OR51F2 | NP_001004753.2 | 451 | 609 |
| 201 | OR8S1 | NP_001377778.1 | 413 | 571 | 240 | OR51G1 | NP_001005237.1 | 452 | 610 |
| 202 | OR9A2 | NP_001001658.1 | 414 | 572 | 241 | OR51G2 | NP_001005238.1 | 453 | 611 |
| 203 | OR9A4 | NP_001001656.1 | 415 | 573 | 242 | OR51I1 | NP_001005288.1 | 454 | 612 |
| 204 | OR10A3 | NP_001003745.1 | 416 | 574 | 243 | OR51I2 | NP_001004754.1 | 455 | 613 |
| 205 | OR10A7 | NP_001005280.1 | 417 | 575 | 244 | OR51J1 | NP_001335153.1 | 456 | 614 |
| 206 | OR10G6 | NP_001342148.1 | 418 | 576 | 245 | OR51L1 | NP_001004755.1 | 457 | 615 |
| 207 | OR10G9 | NP_001001953.1 | 419 | 577 | 246 | OR51M1 | NP_001004756.2 | 458 | 616 |
| 208 | OR10H1 | NP_039228.1 | 420 | 578 | 247 | OR51Q1 | NP_001004757.1 | 459 | 617 |
| 209 | OR10H2 | NP_039227.1 | 421 | 579 | 248 | OR51S1 | NP_001004758.1 | 460 | 618 |
| 210 | OR10H3 | NP_039226.1 | 422 | 580 | 249 | OR51T1 | NP_001004759.2 | 461 | 619 |
| 211 | OR10H4 | NP_001004465.1 | 423 | 581 | 250 | OR51V1 | NP_001004760.3 | 462 | 620 |
| 212 | OR10H5 | NP_001004466.1 | 424 | 582 | 251 | OR52A1 | NP_036507.2 | 463 | 621 |
| 213 | OR10P1 | NP_996782.1 | 425 | 583 | 252 | OR52A5 | NP_001005160.1 | 464 | 622 |
| 214 | OR10S1 | NP_001004474.1 | 426 | 584 | 253 | OF52B2 | NP_001004052.1 | 465 | 623 |
| 215 | OR10V1 | NP_001005324.1 | 427 | 585 | 254 | OR52B4 | NP_001005161.2 | 466 | 624 |
| 216 | OR10W1 | NP_997257.2 | 428 | 586 | 255 | OR52B6 | NP_001005162.2 | 467 | 625 |
| 217 | OR10AD1 | NP_001004134.1 | 429 | 587 | 256 | OR52D1 | NP_001005163.1 | 468 | 626 |
| 218 | OR10AG1 | NP_001005491.2 | 430 | 588 | 257 | OR52E5 | NP_001005166.3 | 469 | 627 |
| 219 | OR11A1 | NP_039225.1 | 431 | 589 | 258 | OR52E6 | NP_001005167.1 | 470 | 628 |
| 220 | OR11H2 | NP_001184216.2 | 432 | 590 | 259 | OR52H1 | NP_001005289.2 | 471 | 629 |
| 221 | OR12D2 | NP_039224.2 | 433 | 591 | 260 | OR5211 | NP_001005169.1 | 472 | 630 |
| 222 | OR12D3 | NP_112221.1 | 434 | 592 | 261 | OR52I2 | NP_001005170.1 | 473 | 631 |
| 223 | OR13A1 | NP_001004297.2 | 435 | 593 | 262 | OR52J3 | NP_001001916.2 | 474 | 632 |
| 224 | OR13C2 | NP_001004481.1 | 436 | 594 | 263 | OR52K1 | NP_001005171.2 | 475 | 633 |
| 225 | OR13C3 | NP_001001961.2 | 437 | 595 | 264 | OR52K2 | NP_001005172.2 | 476 | 634 |
| 226 | OR13C4 | NP_001001919.1 | 438 | 596 | 265 | OR52N1 | NP_001001913.1 | 477 | 635 |
| 227 | OR13C9 | NP_001001956.1 | 439 | 597 | 266 | OR52R1 | NP_001005177.3 | 478 | 636 |
| 228 | OR13F1 | NP_001004485.1 | 440 | 598 | 267 | OR52W1 | NP_001005178.1 | 479 | 637 |
| 229 | OR51A2 | NP_001004748.1 | 441 | 599 | 268 | OR56A5 | NP_001139505.1 | 480 | 638 |
| 230 | OR51A4 | NP_001005329.1 | 442 | 600 | 269 | OR56B4 | NP_001005181.1 | 481 | 639 |
| 231 | OR51A7 | NP_001004749.1 | 443 | 601 | 270 | OR5AN1 | NP_001004729.1 | 482 | 640 |
| 232 | OR51B2 | NP_149420.4 | 444 | 602 | 271 | M71 | NP_997547.1 | 483 | 641 |
| 233 | OR51B4 | NP_149419.2 | 445 | 603 | 272 | OR9K2 | NP_001005243.2 | 484 | 642 |

**[Table 1-5]**

| No. | (1) Olfactory receptor | Accession No. | (2) SEQ ID NO of amino acid sequence | (3) SEQ ID NO of consensus amino acid sequence | No. | (1) Olfactory receptor | Accession No. | (2) SEQ ID NO of amino acid sequence | (3) SEQ ID NO of consensus amino acid sequence |
|---|---|---|---|---|---|---|---|---|---|
| 273 | OR2A2 | NP_001005480.2 | 801 | 851 | 298 | OR6P1 | NP_001153797.1 | 826 | 876 |
| 274 | OR2A12 | NP_001004135.1 | 802 | 852 | 299 | OR6Q1 | NP_001005186.2 | 827 | 877 |
| 275 | OR4S1 | NP_001004725.1 | 803 | 853 | 300 | OR6T1 | NP_001005187.1 | 828 | 878 |
| 276 | OR5AC2 | NP_473447.1 | 804 | 854 | 301 | OR6X1 | NP_001005188.1 | 829 | 879 |
| 277 | OR5B3 | NP_001005469.1 | 805 | 855 | 302 | OR8B4 | NP_001005196.1 | 830 | 880 |
| 278 | OR5D13 | NP_001001967.1 | 806 | 856 | 303 | OR8B8 | NP_036510.1 | 831 | 881 |
| 279 | OR5D14 | NP_001004735.1 | 807 | 857 | 304 | OR8B12 | NP_001005195.1 | 832 | 882 |
| 280 | OR5D16 | NP_001005496.1 | 808 | 858 | 305 | OR8D1 | NP_001002917.1 | 833 | 883 |
| 281 | OR5D18 | NP_001001952.1 | 809 | 859 | 306 | OR8G1 | NP_001002905.1 | 834 | 884 |
| 282 | OR5J2 | NP_001005492.1 | 810 | 860 | 307 | OR8G5 | NP_001005198.2 | 835 | 885 |
| 283 | OR5K4 | NP_001005517.1 | 811 | 861 | 308 | OR8H2 | NP_001372993.1 | 836 | 886 |
| 284 | OR5L1 | NP_001004738.1 | 812 | 862 | 309 | OR812 | NP_001003750.1 | 837 | 887 |
| 285 | OR5L2 | NP_001004739.1 | 813 | 863 | 310 | OR8J1 | NP_001005205.2 | 838 | 888 |
| 286 | OR5M1 | NP_001004740.1 | 814 | 864 | 311 | OR8K1 | NP_001002907.1 | 839 | 889 |
| 287 | OR5M3 | NP_001004742.2 | 815 | 865 | 312 | OR8K3 | NP_001005202.1 | 840 | 890 |
| 288 | OR5M8 | NP_001005282.1 | 816 | 866 | 313 | OR8K5 | NP_001004058.2 | 841 | 891 |
| 289 | OR5M9 | NP_001004743.1 | 817 | 867 | 314 | OR8U1 | NP_001005204.1 | 842 | 892 |
| 290 | OR5M10 | NP_001004741.1 | 818 | 868 | 315 | OR9G1 | NP_001005213.1 | 843 | 893 |
| 291 | OR5M11 | NP_001005245.1 | 819 | 869 | 316 | OR9G4 | NP_001377761.1 | 844 | 894 |
| 292 | OR5P2 | NP_703145.1 | 820 | 870 | 317 | OR9I1 | NP_001005211.1 | 845 | 895 |
| 293 | OR5T1 | NP_001004745.1 | 821 | 871 | 318 | OR11H12 | NP_001013372.1 | 846 | 896 |
| 294 | OR5T2 | NP_001004746.2 | 822 | 872 | 319 | OR52M1 | NP _001004137.1 | 847 | 897 |
| 295 | OR5T3 | NP_001004747.2 | 823 | 873 | 320 | OR52N5 | NP_001372591.1 | 848 | 898 |
| 296 | OR6M1 | NP_001005325.1 | 824 | 874 | 321 | OR56A3 | NP_001003443.2 | 849 | 899 |
| 297 | OR6N1 | NP_001005185.1 | 825 | 875 | 322 | OR5I1 | NP_006628.1 | 850 | 900 |

In the above Tables 1-1 to 1-5, the olfactory receptor polypeptide consisting of an amino acid sequence of any of SEQ ID NOs: 97, 104, 107, 119, 122, 131, 134, 137, and 146 in (3) is an olfactory receptor polypeptide consisting of the consensus amino acid sequence used in the consensus design of the olfactory receptors (a). The olfactory receptor polypeptide consisting of an amino acid sequence of any of SEQ ID NOs: 98, 105, 108, 120, 123, 132, 135, 138, and 147 is an olfactory receptor polypeptide consisting of the consensus amino acid sequence used in the consensus design of the olfactory receptors (b). The olfactory receptor polypeptide consisting of an amino acid sequence of any of SEQ ID NOs: 99 to 103, 106, 109 to 118, 121, 124 to 130, 133, 136, 139 to 144, 148 to 167, 169 to 178, 180 to 210, 485, 486, 488 to 524, 526 to 589, 591 to 598, 600 to 627, 629 to 642, 850 to 885, and 887 to 900 is an olfactory receptor polypeptide consisting of the consensus amino acid sequence used in the consensus design of the olfactory receptors (c). Among them, the olfactory receptor polypeptide consisting of an amino acid sequence of SEQ ID NO: 210 or 900 is an olfactory receptor polypeptide consisting of the consensus amino acid sequence obtained by changing a consensus residue nearest to the N terminus among the consensus residues to a consensus residue consisting of a methionine residue in the criterion (ii), and the olfactory receptor polypeptide consisting of an amino acid sequence of SEQ ID NO: 642 is an olfactory receptor polypeptide consisting of the consensus amino acid sequence obtained by maintaining an N-terminal structure of the olfactory receptor of interest as it is in the criterion (i), (i-iv). The olfactory receptor polypeptide consisting of an amino acid sequence of any of SEQ ID NOs: 145, 168, 179, 487, 525, 590, 599, 628, and 886 is an olfactory receptor polypeptide consisting of the consensus amino acid sequence used in the consensus design of the olfactory receptors (d).

The "expression" of the olfactory receptor polypeptide of the present invention means that a translation product is produced from a polynucleotide encoding the polypeptide and the translation product is localized in a functional state on a cell membrane which is a site of its action. The olfactory receptor polypeptide of the present invention can be expressed in cells by an approach known in the art. For example, the olfactory receptor polypeptide can be expressed on cell membranes of cells by transferring a vector containing a polynucleotide encoding the polypeptide to the cells serving as a host, or by transferring a DNA fragment containing a polynucleotide encoding the polypeptide to the genome of the cells serving as a host. Preferably, the olfactory receptor polypeptide is expressed on cell membranes of host cells by transforming the host cells using a vector containing a polynucleotide encoding the polypeptide. The host cells may not be limited as long as the cells can functionally express a foreign olfactory receptor. Specific examples of the cells include, but are not limited to, human embryonic kidney cells (HEK293 cells), Chinese hamster cells (CHO cells), monkey cells (COS cells), isolated olfactory neurons, *Xenopus* oocytes, insect cells, yeasts, and bacteria.

The polynucleotide encoding the olfactory receptor polypeptide can be obtained by use of various mutagenesis techniques known in the art. The polynucleotide encoding the olfactory receptor polypeptide can be obtained, for example, by altering a nucleotide sequence encoding the amino acid residue to be altered to a nucleotide sequence encoding an amino acid residue after alteration in a polynucleotide encoding the amino acid sequence of the olfactory receptor of interest.

A mutation of interest can be introduced to the polynucleotide encoding the amino acid sequence of the olfactory receptor of interest by use of various site-directed mutagenesis techniques well known to those skilled in the art. The site-directed mutagenesis techniques can be performed by an arbitrary approach, for example, inverse PCR or annealing. A commercially available kit for site-directed mutagenesis (e.g., QuickChange II Site-Directed Mutagenesis Kit or QuickChange Multi Site-Directed Mutagenesis Kit from Stratagene California) may be used.

Alternatively, the polynucleotide encoding the olfactory receptor polypeptide can also be obtained by genome editing using artificial DNA nucleases or programmable nuclease, DNA synthesis based on the nucleotide sequence, or the like.

The polynucleotide encoding the olfactory receptor polypeptide can contain single-stranded or doublestranded DNA, cDNA, RNA, or other artificial nucleic acids. The DNA, the cDNA, and the RNA may be chemically synthesized. The polynucleotide may contain nucleotide sequences of an open reading frame (ORF) as well as an untranslated region (UTR). The polynucleotide may be codon-optimized for the species of cells for olfactory receptor expressions. Information on codons for use in various organisms is available from Codon Usage Database ([www.kazusa.or.jp/codon/]).

In a preferred example, the polynucleotide encoding the olfactory receptor polypeptide consists of a nucleotide sequence of any of SEQ ID NOs: 211 to 324, 643 to 800, and 901 to 950. These polynucleotides encode olfactory receptor polypeptides consisting of amino acid sequences of SEQ ID NOs: 97 to 210, 485 to 642, and 851 to 900, respectively. In a more preferred example, the polynucleotide encoding the olfactory receptor polypeptide consists of a nucleotide sequence of any of SEQ ID NOs: 213 to 218, 223 to 232, 235, 237, 239 to 244, 247, 250, 252, 254 to 260, 263 to 324, 643 to 800, and 901 to 950. These polynucleotides encode olfactory receptor polypeptides consisting of amino acid sequences of SEQ ID NOs: 99 to 104, 109 to 118, 121, 123, 125 to 130, 133, 136, 138, 140 to 146, 149 to 210, 485 to 642, and 851 to 900, respectively.

The obtained polynucleotide encoding the olfactory receptor polypeptide can be incorporated into a vector or a DNA fragment. Preferably, the vector is an expression vector. Also preferably, the vector is an expression vector which can transfer the olfactory receptor polynucleotide to host cells and enables the polynucleotide to be expressed in the host cells. Preferably, the vector may be a vector, such as a plasmid, capable of autonomously proliferating or replicating extrachromosomally, or may be a vector which is integrated into the chromosome. Specific examples of the vector include, but are not limited to, pME18S.

The vector preferably contains the polynucleotide encoding the olfactory receptor polypeptide, and a control region operably linked thereto. The control region is a sequence for expressing the transferred polynucleotide encoding the olfactory receptor polypeptide in host cells harboring the vector. Examples thereof include expression regulatory regions such as promoters and terminators, and transcription initiation points. The type of the control region can be appropriately selected depending on the type of the vector. The vector or the DNA fragment may optionally further contain a drug (e.g., ampicillin) resistance gene as a selective marker. The control region and the selective marker gene originally contained in the vector may be used, or the control region and the selective marker gene may be incorporated, together with or separately from the polynucleotide encoding the olfactory receptor polypeptide, into the vector.

Examples of the DNA fragment containing the polynucleotide encoding the olfactory receptor polypeptide include PCR-amplified DNA fragments and restriction enzyme-cleaved DNA fragments. Preferably, the DNA fragment can be an expression cassette containing the polynucleotide and a control region operably linked thereto. Examples of the control region that can be used are the same as in the case of the vector.

Preferably, a polynucleotide encoding RTP (receptor-transporting protein) (in the present specification, this polynucleotide is also referred to as RTP gene) is transferred, together with the polynucleotide encoding the olfactory receptor polypeptide, into cells in order to promote the cell membrane expression of the olfactory receptor polypeptide. For example, a vector containing the RTP gene and the polynucleotide encoding the olfactory receptor polypeptide may be constructed and transferred into host cells, or a vector containing the RTP gene and a vector containing the polynucleotide encoding the olfactory receptor polypeptide may be transferred into host cells. Examples of the RTP include RTP1S, and examples of the RTP1S include human RTP1S. The human RTP1S is registered as AY562235 in GenBank and is a polypeptide which consists of an amino acid sequence of SEQ ID NO: 326 and is encoded by a gene having a nucleotide sequence of SEQ ID NO: 325.

A general transformation technique for mammalian cells, for example, an electroporation technique, a lipofection technique, or a particle gun technique, can be used in the transfer of the vector or the DNA fragment into host cells. Transformed cells harboring the vector or the DNA fragment of interest can be selected by exploiting the selective marker. Alternatively, the transfer of the vector or the DNA fragment of interest may be confirmed by examining the DNA sequences of the cells.

The olfactory receptor polypeptide of the present invention is produced from the polynucleotide encoding the olfactory receptor polypeptide contained in the vector or the DNA fragment transferred into cells by the procedures described above, and integrated to cell membranes. Accordingly, the olfactory receptor polypeptide expressed by the expression method of the present invention is expressed on cell membranes of transformed cells genetically manipulated so as to express the olfactory receptor polypeptide.

The cell membrane expression (level) of the olfactory receptor polypeptide of the present invention can be measured, for example, by fusing a tag such as a FLAG tag with the olfactory receptor polypeptide in advance, and using an antibody which specifically recognizes the tag in an approach known in the art such as flow cytometry.

The olfactory receptor polypeptide expressed by the expression method of the present invention is improved in odor responsiveness in comparison with the original olfactory receptor and well maintains the ligand selectivity of the original olfactory receptor. Accordingly, a response of the olfactory receptor polypeptide to an odorant can be regarded as reflecting a response of the original olfactory receptor to olfactory cells. Thus, in an alternative aspect, the present invention provides a method for measuring a response of an olfactory receptor of interest. The method includes measuring a response of an olfactory receptor polypeptide expressed by the method for expressing an olfactory receptor polypeptide according to the present invention. The response measurement method of the present invention can improve the response measurement efficiency of an olfactory receptor of interest and enables response measurement of an olfactory receptor of interest never before possible to functionally analyze due to insufficient cell membrane expression in cultured cells.

The olfactory receptor polypeptide for use in the response measurement method of the present invention may be an olfactory receptor polypeptide expressed by the expression method of the present invention. The olfactory receptor polypeptide is as mentioned above. Specific examples of the olfactory receptor polypeptide preferably include an olfactory receptor polypeptide consisting of an amino acid sequence of any of SEQ ID NOs: 97 to 210, 485 to 642, and 851 to 900, and more preferably include an olfactory receptor polypeptide consisting of an amino acid sequence of any of SEQ ID NOs: 99 to 104, 109 to 118, 121, 123, 125 to 130, 133, 136, 138, 140 to 146, 149 to 210, 485 to 642, and 851 to 900. The olfactory receptor polypeptide can be used in an arbitrary form without losing its responsiveness to an odorant. The olfactory receptor polypeptide can be used in the form of, for example, a transformed cell expressing the olfactory receptor polypeptide or its cultures, a membrane of the transformed cell having the olfactory receptor polypeptide, or an artificial lipid bilayer having the olfactory receptor polypeptide. Preferably, a transformed cell expressing the olfactory receptor polypeptide or its cultures are used as the olfactory receptor polypeptide.

In the response measurement method of the present invention, the measurement of the response of the olfactory receptor polypeptide may be performed by an arbitrary method known in the art as a method for measuring responses of olfactory receptors, for example, intracellular cAMP level measurement. For example, it is known that olfactory receptors, when activated by odor molecules, are coupled to G protein α subunit classified into the Gs family in cells to activate adenylate cyclase, thereby increasing intracellular cAMP levels (Nat. Neurosci., 2004, 5: 263-278). Meanwhile, the olfactory receptors, when activated by odor molecules, can also be coupled to proteins belonging to the Gq family, such as Gα15, in cells, thereby increasing calcium ion levels in the cells. Thus, the response of the olfactory receptor polypeptide can be measured by using, as an index, an intracellular cAMP level or a calcium ion level after odor molecule addition, or a behavior of a downstream molecule activated via such a level. Examples of a method for measuring the cAMP level include ELISA and reporter gene assay. Examples of a method for measuring the calcium ion concentration include calcium imaging techniques and TGFα shedding assay. As an example of a method using the behavior of the downstream molecule activated via the cAMP level as an index, a two-electrode voltage clamp technique is also effective which measures potential change inside and outside cell membranes via a cystic fibrosis transmembrane conductance regulator CFTR activated by cAMP signals in *Xenopus* oocytes.

As mentioned above, the response of the olfactory receptor polypeptide expressed by the expression method of the present invention can be regarded as reflecting a response of the original olfactory receptor to olfactory cells. Therefore, a ligand for the original olfactory receptor can be searched for using the olfactory receptor polypeptide. Thus, in an alternative aspect, the present invention provides a method for searching for a ligand for an olfactory receptor of interest. The method includes: measuring a response of an olfactory receptor polypeptide expressed by the expression method of the present invention in the presence of a test substance; and selecting a test substance to which the olfactory receptor polypeptide has responded. The ligand search method of the present invention can improve search efficiency of a ligand for an olfactory receptor of interest and enables search for a ligand for an olfactory receptor of interest never before possible to functionally analyze due to insufficient cell membrane expression in cultured cells.

The test substance for use in the ligand search method of the present invention is not particularly limited as long as it is a substance for which it is desired to confirm whether or not to be a ligand for an olfactory receptor of interest. The test substance may be a naturally occurring substance or a substance artificially synthesized by a chemical or biological method or the like, or may be a compound, a composition, or a mixture.

The olfactory receptor polypeptide for use in the ligand search method of the present invention and its form are the same as the olfactory receptor polypeptide for use in the response measurement method of the present invention and its form.

In the ligand search method of the present invention, the test substance is applied to the olfactory receptor polypeptide expressed by the expression method of the present invention. Examples of an approach of applying the test substance to the olfactory receptor polypeptide include, but are not particularly limited to, a method of adding the test substance to a medium for culture of cells expressing the olfactory receptor polypeptide.

In the ligand search method of the present invention, a response of the olfactory receptor polypeptide to the test substance is measured following the addition of the test substance to the olfactory receptor polypeptide. The same approach as that for the response measurement method of the present invention can be used as a method for measuring the response.

Subsequently, the test substance is evaluated on the basis of the measured response of the olfactory receptor polypeptide. A test substance which causes the response of the olfactory receptor polypeptide can be determined as a ligand for the olfactory receptor polypeptide, i.e., a ligand for the original olfactory receptor. Thus, in the ligand search method of the present invention, a test substance to which the olfactory receptor polypeptide has responded is selected as a ligand for the original olfactory receptor.

Preferably, the response of the olfactory receptor polypeptide to the test substance can be evaluated by comparing the response of the olfactory receptor polypeptide supplemented with the test substance (test group) with a response of the olfactory receptor polypeptide in a control group. Examples of the control group can include the olfactory receptor polypeptide non-supplemented with the test substance, the olfactory receptor polypeptide supplemented with a control substance, the olfactory receptor polypeptide supplemented with a lower concentration of the test substance, and the olfactory receptor polypeptide before addition of the test substance. When the response in the test group is higher than that in the control group, the olfactory receptor polypeptide is evaluated as having responded to the test substance and the test substance is selected as a ligand for the original olfactory receptor.

Thus, in one embodiment of the ligand search method of the present invention, a response of the olfactory receptor polypeptide is measured in the presence and in the absence of a test substance, and subsequently, whether or not the response in the presence of the test substance is higher than that in the absence of the test substance is determined. When the response in the presence of the test substance is higher, the test substance is selected as a ligand. In a preferred embodiment, provided that the response intensity of the olfactory receptor polypeptide in the presence of the test substance is preferably 120% or more, more preferably 150% or more, further more preferably 200% or more, in comparison with that in the absence of the test substance, the test substance is selected as a ligand for the original olfactory receptor. In another preferred embodiment, provided that the response intensity of the olfactory receptor polypeptide in the presence of the test substance is statistically significantly increased in comparison with that in the absence of the test substance, the test substance is selected as a ligand for the original olfactory receptor.

As mentioned above, the response of the olfactory receptor polypeptide expressed by the expression method of the present invention can be regarded as reflecting a response of the original olfactory receptor to olfactory cells. Therefore, a substance which suppresses the odor recognition of a ligand (odorant) by the original olfactory receptor can be evaluated and/or selected using the olfactory receptor polypeptide. A substance which suppresses the response of the olfactory receptor polypeptide can change the ligand response of the olfactory receptor polypeptide, i.e., change the ligand response of the original olfactory receptor, and consequently selectively suppress odor of the ligand on the basis of olfactory receptor antagonism. On the other hand, a substance which enhances the response of the olfactory receptor polypeptide can change the ligand response of the olfactory receptor polypeptide, i.e., change the ligand response of the original olfactory receptor, and consequently selectively suppress odor of the ligand on the basis of odor cross-adaptation ascribable to olfactory receptor agonism.

Thus, in an alternative aspect, the present invention provides a method for evaluating and/or selecting a suppressor of odor of a ligand for an olfactory receptor of interest. The method includes measuring a response of the olfactory receptor polypeptide after test substance addition. A test substance which suppresses or enhances the response of the olfactory receptor polypeptide is detected on the basis of the measured response. The detected test substance is selected as a suppressor of odor of the target ligand. Specifically, a test substance which suppresses the response of the olfactory receptor polypeptide is selected as a suppressor of odor of the ligand based on olfactory receptor antagonism, and a test substance which enhances the response of the olfactory receptor polypeptide is selected as a suppressor of odor of the ligand based on odor cross-adaptation ascribable to olfactory receptor agonism. The ligand for an olfactory receptor of interest is preferably a ligand selected by the ligand search method of the present invention. The odor suppressor evaluation and/or selection method of the present invention can efficiently evaluate or select a substance which can selectively suppress odor of the target ligand, and can evaluate or select a substance which can selectively suppress odor of the target ligand even if the target ligand is a ligand for an olfactory receptor never before possible to functionally analyze due to insufficient cell membrane expression in cultured cells.

The odor suppressor evaluation and/or selection of the present invention can be a method which is performed *in vitro* or *ex vivo.*

The test substance for use in the odor suppressor evaluation and/or selection method of the present invention is not particularly limited as long as the substance is desired to be used as a suppressor of odor of the target ligand. The test substance may be a naturally occurring substance or a substance artificially synthesized by a chemical or biological method or the like, or may be a compound, a composition, or a mixture.

The olfactory receptor polypeptide for use in the odor suppressor evaluation and/or selection method of the present invention and its form are the same as the olfactory receptor polypeptide for use in the response measurement method of the present invention and its form.

In the odor suppressor evaluation and/or selection method of the present invention, the test substance is applied to the olfactory receptor polypeptide expressed by the expression method of the present invention. Examples of an approach of applying the test substance to the olfactory receptor polypeptide include, but are not particularly limited to, a method of adding the test substance to a medium for culture of cells expressing the olfactory receptor polypeptide.

In the odor suppressor evaluation and/or selection method of the present invention, a response of the olfactory receptor polypeptide to the test substance is measured following the addition of the test substance to the olfactory receptor polypeptide. The same approach as that for the response measurement method of the present invention can be used as a method for measuring the response.

In a first embodiment, the odor suppressor evaluation and/or selection method of the present invention includes: adding a test substance and a target ligand to an olfactory receptor polypeptide expressed by the expression method of the present invention; and measuring a response of the olfactory receptor polypeptide to the ligand. The same approach as that for the method for applying the test substance can be used as a method for applying the ligand to the olfactory receptor polypeptide. Subsequently, a test substance which suppresses the response of the olfactory receptor polypeptide to the ligand is detected on the basis of the measured response. The detected test substance is selected as a suppressor of odor of the ligand.

In the first embodiment, the test substance which suppresses the response of the olfactory receptor polypeptide to the ligand is selected as a suppressor of odor of the ligand based on olfactory receptor antagonism. The action of the test substance on the response of the olfactory receptor polypeptide to the ligand can be evaluated, for example, by comparing the response of the olfactory receptor polypeptide supplemented with the test substance (test group) to the ligand with a response to the ligand in a control group. Examples of the control group can include the olfactory receptor polypeptide non-supplemented with the test substance, the olfactory receptor polypeptide supplemented with a control substance, the olfactory receptor polypeptide supplemented with a lower concentration of the test substance, the olfactory receptor polypeptide before addition of the test substance, and cells which do not express the olfactory receptor polypeptide. Preferably, the odor suppressor evaluation and/or selection method of the present invention according to the first embodiment includes measuring activity of the olfactory receptor polypeptide against the ligand in the presence and in the absence of the test substance.

For example, when the response in the test group is more suppressed than the response in the control group, the test substance can be identified as a substance which suppresses the response of the olfactory receptor polypeptide to the target ligand, i.e., a substance which suppresses the response of the original olfactory receptor to the ligand. For example, provided that the response of the olfactory receptor polypeptide in the test group is suppressed to preferably 60% or less, more preferably 50% or less, further more preferably 25% or less, in comparison with the control group, the test substance can be identified as a substance which suppresses the response of the olfactory receptor polypeptide to the ligand, i.e., a substance which suppresses the response of the original olfactory receptor to the ligand. Alternatively, provided that the response of the olfactory receptor polypeptide in the test group is statistically significantly suppressed in comparison with the control group, the test substance can be identified as a substance which suppresses the response of the olfactory receptor polypeptide to the ligand, i.e., a substance which suppresses the response of the original olfactory receptor to the ligand.

In a second embodiment, the odor suppressor evaluation and/or selection method of the present invention includes: adding a test substance to an olfactory receptor polypeptide expressed by the expression method of the present invention; and measuring a response of the olfactory receptor polypeptide to the test substance. Subsequently, a test substance which enhances the response of the olfactory receptor polypeptide to the target ligand is detected on the basis of the measured response. The detected test substance is selected as a suppressor of odor of the ligand.

The test substance which enhances the response of the olfactory receptor polypeptide can first enhance the response of the olfactory receptor, thereby weakening the response of the olfactory receptor when later exposed to the target ligand. As a result, the odor recognition of the ligand by an individual can be suppressed on the basis of odor cross-adaptation. Thus, in the second embodiment, a suppressor of odor of the ligand based on odor cross-adaptation ascribable to olfactory receptor agonism is selected.

The action of the test substance on the olfactory receptor polypeptide can be evaluated, for example, by comparing the response of the olfactory receptor polypeptide supplemented with the test substance (test group) with a response in a control group. Examples of the control group include those mentioned above. Preferably, the odor suppressor evaluation and/or selection method of the present invention according to the second embodiment includes measuring activity of the olfactory receptor polypeptide in the presence and in the absence of the test substance. Also preferably, the odor suppressor evaluation and/or selection method of the present invention according to the second embodiment includes measuring responses of a cell expressing the olfactory receptor polypeptide and a non-expressing cell to the ligand in the presence of the test substance.

For example, when the response in the test group is enhanced in comparison with the control group, the test substance can be identified as a substance which suppresses the response of the olfactory receptor polypeptide to the target ligand, i.e., a substance which suppresses the response of the original olfactory receptor to the ligand. For example, provided that the response of the olfactory receptor polypeptide in the test group is enhanced to preferably 120% or more, more preferably 150% or more, further more preferably 200%, in comparison with the control group, the test substance can be identified as a substance which suppresses the response of the olfactory receptor polypeptide to the ligand, i.e., a substance which suppresses the response of the original olfactory receptor to the ligand. Alternatively, provided that the response of the olfactory receptor polypeptide in the test group is statistically significantly enhanced in comparison with the control group, the test substance can be identified as a substance which suppresses the response of the olfactory receptor polypeptide to the ligand, i.e., a substance which suppresses the response of the original olfactory receptor to the ligand.

The test substance identified by the procedures described above is a substance which can suppress the response of the olfactory receptor to the target ligand, thereby suppressing the odor recognition of the ligand by an individual. Thus, the test substance identified by the procedures described above can be selected as a suppressor of odor of the ligand. The substance selected as a suppressor of odor of the target ligand by the odor suppressor evaluation and/or selection method of the present invention can suppress odor of the ligand by suppressing the response of the olfactory receptor to the ligand.

Thus, in one embodiment, the substance selected by the ligand odor suppressor evaluation and/or selection method of the present invention can be an active ingredient for a suppressor of odor of the ligand. Alternatively, the substance selected by the ligand odor suppressor evaluation and/or selection method of the present invention can be contained as an active ingredient for suppressing odor of the ligand in a compound or a composition for suppressing odor of the ligand. Alternatively, the substance selected by the ligand odor suppressor evaluation and/or selection method of the present invention can be used for producing a suppressor of odor of the ligand or for producing a compound or a composition for suppressing odor of the ligand. The substance can eliminate odor of the target ligand without causing problems, such as discomfort based on strong odor of an aromatic agent and suppression of other odors, which arise in an odor elimination method using a conventional deodorant or aromatic agent.

Hereinafter, one example of the method for searching for a ligand for an olfactory receptor of interest and the method for evaluating and/or selecting a suppressor of odor of a ligand for an olfactory receptor of interest according to the present invention will be described.

As shown in Examples mentioned later, as a result of measuring a response of each consensus olfactory receptor expressed by the expression method of the present invention in the presence of methyl mercaptan, dimethyl sulfide, dimethyl disulfide, or dimethyl trisulfide, all of which are sulfur compounds, consensus OR2T29, consensus OR4K17, consensus OR2T27, consensus OR2T5, consensus OR2T4, consensus OR11H2, consensus OR6B3, consensus OR12D3, consensus OR2T11, consensus OR2T1, consensus OR4C15, consensus OR4E2, and consensus OR2L13 responded to methyl mercaptan in the presence of a metal ion (Figure 10). Consensus OR4K17, consensus OR11H2, consensus OR4C15, and consensus OR4E2 responded to dimethyl sulfide in the presence of a metal ion (Figure 10). Consensus OR2T29, consensus OR4K17, consensus OR2T27, consensus OR2T5, consensus OR2T4, consensus OR11H2, consensus OR6B3, consensus OR12D3, consensus OR2T11, consensus OR2T1, consensus OR4C15, consensus OR4E2, and consensus OR2L13 responded to dimethyl disulfide in the presence of a metal ion (Figure 10). Consensus OR2T29, consensus OR2T4, consensus OR11H2, consensus OR6B3, consensus OR12D3, consensus OR6B1, consensus OR2T11, consensus OR2T1, consensus OR4C15, consensus OR4E2, and consensus OR2L13 responded to dimethyl trisulfide in the presence of a metal ion (Figure 10). Each sulfur compound which causes the response of each of the consensus olfactory receptors is a ligand for each of the consensus olfactory receptors, i.e., a ligand for the original olfactory receptor from which each of the consensus olfactory receptors is derived.

It has not been recognized so far that OR2T29, OR4K17, OR2T27, OR2T5, OR2T4, OR11H2, OR6B3, OR12D3, and OR6B1 respond to a sulfur compound, particularly, in the presence of a metal ion, or there is that possibility. Meanwhile, it is known that OR2T11 and OR2T1 respond to methyl mercaptan in the presence of a metal ion (JP-B-6122181); OR4C15 and OR2L13 respond to dimethyl trisulfide (JP-A-2020-510436); OR4E2 responds to 3-mercapto-3-methylbutanol and diallyl trisulfide in the presence of a metal ion (JP-A-2020-513564); and OR2T11 responds to dimethyl disulfide, methanethiol, ethanethiol, 1-propanethiol, 2-propanethiol, 1-butanethiol, 2-methyl-2-propanethiol, 2-methyl-1-propanethiol, 2-butanethiol, 3-methyl-2-butanethiol, 2-pentanethiol, and cyclopentanethiol (Block E et al., Nat. Prod. Rep., 34 (5): 529-557 (2017)). Nonetheless, it has not been known so far that these receptors respond to other sulfur compounds, particularly, in the presence of a metal ion.

Accordingly, OR2T29, OR4K17, OR2T27, OR2T5, OR2T4, OR11H2, OR6B3, OR12D3, and OR6B1 are newly found sulfur compound receptors. In other words, sulfur compounds are newly found ligands for OR2T29, OR4K17, OR2T27, OR2T5, OR2T4, OR11H2, OR6B3, OR12D3, and OR6B1. OR4C15, OR4E2, and OR2L13 are newly found methyl mercaptan receptors; OR4C15 and OR4E2 are newly found dimethyl sulfide receptors; OR2T1, OR4C15, OR4E2, and OR2L13 are newly found dimethyl disulfide receptors; and OR2T11, OR2T1, and OR4E2 are newly found dimethyl trisulfide receptor. In other words, methyl mercaptan is a newly found ligand for OR4C15, OR4E2, and OR2L13; dimethyl sulfide is a newly found ligand for OR4C15 and OR4E2; dimethyl disulfide is a newly found ligand for OR2T1, OR4C15, OR4E2, and OR2L13; and dimethyl trisulfide is a newly found ligand for OR2T11, OR2T1, and OR4E2.

Thus, in accordance with the method for evaluating and/or selecting a suppressor of odor of a ligand for an olfactory receptor of interest according to the present invention, a suppressor of odor caused by a sulfur compound can be evaluated and/or selected by measuring a response of at least one olfactory receptor polypeptide selected from the group consisting of consensus OR2T29, consensus OR4K17, consensus OR2T27, consensus OR2T5, consensus OR2T4, consensus OR11H2, consensus OR6B3, consensus OR12D3, and consensus OR6B1 after test substance addition in the presence of a metal ion. In the method, a response of at least one olfactory receptor polypeptide selected from the group consisting of consensus OR2T11, OR4S2, consensus OR2T1, consensus OR4C15, consensus OR4E2, and consensus OR2L13 may be further measured. More specifically, a suppressor of odor caused by methyl mercaptan can be evaluated and/or selected by measuring a response of at least one olfactory receptor polypeptide selected from the group consisting of consensus OR4C15, consensus OR4E2, and consensus OR2L13 after test substance addition in the presence of a metal ion. A suppressor of odor caused by dimethyl sulfide can be evaluated and/or selected by measuring a response of at least one olfactory receptor polypeptide selected from the group consisting of consensus OR4C15 and consensus OR4E2 after test substance addition in the presence of a metal ion. A suppressor of odor caused by dimethyl disulfide can be evaluated and/or selected by measuring a response of at least one olfactory receptor polypeptide selected from the group consisting of consensus OR2T1, consensus OR4C15, consensus OR4E2, and consensus OR2L13 after test substance addition in the presence of a metal ion. A suppressor of odor caused by dimethyl trisulfide can be evaluated and/or selected by measuring a response of at least one olfactory receptor polypeptide selected from the group consisting of consensus OR2T11, consensus OR2T1, and consensus OR4E2 after test substance addition in the presence of a metal ion.

In this context, the "sulfur compound" is a general name for a compound containing sulfur. Preferably, the sulfur compound is a thiol or sulfide compound. More preferred examples of the sulfur compound include methyl mercaptan, dimethyl sulfide, dimethyl disulfide, and dimethyl trisulfide.

The "odor caused by a sulfur compound" is odor resulting from the sulfur compound mentioned above, preferably odor resulting from a thiol or sulfide compound, more preferably odor resulting from at least one compound selected from the group consisting of methyl mercaptan, dimethyl sulfide, dimethyl disulfide, and dimethyl trisulfide. For example, the odor of methyl mercaptan is included in bad odor coming from a permanent agent, odor of feces, body odor, bad breath, armpit odor, aging odor, old person's smell, smell of garbage, and the like. The odor of dimethyl sulfide, dimethyl disulfide, or dimethyl trisulfide is included in bad odor coming from a permanent agent, bad breath, smell of garbage, sewage odor, bad odor coming from a drainage, and the like. Thus, the "odor caused by a sulfur compound" can be typically bad odor coming from a permanent agent, odor of feces, body odor, bad breath, armpit odor, aging odor, old person's smell, smell of garbage, sewage odor, or bad odor coming from a drainage and is preferably bad odor of a permanent agent or bad breath. The odor suppressor evaluation and/or selection method of the present invention can efficiently evaluate or select a substance which can selectively suppress bad odor, odor caused by a sulfur compound.

As mentioned above, the response of the olfactory receptor polypeptide expressed by the expression method of the present invention can be regarded as reflecting a response of the original olfactory receptor to olfactory cells. Therefore, a substance which enhances the odor recognition of a ligand (odorant) by the original olfactory receptor can be evaluated and/or selected using the olfactory receptor polypeptide. A substance which enhances the response of the olfactory receptor polypeptide can change the ligand response of the olfactory receptor polypeptide, i.e., change the ligand response of the original olfactory receptor, and consequently selectively enhance odor of the ligand.

Thus, in an alternative aspect, the present invention provides a method for evaluating and/or selecting an enhancer of odor of a ligand for an olfactory receptor of interest. The method includes: adding a test substance and a target ligand to an olfactory receptor polypeptide expressed by the expression method of the present invention; and measuring a response of the olfactory receptor polypeptide to the ligand. The ligand for an olfactory receptor of interest is preferably a ligand selected by the ligand search method of the present invention. The enhancer evaluation and/or selection method of the present invention can efficiently evaluate or select a substance which can selectively enhance odor of the target ligand, and can evaluate or select a substance which can selectively enhance odor of the target ligand even if the target ligand is a ligand for an olfactory receptor never before possible to functionally analyze due to insufficient cell membrane expression in cultured cells.

The odor enhancer evaluation and/or selection of the present invention can be a method which is performed *in vitro* or ex *vivo.*

The test substance for use in the odor enhancer evaluation and/or selection method of the present invention is not particularly limited as long as the substance is desired to be used as an enhancer of odor of the target ligand. The test substance may be a naturally occurring substance or a substance artificially synthesized by a chemical or biological method or the like, or may be a compound, a composition, or a mixture.

The olfactory receptor polypeptide for use in the odor enhancer evaluation and/or selection method of the present invention and its form are the same as the olfactory receptor polypeptide for use in the response measurement method of the present invention and its form.

In the odor enhancer evaluation and/or selection method of the present invention, the test substance and the target ligand are applied to the olfactory receptor polypeptide expressed by the expression method of the present invention. Examples of an approach of applying the test substance and the target ligand to the olfactory receptor polypeptide include, but are not particularly limited to, a method of adding the test substance and the target ligand to a medium for culture of cells expressing the olfactory receptor polypeptide.

In the odor enhancer evaluation and/or selection method of the present invention, a response of the olfactory receptor polypeptide to the ligand is measured following the addition of the test substance and the target ligand to the olfactory receptor polypeptide. The same approach as that for the response measurement method of the present invention can be used as a method for measuring the response.

Subsequently, a test substance which enhances the response of the olfactory receptor polypeptide to the ligand is detected on the basis of the measured response. The detected test substance is selected as an enhancer of odor of the ligand.

The test substance which enhances the response of the olfactory receptor polypeptide to the ligand is selected as an enhancer of odor of the ligand. The action of the test substance on the response of the olfactory receptor polypeptide to the ligand can be evaluated, for example, by comparing the response of the olfactory receptor polypeptide supplemented with the test substance (test group) to the ligand with a response to the ligand in a control group. Examples of the control group can include the olfactory receptor polypeptide non-supplemented with the test substance, the olfactory receptor polypeptide supplemented with a control substance, the olfactory receptor polypeptide supplemented with a lower concentration of the test substance, the olfactory receptor polypeptide before addition of the test substance, and cells which do not express the olfactory receptor polypeptide. Preferably, the odor enhancer evaluation and/or selection method of the present invention includes measuring activity of the olfactory receptor polypeptide against the ligand in the presence and in the absence of the test substance.

For example, when the response in the test group is more enhanced than the response in the control group, the test substance can be identified as a substance which enhances the response of the olfactory receptor polypeptide to the target ligand, i.e., a substance which enhances the response of the original olfactory receptor to the ligand. For example, provided that the response of the olfactory receptor polypeptide in the test group is enhanced to preferably 120% or more, more preferably 150% or more, further more preferably 200% or more, in comparison with the control group, the test substance can be identified as a substance which enhances the response of the olfactory receptor polypeptide to the ligand, i.e., a substance which enhances the response of the original olfactory receptor to the ligand. Alternatively, provided that the response of the olfactory receptor polypeptide in the test group is statistically significantly enhanced in comparison with the control group, the test substance can be identified as a substance which enhances the response of the olfactory receptor polypeptide to the ligand, i.e., a substance which enhances the response of the original olfactory receptor to the ligand.

The test substance identified by the procedures described above is a substance which can enhance the response of the olfactory receptor to the target ligand, thereby enhancing the odor recognition of the ligand by an individual. Thus, the test substance identified by the procedures described above can be selected as an enhancer of odor of the ligand. The substance selected as an enhancer of odor of the target ligand by the odor enhancer evaluation and/or selection method of the present invention can enhance odor of the ligand by enhancing the response of the olfactory receptor to the ligand.

Thus, in one embodiment, the substance selected by the ligand odor enhancer evaluation and/or selection method of the present invention can be an active ingredient for an enhancer of odor of the ligand. Alternatively, the substance selected by the ligand odor enhancer evaluation and/or selection method of the present invention can be contained as an active ingredient for enhancing odor of the ligand in a compound or a composition for enhancing odor of the ligand. Alternatively, the substance selected by the ligand odor enhancer evaluation and/or selection method of the present invention can be used for producing an enhancer of odor of the ligand or for producing a compound or a composition for enhancing odor of the ligand. The substance can enhance favorable odor of the target ligand, for example.

The present specification further discloses the following compositions, production methods, uses, or methods as exemplary embodiments of the present invention. However, the present invention is not limited by these embodiments.

[1] A method for expressing an olfactory receptor polypeptide, comprising
expressing, in a cell, an olfactory receptor polypeptide consisting of an amino acid sequence obtained by, in an amino acid sequence of an olfactory receptor of interest, altering at least one amino acid residue different from that in a consensus amino acid sequence to an amino acid residue at a position corresponding thereto in the consensus amino acid sequence, wherein
the consensus amino acid sequence is an amino acid sequence derived by alignment of the amino acid sequence of the olfactory receptor of interest and amino acid sequences of any of the following olfactory receptors (a) to (d):
   (a) at least 11 olfactory receptors selected from the group consisting of olfactory receptors encoded by orthologs of the olfactory receptor of interest in the same order as that of an organism species from which the olfactory receptor of interest is derived;
   (b) at least 11 olfactory receptors selected from the group consisting of the olfactory receptors encoded by orthologs of the olfactory receptor of interest in the same order as that of an organism species from which the olfactory receptor of interest is derived and olfactory receptors encoded by paralogs of the olfactory receptor of interest, the 11 olfactory receptors including at least one of the olfactory receptor encoded by the paralog of the olfactory receptor of interest;
   (c) at least 11 olfactory receptors selected from the group consisting of olfactory receptors encoded by orthologs of the olfactory receptor of interest in vertebrates, the 11 olfactory receptors including at least one olfactory receptor encoded by a vertebrate ortholog in an order different from the order of an organism species from which the olfactory receptor of interest is derived; and
   (d) at least 11 olfactory receptors selected from the group consisting of olfactory receptors encoded by orthologs of the olfactory receptor of interest in vertebrates and olfactory receptors encoded by orthologs in a vertebrate with a paralog having the highest homology to the olfactory receptor of interest among paralogs of the olfactory receptor of interest having 11 or more vertebrate orthologs, the 11 olfactory receptors including at least one olfactory receptor encoded by a vertebrate ortholog of orders different from the order of an organism species from which the olfactory receptor of interest is derived, and
an olfactory receptor encoded by the paralog.
[2] The method according to [1] which is a method for improving expression of an olfactory receptor polypeptide.
[3] A method for functionalizing an olfactory receptor of interest, comprising
expressing, in a cell, an olfactory receptor polypeptide consisting of an amino acid sequence obtained by, in an amino acid sequence of the olfactory receptor of interest, altering at least one amino acid residue different from that in a consensus amino acid sequence to the amino acid residue at a position corresponding thereto in the consensus amino acid sequence, wherein
the consensus amino acid sequence is an amino acid sequence derived by alignment of the amino acid sequence of the olfactory receptor of interest and amino acid sequences of any of the following olfactory receptors (a) to (d):
   (a) at least 11 olfactory receptors selected from the group consisting of olfactory receptors encoded by orthologs of the olfactory receptor of interest in the same order as that of an organism species from which the olfactory receptor of interest is derived;
   (b) at least 11 olfactory receptors selected from the group consisting of the olfactory receptors encoded by orthologs of the olfactory receptor of interest in the same order as that of an organism species from which the olfactory receptor of interest is derived and olfactory receptors encoded by paralogs of the olfactory receptor of interest, the 11 olfactory receptors including at least one of the olfactory receptor encoded by the paralog of the olfactory receptor of interest;
   (c) at least 11 olfactory receptors selected from the group consisting of olfactory receptors encoded by orthologs of the olfactory receptor of interest in vertebrates, the 11 olfactory receptors including at least one olfactory receptor encoded by a vertebrate ortholog in an order different from the order of an organism species from which the olfactory receptor of interest is derived; and
   (d) at least 11 olfactory receptors selected from the group consisting of olfactory receptors encoded by orthologs of the olfactory receptor of interest in vertebrates and olfactory receptors encoded by orthologs in a vertebrate with a paralog having the highest homology to the olfactory receptor of interest among paralogs of the olfactory receptor of interest having 11 or more vertebrate orthologs, the 11 olfactory receptors including at least one olfactory receptor encoded by a vertebrate ortholog in an order different from the order of an organism species from which the olfactory receptor of interest is derived, and an olfactory receptor encoded by the paralog.

[4] The method according to any one of [1] to [3], wherein the consensus amino acid sequence is an amino acid sequence consisting of consensus residues identified in accordance with the following criteria (i) to (iii) from the alignment of the amino acid sequence of the olfactory receptor of interest and the amino acid sequences of any of the olfactory receptors (a) to (d):
(i) at each amino acid position of the alignment,
   (i-i) when there exists one amino acid residue which is different from the amino acid residue of the olfactory receptor of interest and has a frequency of appearance of 50% or more, the amino acid residue is identified as a consensus residue,
   (i-ii) when there exist two amino acid residues having a frequency of appearance of 50%, the amino acid residues of the olfactory receptor of interest are identified as a consensus residue,
   (i-iii) when there exists an amino acid residue in the olfactory receptor of interest and there exists no amino acid residue having a frequency of appearance of 40% or more, the absence of a consensus residue is identified,
   (i-iv) when there exists no amino acid residue in the olfactory receptor of interest and there exists an amino acid residue having a frequency of appearance of 60% or more, an amino acid residue having the highest frequency of appearance is identified as a consensus residue, and when there exist two or more amino acid residues having the highest frequency of appearance, an amino acid residue having the smallest molecular weight among the amino acid residues is identified as a consensus residue, and
   (i-v) if none of the above (i-i) to (i-iv) is appropriate, the amino acid residue of the olfactory receptor of interest is identified as a consensus residue;
   (ii) when the consensus residues are identified in accordance with the criterion (i) and when a consensus residue nearest to the N terminus is a consensus residue at a position corresponding to the N terminus of the olfactory receptor of interest or a C-terminal side thereof and is not a methionine residue, a consensus residue on an N-terminal side of a consensus residue consisting of a methionine residue positioned nearest to the N terminus is changed to the absence of a consensus residue; and
   (iii) when the consensus residues are identified in accordance with the criterion (i) and when a consensus residue nearest to the N terminus is a consensus residue at a position corresponding to an N-terminal side of the N terminus of the olfactory receptor of interest and is not a methionine residue, an amino acid residue having the highest frequency of appearance is identified as a consensus residue until a methionine residue appears by tracing back one by one amino acid positions on an N-terminal side of the position of the consensus residue of the alignment, and when there exist two or more amino acid residues having the highest frequency of appearance, an amino acid residue having the smallest molecular weight among the amino acid residues is identified as a consensus residue.

[5] The method according to any one of [1] to [4], wherein the olfactory receptors (a) are preferably at least 15 olfactory receptors selected from the group consisting of the olfactory receptors encoded by orthologs of the olfactory receptor of interest in the same order as that of an organism species from which the olfactory receptor of interest is derived.
[6] The method according to any one of [1] to [4], wherein the olfactory receptors (b) are preferably at least 25 olfactory receptors, more preferably at least 35 olfactory receptors, selected from the group consisting of the olfactory receptors encoded by orthologs of the olfactory receptor of interest in the same order as that of an organism species from which the olfactory receptor of interest is derived and olfactory receptor encoded by the paralog of the olfactory receptor of interest, and
include an olfactory receptor encoded by a paralog having the highest homology to a gene encoding the olfactory receptor of interest.
[7] The method according to any one of [1] to [4], wherein the olfactory receptors (c) are preferably at least 15 olfactory receptors, more preferably at least 30 olfactory receptors, selected from the group consisting of the olfactory receptors encoded by orthologs of the olfactory receptor of interest in vertebrates, and
include at least one, preferably 5, more preferably 10 olfactory receptors encoded by vertebrate orthologs in orders different from the order of an organism species from which the olfactory receptor of interest is derived.
[8] The method according to any one of [1] to [4], wherein the olfactory receptors (d) are preferably at least 30 olfactory receptors, more preferably at least 60 olfactory receptors, selected from the group consisting of the olfactory receptors encoded by orthologs of the olfactory receptor of interest in vertebrates, and the olfactory receptors encoded by orthologs in a vertebrate with a paralog having the highest homology to the olfactory receptor of interest among paralogs of the olfactory receptor of interest having 11 or more vertebrate orthologs, and an olfactory receptor encoded by the paralog, and
include at least one, preferably 5, more preferably 10 olfactory receptors encoded by vertebrate orthologs in orders different from the order of an organism species from which the olfactory receptor of interest is derived.
[9] The method according to any one of [1] to [8], wherein the alignment is preferably alignment of the amino acid sequence of the olfactory receptor of interest and the olfactory receptors (c).
[10] The method according to any one of [1] to [9], wherein the olfactory receptor of interest is a human olfactory receptor.
[11] The method according to any one of [1] to [8], wherein the olfactory receptor polypeptide preferably consists of an amino acid sequence derived from an amino acid sequence of sequence identification number (2) of an olfactory receptor (1) in the following Tables 2-1 to 2-5 by altering at least one amino acid residue different from that in a consensus amino acid sequence s sequence identification number (3) to the amino acid residue at a position corresponding thereto in the consensus amino acid sequence, and more preferably consists of a consensus amino acid sequence of sequence identification number (3) in the following Tables 2-1 to 2-5:

**[Table 2-1]**

| No. | (1) Olfactory receptor | (2) SEQ ID NO of amino acid sequence | (3) SEQ ID NO of consensus amino acid sequence | No. | (1) Olfactory receptor | (2) SEQ ID NO of amino acid sequence | (3) SEQ ID NO of consensus amino acid sequence |
|---|---|---|---|---|---|---|---|
| 1 | OR2A25 | 1 | 97 | 31 | OR5W2 | 21 | 127 |
| 2 | OR2A25 | 1 | 98 | 32 | OR6A2 | 22 | 128 |
| 3 | OR2A25 | 1 | 99 | 33 | OR6B2 | 23 | 129 |
| 4 | OR2AG1 | 2 | 100 | 34 | OR6C1 | 24 | 130 |
| 5 | OR2AG2 | 3 | 101 | 35 | OR6Y1 | 25 | 131 |
| 6 | OR2D2 | 4 | 102 | 36 | OR6Y1 | 25 | 132 |
| 7 | OR2D3 | 5 | 103 | 37 | OR6Y1 | 25 | 133 |
| 8 | OR2T11 | 6 | 104 | 38 | OR7A17 | 26 | 134 |
| 9 | OR2T11 | 6 | 105 | 39 | OR7A17 | 26 | 135 |
| 10 | OR2T11 | 6 | 106 | 40 | OR7A17 | 26 | 136 |
| 11 | OR2W1 | 7 | 107 | 41 | OR7D4 | 27 | 137 |
| 12 | OR2W1 | 7 | 108 | 42 | OR7D4 | 27 | 138 |
| 13 | OR2W1 | 7 | 109 | 43 | OR7D4 | 27 | 139 |
| 14 | OR2W3 | 8 | 110 | 44 | OR10A2 | 28 | 140 |
| 15 | OR2Y1 | 9 | 111 | 45 | OR10A4 | 29 | 141 |
| 16 | OR3A2 | 10 | 112 | 46 | OR10D3 | 30 | 142 |
| 17 | OR4A16 | 11 | 113 | 47 | OR10Q1 | 31 | 143 |
| 18 | OR4C15 | 12 | 114 | 48 | OR10Z1 | 32 | 144 |
| 19 | OR4D5 | 13 | 115 | 49 | OR11H1 | 33 | 145 |
| 20 | OR4D6 | 14 | 116 | 50 | OR11H4 | 34 | 146 |
| 21 | OR4F15 | 15 | 117 | 51 | OR11H4 | 34 | 147 |
| 22 | OR4K15 | 16 | 118 | 52 | OR11H4 | 34 | 148 |
| 23 | OR4Q3 | 17 | 119 | 53 | OR13C8 | 35 | 149 |
| 24 | OR4Q3 | 17 | 120 | 54 | OR13G1 | 36 | 150 |
| 25 | OR4Q3 | 17 | 121 | 55 | OR52A4 | 37 | 151 |
| 26 | OR4S2 | 18 | 122 | 56 | OR56A4 | 38 | 152 |
| 27 | OR4S2 | 18 | 123 | | | | |
| 28 | OR4S2 | 18 | 124 | | | | |
| 29 | OR5A2 | 19 | 125 | | | | |
| 30 | OR5C1 | 20 | 126 | | | | |

[0130]

**[Table 2-2]**

| No. | (1) Olfactory receptor | (2) SEQ ID NO of amino acid sequence | (3) SEQ ID NO of consensus amino acid sequence | No. | (1) Olfactory receptor | (2) SEQ ID NO of amino acid sequence | (3) SEQ ID NO of consensus amino acid sequence |
|---|---|---|---|---|---|---|---|
| 57 | OR2B11 | 39 | 153 | 87 | OR4K17 | 69 | 183 |
| 58 | OR2C3 | 40 | 154 | 88 | OR4L1 | 70 | 184 |
| 59 | OR2G2 | 41 | 155 | 89 | OR4M1 | 71 | 185 |
| 60 | OR2G3 | 42 | 156 | 90 | OR4N2 | 72 | 186 |
| 61 | OR2L2 | 43 | 157 | 91 | OR4N5 | 73 | 187 |
| 62 | OR2L3 | 44 | 158 | 92 | OR5A1 | 74 | 188 |
| 63 | OR2L5 | 45 | 159 | 93 | OR6F1 | 75 | 189 |
| 64 | OR2L8 | 46 | 160 | 94 | OR6J1 | 76 | 190 |
| 65 | OR2L13 | 47 | 161 | 95 | OR7A10 | 77 | 191 |
| 66 | OR2M2 | 48 | 162 | 96 | OR7C2 | 78 | 192 |
| 67 | OR2M4 | 49 | 163 | 97 | OR7G2 | 79 | 193 |
| 68 | OR2T1 | 50 | 164 | 98 | OR7G3 | 80 | 194 |
| 69 | OR2T4 | 51 | 165 | 99 | OR10G2 | 81 | 195 |
| 70 | OR2T5 | 52 | 166 | 100 | OR10J1 | 82 | 196 |
| 71 | OR2T6 | 53 | 167 | 101 | OR10J3 | 83 | 197 |
| 72 | OR2T7 | 54 | 168 | 102 | OR10K1 | 84 | 198 |
| 73 | OR2T8 | 55 | 169 | 103 | OR10K2 | 85 | 199 |
| 74 | OR2T12 | 56 | 170 | 104 | OR10T2 | 86 | 200 |
| 75 | OR2T27 | 57 | 171 | 105 | OR10X1 | 87 | 201 |
| 76 | OR2T29 | 58 | 172 | 106 | OR11G2 | 88 | 202 |
| 77 | OR2T33 | 59 | 173 | 107 | OR11H6 | 89 | 203 |
| 78 | OR2T34 | 60 | 174 | 108 | OR11H7 | 90 | 204 |
| 79 | OR2T35 | 61 | 175 | 109 | OR11L1 | 91 | 205 |
| 80 | OR2AK2 | 62 | 176 | 110 | OR14A2 | 92 | 206 |
| 81 | OR4D10 | 63 | 177 | 111 | OR14A16 | 93 | 207 |
| 82 | OR4E2 | 64 | 178 | 112 | OR14C36 | 94 | 208 |
| 83 | OR4F5 | 65 | 179 | 113 | OR14K1 | 95 | 209 |
| 84 | OR4K1 | 66 | 180 | | | | |
| 85 | OR4K2 | 67 | 181 | | | | |
| 86 | OR4K5 | 68 | 182 | | | | |

[0131]

**[Table 2-3]**

| No. | (1) Olfactory receptor | (2) SEQ ID NO of amino acid sequence | (3) SEQ ID NO of consensus amino acid sequence | No. | (1) Olfactory receptor | (2) SEQ ID NO of amino acid sequence | (3) SEQ ID NO of consensus amino acid sequence |
|---|---|---|---|---|---|---|---|
| 115 | OR1B1 | 327 | 485 | 155 | OR3A3 | 367 | 525 |
| 116 | OR1D2 | 328 | 486 | 156 | OR4A5 | 368 | 526 |
| 117 | OR1D4 | 329 | 487 | 157 | OR4A15 | 369 | 527 |
| 118 | OR1E2 | 330 | 488 | 158 | OR4A47 | 370 | 528 |
| 119 | OR1F1 | 331 | 489 | 159 | OR4B1 | 371 | 529 |
| 120 | OR1G1 | 332 | 490 | 160 | OR4C3 | 372 | 530 |
| 121 | OR1I1 | 333 | 491 | 161 | OR4G6 | 373 | 531 |
| 122 | OR1J1 | 334 | 492 | 162 | OR4C11 | 374 | 532 |
| 123 | OR1J4 | 335 | 493 | 163 | OR4C12 | 375 | 533 |
| 124 | OR1K1 | 336 | 494 | 164 | OR4C16 | 376 | 534 |
| 125 | OR1L1 | 337 | 495 | 165 | OR4C46 | 377 | 535 |
| 126 | OR1L3 | 338 | 496 | 166 | OR4D1 | 378 | 536 |
| 127 | OR1L4 | 339 | 497 | 167 | OR4D2 | 379 | 537 |
| 128 | OR1L6 | 340 | 498 | 168 | OR4D9 | 380 | 538 |
| 129 | OR1L8 | 341 | 499 | 169 | OR4D11 | 381 | 539 |
| 130 | OR1M1 | 342 | 500 | 170 | OR4F17 | 382 | 540 |
| 131 | OR1Q1 | 343 | 501 | 171 | OR4F21 | 383 | 541 |
| 132 | OR1S1 | 344 | 502 | 172 | OR4P4 | 384 | 542 |
| 133 | OR1S2 | 345 | 503 | 173 | OR4X1 | 385 | 543 |
| 134 | OR2A1 | 346 | 504 | 174 | OR4X2 | 386 | 544 |
| 135 | OR2A5 | 347 | 505 | 175 | OR5B2 | 387 | 545 |
| 136 | OR2A7 | 348 | 506 | 176 | OR5B12 | 388 | 546 |
| 137 | OR2A14 | 349 | 507 | 177 | OR5B17 | 389 | 547 |
| 138 | OR2B2 | 350 | 508 | 178 | OR5H1 | 390 | 548 |
| 139 | OR2B3 | 351 | 509 | 179 | OR5H2 | 391 | 549 |
| 140 | OR2B6 | 352 | 510 | 180 | OR5H6 | 392 | 550 |
| 141 | OR2C1 | 353 | 511 | 181 | OR5H14 | 393 | 551 |
| 142 | OR2F1 | 354 | 512 | 182 | OR5K3 | 394 | 552 |
| 143 | OR2F2 | 355 | 513 | 183 | OR5AK2 | 395 | 553 |
| 144 | OR2H1 | 356 | 514 | 184 | OR5AR1 | 396 | 554 |
| 145 | OR2H2 | 357 | 515 | 185 | OR5AS1 | 397 | 555 |
| 146 | OR2K2 | 358 | 516 | 186 | OR6B1 | 398 | 556 |
| 147 | OR2S2 | 359 | 517 | 187 | OR6B3 | 399 | 557 |
| 148 | OR2V1 | 360 | 518 | 188 | OR6C2 | 400 | 558 |
| 149 | OR2V2 | 361 | 519 | 189 | OR6C3 | 401 | 559 |
| 150 | OR2Z1 | 362 | 520 | 190 | OR6C4 | 402 | 560 |
| 151 | OR2AE1 | 363 | 521 | 191 | OR6C6 | 403 | 561 |
| 152 | OR2AP1 | 364 | 522 | 192 | OR6C65 | 404 | 562 |
| 153 | OR2AT4 | 365 | 523 | 193 | OR6C68 | 405 | 563 |
| 154 | OR3A1 | 366 | 524 | 194 | OR6C70 | 406 | 564 |

[0132]

**[Table 2-4]**

| No. | (1) Olfactory receptor | (2) SEQ ID NO of amino acid sequence | (3) SEQ ID NO of consensus amino acid sequence | No. | (1) Olfactory receptor | (2) SEQ ID NO of amino acid sequence | (3) SEQ ID NO of consensus amino acid sequence |
|---|---|---|---|---|---|---|---|
| 195 | OR6C74 | 407 | 565 | 234 | OR51B5 | 446 | 604 |
| 196 | OR6C75 | 408 | 566 | 235 | OR51B6 | 447 | 605 |
| 197 | OR6C76 | 409 | 567 | 236 | OR51D1 | 448 | 606 |
| 198 | OR6K3 | 410 | 568 | 237 | OR51E2 | 449 | 607 |
| 199 | OR6K6 | 411 | 569 | 238 | OR51F1 | 450 | 608 |
| 200 | OR6N2 | 412 | 570 | 239 | OR51F2 | 451 | 609 |
| 201 | OR8S1 | 413 | 571 | 240 | OR51G1 | 452 | 610 |
| 202 | OR9A2 | 414 | 572 | 241 | OR51G2 | 453 | 611 |
| 203 | OR9A4 | 415 | 573 | 242 | OR51I1 | 454 | 612 |
| 204 | OR10A3 | 416 | 574 | 243 | OR51I2 | 455 | 613 |
| 205 | OR10A7 | 417 | 575 | 244 | OR51J1 | 456 | 614 |
| 206 | OR10G6 | 418 | 576 | 245 | OR51L1 | 457 | 615 |
| 207 | OR10G9 | 419 | 577 | 246 | OR51M1 | 458 | 616 |
| 208 | OR10H1 | 420 | 578 | 247 | OR51Q1 | 459 | 617 |
| 209 | OR10H2 | 421 | 579 | 248 | OR51S1 | 460 | 618 |
| 210 | OR10H3 | 422 | 580 | 249 | OR51T1 | 461 | 619 |
| 211 | OR10H4 | 423 | 581 | 250 | OR51V1 | 462 | 620 |
| 212 | OR10H5 | 424 | 582 | 251 | OR52A1 | 463 | 621 |
| 213 | OR10P1 | 425 | 583 | 252 | OR52A5 | 464 | 622 |
| 214 | OR10S1 | 426 | 584 | 253 | OR52B2 | 465 | 623 |
| 215 | OR10V1 | 427 | 585 | 254 | OR52B4 | 466 | 624 |
| 216 | OR10W1 | 428 | 586 | 255 | OR52B6 | 467 | 625 |
| 217 | OR10AD1 | 429 | 587 | 256 | OR52D1 | 468 | 626 |
| 218 | OR10AG1 | 430 | 588 | 257 | OR52E5 | 469 | 627 |
| 219 | OR11A1 | 431 | 589 | 258 | OR52E6 | 470 | 628 |
| 220 | OR11H2 | 432 | 590 | 259 | OR52H1 | 471 | 629 |
| 221 | OR12D2 | 433 | 591 | 260 | OR52I1 | 472 | 630 |
| 222 | OR12D3 | 434 | 592 | 261 | OR52I2 | 473 | 631 |
| 223 | OR13A1 | 435 | 593 | 262 | OR52J3 | 474 | 632 |
| 224 | OR13C2 | 436 | 594 | 263 | OR52K1 | 475 | 633 |
| 225 | OR13C3 | 437 | 595 | 264 | OR52K2 | 476 | 634 |
| 226 | OR13C4 | 438 | 596 | 265 | OR52N1 | 477 | 635 |
| 227 | OR13C9 | 439 | 597 | 266 | OR52R1 | 478 | 636 |
| 228 | OR13F1 | 440 | 598 | 267 | OR52W1 | 479 | 637 |
| 229 | OR51A2 | 441 | 599 | 268 | OR56A5 | 480 | 638 |
| 230 | OR51A4 | 442 | 600 | 269 | OR56B4 | 481 | 639 |
| 231 | OR51A7 | 443 | 601 | 270 | OR5AN1 | 482 | 640 |
| 232 | OR51B2 | 444 | 602 | 271 | M71 | 483 | 641 |
| 233 | OR51B4 | 445 | 603 | | | | |

[0133]

**[Table 2-5]**

| No. | (1) Olfactory receptor | (2) SEQ ID NO of amino acid sequence | (3) SEQ ID NO of consensus amino acid sequence | No. | (1) Olfactory receptor | (2) SEQ ID NO of amino acid sequence | (3) SEQ ID NO of consensus amino acid sequence |
|---|---|---|---|---|---|---|---|
| 273 | OR2A2 | 801 | 851 | 298 | OR6P1 | 826 | 876 |
| 274 | OR2A12 | 802 | 852 | 299 | OR6Q1 | 827 | 877 |
| 275 | OR4S1 | 803 | 853 | 300 | OR6T1 | 828 | 878 |
| 276 | OR5AC2 | 804 | 854 | 301 | OR6X1 | 829 | 879 |
| 277 | OR5B3 | 805 | 855 | 302 | OR8B4 | 830 | 880 |
| 278 | OR5D13 | 806 | 856 | 303 | OR8B8 | 831 | 881 |
| 279 | OR5D14 | 807 | 857 | 304 | OR8B12 | 832 | 882 |
| 280 | OR5D16 | 808 | 858 | 305 | OR8D1 | 833 | 883 |
| 281 | OR5D18 | 809 | 859 | 306 | OR8G1 | 834 | 884 |
| 282 | OR5J2 | 810 | 860 | 307 | OR8G5 | 835 | 885 |
| 283 | OR5K4 | 811 | 861 | 308 | OR8H2 | 836 | 886 |
| 284 | OR5L1 | 812 | 862 | 309 | OR8I2 | 837 | 887 |
| 285 | OR5L2 | 813 | 863 | 310 | ORBJ1 | 838 | 888 |
| 286 | OR5M1 | 814 | 864 | 311 | OR8K1 | 839 | 889 |
| 287 | OR5M3 | 815 | 865 | 312 | OR8K3 | 840 | 890 |
| 288 | OR5M8 | 816 | 866 | 313 | OR8K5 | 841 | 891 |
| 289 | OR5M9 | 817 | 867 | 314 | OR8U1 | 842 | 892 |
| 290 | OR5M10 | 818 | 868 | 315 | OR9G1 | 843 | 893 |
| 291 | OR5M11 | 819 | 869 | 316 | OR9G4 | 844 | 894 |
| 292 | OR5P2 | 820 | 870 | 317 | OR9I1 | 845 | 895 |
| 293 | OR5T1 | 821 | 871 | 318 | OR11H12 | 846 | 896 |
| 294 | OR5T2 | 822 | 872 | 319 | OR52M1 | 847 | 897 |
| 295 | OR5T3 | 823 | 873 | 320 | OR52N5 | 848 | 898 |
| 296 | OR6M1 | 824 | 874 | 321 | OR56A3 | 849 | 899 |
| 297 | OR6N1 | 825 | 875 | | | | |

[12] The method according to [11], wherein the olfactory receptor polypeptide preferably consists of an amino acid sequence of any of SEQ ID NOs: 97 to 209, 485 to 641, and 851 to 899, and more preferably consists of an amino acid sequence of any of SEQ ID NOs: 99 to 104, 109 to 118, 121, 123, 125 to 130, 133, 136, 138, 140 to 146, 149 to 209, 485 to 641, and 851 to 899.
[13] A method for expressing an olfactory receptor polypeptide, comprising
expressing, in a cell, an olfactory receptor polypeptide consisting of an amino acid sequence obtained by, in an amino acid sequence of an olfactory receptor of interest, altering at least one amino acid residue different from that in a consensus amino acid sequence to the amino acid residue at a position corresponding thereto in the consensus amino acid sequence, wherein
the olfactory receptor polypeptide consists of an amino acid sequence obtained by, in an amino acid sequence of SEQ ID NO: 96 of a human olfactory receptor OR7E24, altering at least one amino acid residue different from that in a consensus amino acid sequence of SEQ ID NO: 210 to the amino acid residue at a position corresponding thereto in the consensus amino acid sequence, and preferably consists of an amino acid sequence of SEQ ID NO: 210,
the olfactory receptor polypeptide consists of an amino acid sequence obtained by, in an amino acid sequence of SEQ ID NO: 484 of a human olfactory receptor OR9K2, altering at least one amino acid residue different from that in a consensus amino acid sequence of in SEQ ID NO: 642 to the amino acid residue at a position corresponding thereto in the consensus amino acid sequence, and preferably consists of an amino acid sequence of SEQ ID NO: 642, or
the olfactory receptor polypeptide consists of an amino acid sequence obtained by, in an amino acid sequence of SEQ ID NO: 850 of a human olfactory receptor OR5I1, altering at least one amino acid residue different from that in a consensus amino acid sequence of SEQ ID NO: 900 to the amino acid residue at a position corresponding thereto in the consensus amino acid sequence.

[14] The method according to [13] which is a method for improving expression of an olfactory receptor polypeptide.
[15] A method for functionalizing an olfactory receptor of interest, comprising
expressing, in a cell, an olfactory receptor polypeptide consisting of an amino acid sequence obtained by, in an amino acid sequence of the olfactory receptor of interest, altering at least one amino acid residue different from that in a consensus amino acid sequence to the amino acid residue of the consensus amino acid sequence at a position corresponding thereto, wherein
the olfactory receptor polypeptide consists of an amino acid sequence obtained by, in an amino acid sequence of SEQ ID NO: 96 of a human olfactory receptor OR7E24, altering at least one amino acid residue different from that in a consensus amino acid sequence of SEQ ID NO: 210 to the amino acid residue at a position corresponding thereto in the consensus amino acid sequence, and preferably consists of an amino acid sequence of SEQ ID NO: 210,
the olfactory receptor polypeptide consists of an amino acid sequence obtained by, in an amino acid sequence of SEQ ID NO: 484 of a human olfactory receptor OR9K2, altering at least one amino acid residue different from that in a consensus amino acid sequence of SEQ ID NO: 642 to the amino acid residue at a position corresponding thereto in the consensus amino acid sequence, and preferably consists of an amino acid sequence set forth in SEQ ID NO: 642, or
the olfactory receptor polypeptide consists of an amino acid sequence obtained by, in an amino acid sequence of SEQ ID NO: 850 of a human olfactory receptor OR5I1, altering at least one amino acid residue different from that in a consensus amino acid sequence of SEQ ID NO: 900 to the amino acid residue at a position corresponding thereto in the consensus amino acid sequence.

[16] The method according to any one of [1] to [15], wherein the olfactory receptor polypeptide is expressed on a cell membrane of the cell.
[17] The method according to any one of [1] to [16], preferably further comprising causing the cell to express RTP1S.
[18] The method according to any one of [1] to [17], wherein the cell is a HEK293 cell.
[19] A method for measuring a response of an olfactory receptor of interest, comprising
measuring a response of an olfactory receptor polypeptide expressed by the method according to any one of [1] to [18].
[20] A method for searching for a ligand for an olfactory receptor of interest, comprising:
measuring a response of an olfactory receptor polypeptide expressed by the method according to any one of [1] to [18] in the presence of a test substance; and
selecting a test substance to which the olfactory receptor polypeptide has responded.

[21] The method according to [20], preferably further comprising measuring a response of the olfactory receptor polypeptide in the absence of the test substance.
[22] The method according to [21], wherein a test substance is preferably selected which increases the response of the olfactory receptor polypeptide in the presence of the test substance to 120% or more of the response in the absence of the test substance.
[23] The method according to [21], wherein a test substance is preferably selected which statistically significantly increases the response of the olfactory receptor polypeptide in the presence of the test substance in comparison with the response in the absence of the test substance.
[24] A method for evaluating and/or selecting a suppressor of odor of a ligand for an olfactory receptor of interest, comprising:
adding a test substance and the ligand for an olfactory receptor of interest to an olfactory receptor polypeptide expressed by the method according to any one of [1] to [18]; and
measuring a response of the olfactory receptor polypeptide to the ligand.

[25] The method according to [24], wherein the ligand is a ligand selected by the method according to any one of [20] to [23].
[26] The method according to [24] or [25], further comprising identifying a test substance which suppresses the response of the olfactory receptor polypeptide on the basis of the measured response.
[27] The method according to any one of [24] to [26], further comprising measuring a response of the olfactory receptor polypeptide non-supplemented with the test substance to the ligand.
[28] The method according to [27], further comprising identifying the test substance as a substance which suppresses the response of the olfactory receptor polypeptide to the ligand when the response of the olfactory receptor polypeptide supplemented with the test substance to the ligand is more suppressed than the response of the olfactory receptor polypeptide non-supplemented with the test substance to the ligand.
[29] A method for evaluating and/or selecting a suppressor of odor of a ligand for an olfactory receptor of interest, comprising:
adding a test substance to an olfactory receptor polypeptide expressed by the method according to any one of [1] to [18]; and
measuring a response of the olfactory receptor polypeptide to the test substance.

[30] The method according to [29], wherein the ligand is a ligand selected by the method according to any one of [20] to [23].
[31] The method according to [29] or [30], further comprising identifying a test substance which enhances the response of the olfactory receptor polypeptide on the basis of the measured response.
[32] The method according to any one of [29] to [31], further comprising measuring a response of the olfactory receptor polypeptide non-supplemented with the test substance.
[33] The method according to [32], further comprising identifying the test substance as a substance which suppresses the response of the olfactory receptor polypeptide to the ligand when the response of the olfactory receptor polypeptide supplemented with the test substance is more enhanced than the response of the olfactory receptor polypeptide non-supplemented with the test substance.
[34] A method for evaluating and/or selecting an enhancer of odor of a ligand for an olfactory receptor of interest, comprising:
adding a test substance and the ligand for an olfactory receptor of interest to an olfactory receptor polypeptide expressed by the method according to any one of [1] to [18]; and
measuring a response of the olfactory receptor polypeptide to the ligand.

[35] The method according to [34], wherein the ligand is a ligand selected by the method according to any one of [20] to [23].
[36] The method according to [34] or [35], further comprising identifying a test substance which enhances the response of the olfactory receptor polypeptide on the basis of the measured response.
[37] The method according to any one of [34] to [36], further comprising measuring a response of the olfactory receptor polypeptide non-supplemented with the test substance to the ligand.
[38] The method according to [37], further comprising identifying the test substance as a substance which enhances the response of the olfactory receptor polypeptide to the ligand when the response of the olfactory receptor polypeptide supplemented with the test substance to the ligand is more enhanced than the response of the olfactory receptor polypeptide non-supplemented with the test substance to the ligand.
[39] The method according to any one of [19] to [38], wherein the response of the olfactory receptor polypeptide is preferably measured through intracellular cAMP level measurement by ELISA or reporter gene assay, calcium ion level measurement by calcium imaging or TGFα shedding assay, or potential change measurement inside and outside cell membranes by a two-electrode voltage clamp technique using *Xenopus* oocytes.
[40] An altered olfactory receptor polypeptide consisting of
an amino acid sequence obtained by, in an amino acid sequence of an olfactory receptor of interest, altering at least one amino acid residue different from that in a consensus amino acid sequence to the amino acid residue at a position corresponding thereto in the consensus amino acid sequence, wherein
the consensus amino acid sequence is an amino acid sequence obtained by alignment of the amino acid sequence of the olfactory receptor of interest and amino acid sequences of any of the following olfactory receptors (a) to (d):
   (a) at least 11 olfactory receptors selected from the group consisting of olfactory receptors encoded by orthologs of the olfactory receptor of interest in the same order as that of an organism species from which the olfactory receptor of interest is derived;
   (b) at least 11 olfactory receptors selected from the group consisting of the olfactory receptors encoded by orthologs of the olfactory receptor of interest in the same order as that of an organism species from which the olfactory receptor of interest is derived and olfactory receptors encoded by paralogs of the olfactory receptor of interest, the 11 olfactory receptors including at least one of the olfactory receptor encoded by the paralog of the olfactory receptor of interest;
   (c) at least 11 olfactory receptors selected from the group consisting of olfactory receptors encoded by orthologs of the olfactory receptor of interest in vertebrates, the 11 olfactory receptors including at least one olfactory receptor encoded by a vertebrate ortholog in an order different from the order of an organism species from which the olfactory receptor of interest is derived; and
   (d) at least 11 olfactory receptors selected from the group consisting of olfactory receptors encoded by orthologs of the olfactory receptor of interest in vertebrates and olfactory receptors encoded by orthologs in a vertebrate with a paralog having the highest
   homology to the olfactory receptor of interest among paralogs of the olfactory receptor of interest having 11 or more vertebrate orthologs, the 11 olfactory receptors including at least one olfactory receptor encoded by a vertebrate ortholog in an order different from the order of an organism species from which the olfactory receptor of interest is derived, and
an olfactory receptor encoded by the paralog.
[41] The altered olfactory receptor polypeptide according to [40], wherein the consensus amino acid sequence is an amino acid sequence consisting of consensus residues identified in accordance with the following criteria (i) to (iii) from the alignment of the amino acid sequence of the olfactory receptor of interest and the amino acid sequences of any of the olfactory receptors (a) to (d):
(i) at each amino acid position of the alignment,
   (i-i) when there exists one amino acid residue which is different from the amino acid residue of the olfactory receptor of interest and has a frequency of appearance of 50% or more, the amino acid residue is identified as a consensus residue,
   (i-ii) when there exist two amino acid residues having a frequency of appearance of 50%, the amino acid residues of the olfactory receptor of interest are identified as a consensus residue,
   (i-iii) when there exists an amino acid residue in the olfactory receptor of interest and there exists no amino acid residue having a frequency of appearance of 40% or more, the absence of a consensus residue is identified,
   (i-iv) when there exists no amino acid residue in the olfactory receptor of interest and there exists an amino acid residue having a frequency of appearance of 60% or more, an amino acid residue having the highest frequency of appearance is identified as a consensus residue, and when there exist two or more amino acid residues having the highest frequency of appearance, an amino acid residue having the smallest molecular weight among the amino acid residues is identified as a consensus residue, and
   (i-v) if none of the above (i-i) to (i-iv) is appropriate, the amino acid residue of the olfactory receptor of interest is identified as a consensus residue;
(ii) when the consensus residues are identified in accordance with the criterion (i) and when a consensus residue nearest to the N terminus is a consensus residue at a position corresponding to the N terminus of the olfactory receptor of interest or a C-terminal side thereof and is not a methionine residue, a consensus residue on an N-terminal side of a consensus residue consisting of a methionine residue positioned nearest to the N terminus is changed to the absence of a consensus residue; and
(iii) when the consensus residues are identified in accordance with the criterion (i) and when a consensus residue nearest to the N terminus is a consensus residue at a position corresponding to an N-terminal side of the N terminus of the olfactory receptor of interest and is not a methionine residue, an amino acid residue having the highest frequency of appearance is identified as a consensus residue until a methionine residue appears by tracing back one by one amino acid positions on an N-terminal side of the position of the consensus residue of the alignment, and when there exist two or more amino acid residues having the highest frequency of appearance, an amino acid residue having the smallest molecular weight among the amino acid residues is identified as a consensus residue.

[42] The altered olfactory receptor polypeptide according to [40] or [41], wherein the olfactory receptors (a) are preferably at least 15 olfactory receptors selected from the group consisting of the olfactory receptors encoded by orthologs of the olfactory receptor of interest in the same order as that of an organism species from which the olfactory receptor of interest is derived.
[43] The altered olfactory receptor polypeptide according to [40] or [41], wherein the olfactory receptors (b) are preferably at least 25 olfactory receptors, more preferably at least 35 olfactory receptors, selected from the group consisting of the olfactory receptors encoded by orthologs of the olfactory receptor of interest in the same order as that of an organism species from which olfactory receptor of interest is derived and olfactory receptor encoded by the paralog of the olfactory receptor of interest, and
include an olfactory receptor encoded by a paralog having the highest homology to a gene encoding the olfactory receptor of interest.
[44] The altered olfactory receptor polypeptide according to [40] or [41], wherein the olfactory receptors (c) are preferably at least 15 olfactory receptors, more preferably at least 30 olfactory receptors, selected from the group consisting of the olfactory receptors encoded by orthologs of the olfactory receptor of interest in vertebrates, and
include at least one, preferably 5, more preferably 10 olfactory receptors encoded by vertebrate orthologs of orders different from the order of an organism species from which the olfactory receptor of interest is derived.
[45] The altered olfactory receptor polypeptide according to [40] or [41], wherein the olfactory receptors (d) are preferably at least 30 olfactory receptors, more preferably at least 60 olfactory receptors, selected from the group consisting of the olfactory receptors encoded by orthologs of the olfactory receptor of interest in vertebrates, and the olfactory receptors encoded by orthologs in a vertebrate with a paralog having the highest homology to the olfactory receptor of interest among paralogs of the olfactory receptor of interest having 11 or more vertebrate orthologs, and an olfactory receptor encoded by the paralog, and
include at least one, preferably 5, more preferably 10 olfactory receptors encoded by vertebrate orthologs of orders different from the order of an organism species from which the olfactory receptor of interest is derived.
[46] The altered olfactory receptor polypeptide according to any one of [40] to [45], wherein the alignment is preferably alignment of the amino acid sequence of the olfactory receptor of interest and the olfactory receptors (c).
[47] The altered olfactory receptor polypeptide according to any one of [40] to [46], wherein the olfactory receptor of interest is a human olfactory receptor.
[48] The altered olfactory receptor polypeptide according to any one of [40] to [45], which preferably consists of an amino acid sequence derived from an amino acid sequence of sequence identification number (2) of an olfactory receptor (1) in the above Tables 2-1 to 2-5 by altering at least one amino acid residue different from that in a consensus amino acid sequence of sequence identification number (3) to the amino acid residue at a position corresponding thereto in the consensus amino acid sequence, and more preferably consists of a consensus amino acid sequence of sequence identification number (3) in the above Tables 2-1 to 2-5.
[49] The altered olfactory receptor polypeptide according to [48], which preferably consists of an amino acid sequence of any of SEQ ID NOs: 97 to 209, 485 to 641, and 851 to 899, and more preferably consists of an amino acid sequence of any of SEQ ID NOs: 99 to 104, 109 to 118, 121, 123, 125 to 130, 133, 136, 138, 140 to 146, 149 to 209, 485 to 641, and 851 to 899.
[50] An altered olfactory receptor polypeptide
consisting of an amino acid sequence obtained by, in an amino acid sequence of SEQ ID NO: 96 of a human olfactory receptor OR7E24, altering at least one amino acid residue different from that in a consensus amino acid sequence of SEQ ID NO: 210 to the amino acid residue at a position corresponding thereto in the consensus amino acid sequence, and preferably consisting of an amino acid sequence of SEQ ID NO: 210,
consisting of an amino acid sequence obtained by, in an amino acid sequence of SEQ ID NO: 484 of a human olfactory receptor OR9K2, altering at least one amino acid residue different from that in a consensus amino acid sequence of SEQ ID NO: 642 to the amino acid residue at a position corresponding thereto in the consensus amino acid sequence, and preferably consisting of an amino acid sequence set forth in SEQ ID NO: 642, or
consisting of an amino acid sequence obtained by, in an amino acid sequence of SEQ ID NO: 850 of a human olfactory receptor OR5I1, altering at least one amino acid residue different from that in a consensus amino acid sequence of SEQ ID NO: 900 to the amino acid residue at a position corresponding thereto in the consensus amino acid sequence.

[51] A polynucleotide encoding the altered olfactory receptor polypeptide according to any one of [40] to [50].
[52] The polynucleotide according to [51] which consists of a nucleotide sequence of any of SEQ ID NOs: 211 to 324, 643 to 800, and 901 to 950, and preferably consists of a nucleotide sequence of any of SEQ ID NOs: 213 to 218, 223 to 232, 235, 237, 239 to 244, 247, 250, 252, 254 to 260, 263 to 324, 643 to 800, and 901 to 950.
[53] A vector or a DNA fragment comprising the polynucleotide according to [51] or [52].
[54] A transformed cell comprising the vector or the DNA fragment according to [53].
[55] The transformed cell according to [54], preferably further comprising a vector or a DNA fragment comprising a polynucleotide encoding RTP1S.
[56] The transformed cell according to [54] or [55], wherein the cell is a HEK293 cell.

### Examples

Hereinafter, the present invention will be more specifically described with reference to Examples.

### Example 1 Preparation and analysis of consensus olfactory receptor

### 1) Preparation of olfactory receptor gene

For consensus olfactory receptor design, homologous gene candidates of the gene of an olfactory receptor of interest were searched for using NCBI BLAST. An ortholog group or ortholog and paralog groups were identified as to the obtained gene group.

Specifically, when the olfactory receptor of interest was a human olfactory receptor, the identification of primate orthologs in (a) was carried out by phylogenetic tree analysis on primate candidate genes searched for by BLAST. For example, in the case of applying the method (a) to human OR2A25, phylogenetic tree analysis was conducted on homologous genes obtained by BLAST search setting the amino acid sequence of human OR2A25 (NP_001004488.1) to a query sequence and an organism name to be searched to primates. A clade was selected which contained human OR2A25, covered OR2A25 of many other organism species, and contained as few genes other than OR2A25 as possible. Among a total of 18 genes contained in the clade, 17 genes except for human OR2A25 were identified as primate orthologs. As a result, these 17 genes exhibited 82% or more amino acid homology to human OR2A25. Subsequently, the amino acid sequences of these 18 genes (Table 3) were subjected to alignment analysis and consensus amino acid identification as mentioned below.

When the olfactory receptor of interest was a human olfactory receptor, the identification of primate orthologs and paralogs in (b) was carried out by identifying a clade which was adjacent to the identified clade of primate orthologs as a result of the phylogenetic tree analysis for the primate ortholog identification in (a), and contained a gene, having the highest homology to the gene of the olfactory receptor of interest, in the organism species from which the olfactory receptor of interest is derived. Genes contained in the identified clade group were selected as paralogs. Among these paralogs, paralogs were excluded which were not included in top 50 homologous genes of the gene of the olfactory receptor of interest as primate genes searched for by BLAST. For example, in the case of applying the method of (b) to human OR2A25, a clade most proximal to the 18 genes selected as a result of the phylogenetic tree analysis in (a) was selected. In this clade, 20 genes belonged, and human OR2A7 and human OR2A4 were contained as olfactory receptor genes. These 20 genes were included in top 50 genes of a primate gene group having high homology to human OR2A25. In this way, 20 genes including the paralogs were selected, and the amino acid sequences of a total of 38 genes further including the 18 genes used in (a) (Table 3) were subjected to alignment analysis and consensus amino acid identification as mentioned below.

When the olfactory receptor of interest was a human olfactory receptor, the identification of orthologs in (c) was carried out by selecting genes having the same name as that of the olfactory receptor of interest as orthologs from top homologous genes searched for by BLAST. For example, in the case of applying the method (c) to human OR2A25, 67 genes involving OR2A25 in their names were selected from among top 250 homologous genes searched for by BLAST setting the amino acid sequence of human OR2A25 (NP_001004488.1) to a query sequence. In addition, as for *Mus musculus* and *Rattus norvegicus* genes for which a different olfactory receptor nomenclatural system is used, one gene each having the highest homology was selected from among the genes included in the top 250 genes as a result of the search. These 69 genes were identified as orthologs. The amino acid sequences of a total of 70 genes consisting of these 69 genes plus human OR2A25 (Table 3) were subjected to alignment analysis and consensus amino acid identification as mentioned below.

When the olfactory receptor of interest was each of human olfactory receptors OR2T11, OR2W1, OR4Q3, OR4S2, OR6Y1, OR7D4, OR7A17, and OR11H4, an ortholog group or ortholog and paralog groups were identified in the same manner as above. The amino acid sequences of genes of the identified ortholog group or ortholog and paralog groups plus the original human olfactory receptor gene were subjected to alignment analysis and consensus amino acid identification as mentioned below.

The alignment analysis on the identified gene group was conducted using Clustal W, and the alignment was further adjusted and optimized with reference to highly conserved amino acids or amino acid motifs among olfactory receptors. Ballesteros-Weinstein residue numbering was performed with reference to results of aligning all mouse olfactory receptors shown in Non Patent Literature 4. On the basis of the alignment results, consensus olfactory receptor design was performed using Jalview. In the alignment, when one amino acid residue which was different from the amino acid residue of a reference amino acid sequence and had a frequency of appearance of 50% or more existed at a position corresponding to each amino acid position in the amino acid sequence of the original human olfactory receptor serving as the reference, the amino acid residue of the reference amino acid sequence was altered to the amino acid residue. Even in the case where one amino acid residue which was different from the amino acid residue of a reference amino acid sequence and had a frequency of appearance of 50% existed at a position corresponding to each amino acid position in the amino acid sequence of the original human olfactory receptor serving as the reference, the amino acid residue of the reference amino acid sequence was not altered when the amino acid residue of the reference amino acid sequence also had a frequency of appearance of 50%. In the alignment, when deletion existed with a frequency of appearance of 40% or more at a position corresponding to each amino acid position in the amino acid sequence of the original human olfactory receptor serving as the reference, the amino acid residue in the reference amino acid sequence was altered to deleted. For example, when deletion existed with a frequency of appearance of 40% or more at a position corresponding to a C-terminal amino acid position, the amino acid residue of the reference amino acid sequence was altered to deleted. When deletion was found with a frequency of appearance of 40% or more at a position corresponding to an initiation methionine position in the human sequence, the first methionine in the amino acid sequence thus altered by the procedures described above was selected as initiation methionine and an amino acid sequence upstream thereof was deleted. Thus, when a consensus residue nearest to the N terminus was a consensus residue at a position corresponding to the N terminus of the olfactory receptor of interest or a C-terminal side thereof and was not a methionine residue, a consensus residue on an N-terminal side of a consensus residue consisting of a methionine residue positioned nearest to the N terminus was changed to the absence of a consensus residue. On the other hand, when an amino acid existed with a frequency of appearance of 60% or more at a position corresponding to a deletion position in the amino acid sequence of the original human olfactory receptor serving as the reference, alteration was made so as to insert the most highly conservative amino acid at the deletion position of the reference amino acid sequence. For example, when a consensus amino acid existed with a frequency of appearance of 60% or more at a position corresponding to a C-terminal deletion position in the amino acid sequence of the original human olfactory receptor serving as the reference, alteration was made so as to insert the most highly conservative amino acid at the C-terminus. When two or more amino acids were most highly conservative, alteration was made so as to insert thereat an amino acid having the smallest molecular weight.

Olfactory receptor topology in the design was confirmed using TMHMM (transmembrane Hidden Markov model). DNA sequences encoding various olfactory receptor polypeptides thus designed were obtained by DNA synthesis after optimization of nucleotide sequence codons corresponding to the amino acid sequences for expressions in human cultured cells. EcoRI and XhoI sites were added to both ends of the nucleotide sequence and recombined with EcoRI and XhoI sites prepared downstream of a Flag-Rho tag sequence in a pME18S vector. A gene encoding human RTP1S for translocation of an olfactory receptor protein produced in cultured cells onto cell membranes was incorporated into EcoRI and XhoI sites of another pME18S vector to prepare a pME18S-RTP1S vector.

**[Table 3]**

| (a) | | (b) | | (c) | | | |
|---|---|---|---|---|---|---|---|
| 1 | NP_001004488.1 | 1 | NP_001004488.1 | 1 | NP_001004488.1 | 41 | XP_024846708.1 |
| 2 | XP_003820570.1 | 2 | XP_003820570.1 | 2 | XP_003820570.1 | 42 | XP_027962159.1 |
| 3 | XP_527948.3 | 3 | XP_527948.3 | 3 | XP_527948.3 | 43 | XP_005886803.1 |
| 4 | XP_004046463.1 | 4 | XP_004046463.1 | 4 | XP_004046463.1 | 44 | XP_005679621.2 |
| 5 | XP_003270919.1 | 5 | XP_003270919.1 | 5 | XP_003270919.1 | 45 | XP_020766952.1 |
| 6 | XP_011845308.1 | 6 | XP_011845308.1 | 6 | XP_011845308.1 | 46 | XP_019484660.1 |
| 7 | XP_025236749.1 | 7 | XP_025236749.1 | 7 | XP_025236749.1 | 47 | XP_024420338.1 |
| 8 | NP_001180903.1 | 8 | NP_001180903.1 | 8 | NP_001180903.1 | 48 | XP_025710621.1 |
| 9 | XP_021792059.1 | 9 | XP_021792059.1 | 9 | XP_021792059.1 | 49 | XP_004772159.1 |
| 10 | XP_011912955.1 | 10 | XP_011912955.1 | 10 | XP_011912955.1 | 50 | XP_027429786.1 |
| 11 | XP_007981509.1 | 11 | XP_007981509.1 | 11 | XP_007981509.1 | 51 | XP_011376358.1 |
| 12 | XP_023046787.1 | 12 | XP_023046787.1 | 12 | XP_023046787.1 | 52 | NP_667199.1 |
| 13 | XP_011796792.1 | 13 | XP_011796792.1 | 13 | XP_011796792.1 | 53 | NP_001000846.1 |
| 14 | XP_002818656.2 | 14 | XP_002818656.2 | 14 | XP_002818656.2 | 54 | XP_012412736.2 |
| 15 | XP_003791583.1 | 15 | XP_003791583.1 | 15 | XP_003791583.1 | 55 | XP_028381731.1 |
| 16 | XP_020145275.1 | 16 | XP_020145275.1 | 16 | XP_020145275.1 | 56 | XP_006910505.1 |
| 17 | XP_012506030.1 | 17 | XP_012506030.1 | 17 | XP_012506030.1 | 57 | XP_003420628.1 |
| 18 | XP_008057851.1 | 18 | XP_008057851.1 | 18 | XP_007090917.1 | 58 | XP_016012670.1 |
| | | 19 | XP_012302993.1 | 19 | XP_008152181.1 | 59 | XP_028381668.1 |
| | | 20 | XP_003929872.1 | 20 | XP_008057851.1 | 60 | XP_004409659.1 |
| | | 21 | XP_017831027.1 | 21 | XP_003983225.1 | 61 | XP_028381669.1 |
| | | 22 | XP_015303692.1 | 22 | XP_014934312.1 | 62 | XP_029810838.1 |
| | | 23 | XP_011727930.1 | 23 | XP_006159670.1 | 63 | XP_017513807.1 |
| | | 24 | XP_025236055.1 | 24 | XP_022365921.1 | 64 | XP_010985279.1 |
| | | 25 | XP_001118110.3 | 25 | XP_014643256.1 | 65 | XP_010952531.1 |
| | | 26 | XP_011912966.1 | 26 | XP_001503794.2 | 66 | XP_002921862.1 |
| | | 27 | XP_012506027.1 | 27 | XP_025785816.1 | 67 | XP_028381686.1 |
| | | 28 | XP_017749246.1 | 28 | XP_021548542.1 | 68 | XP_014420418.1 |
| | | 29 | XP_003896835.1 | 29 | XP_012589341.1 | 69 | XP_028381694.1 |
| | | 30 | XP_011845314.1 | 30 | XP_028381605.1 | 70 | XP_017198162.1 |
| | | 31 | XP_010377726.1 | 31 | XP_027396106.1 | | |
| | | 32 | XP_012367550.1 | 32 | XP_008695504.1 | | |
| | | 33 | XP_017363822.1 | 33 | XP_008527468.1 | | |
| | | 34 | XP_012623594.1 | 34 | XP_025874141.1 | | |
| | | 35 | XP_003791581.1 | 35 | XP_025289742.1 | | |
| | | 36 | XP_002818667.2 | 36 | XP_030151663.1 | | |
| | | 37 | NP_001005328.1 | 37 | XP_025147526.1 | | |
| | | 38 | NP_112170.1 | 38 | XP_026370229.1 | | |
| | | | | 39 | XP_004008194.1 | | |
| | | | | 40 | XP_010843156.1 | | |

### 2) Preparation of cultured cell transformed with olfactory receptor gene

For luciferase assay, a reaction solution was prepared according to composition shown in Table 4, left standing for 20 minutes in a clean bench, and then added to each well of a 96-well plate (Becton, Dickinson and Company). Subsequently, 100 µL of HEK293 cells suspended in DMEM (Nacalai Tesque, Inc.) was inoculated at 2 × 10⁵ cells/cm² to each well, and cultured for 24 hours in an incubator kept at 37°C and 5% CO₂. Cells expressing no olfactory receptor (Mock) were provided as a control.

For flow cytometry, 3.3 × 10⁵ cells were inoculated to each well of a 6-well dish, and 24 hours later, a reaction solution was prepared according to composition shown in Table 5, left standing for 20 minutes in a clean bench, and then added to each well of the 6-well dish. The cells were cultured for 24 hours in an incubator kept at 37°C and 5% CO₂. In an experiment shown in Figure 3, the culture was performed for 42 hours as to the original receptors of OR7A17 and OR7D4. Cells expressing no receptor (Mock) were provided as a control.

**[Table 4]**

| Reaction solution composition: per well of 96-well plate | |
|---|---|
| DMEM (Nacalai) | 10 µL |
| Olfactory receptor gene (incorporated in pME18S vector) | 0.075 µg |
| pGL4.29 (fluc2P- CRE- hygro, Promega) | 0.03 µg |
| pGL4.75 (hRluc- CMV, Promega) | 0.03 µg |
| pME18S- RTPLS | 0.03 µg |
| Polyethylenimine- MAX (0.1% Aqueous solution (pH7.4), COSMO BIO) | 0.4 µL |

**[Table 5]**

| | |
|---|---|
| DMEM (Nacalai) | 100 µL |
| Olfactory receptor gene (incorporated in pME18S vector) | 3.0 µg |
| pME18S- RTP1S | 1.0 µg |
| Polyethylenimine- MAX (0.1% Aqueous solution (pH7.4), COSMO BIO) | 10 µL |

### 3) Measurement of olfactory receptor protein level on cell membrane (flow cytometry)

An anti-FLAG antibody (Cosmo Bio Co., Ltd.) was allowed to act as a primary antibody on cells recovered using a cell stripper on ice for 1 hour. After washing of the cells, PE (phycoerythrin)-conjugated anti-mouse IgG (Abeam plc.) was allowed to act thereon as a secondary antibody on ice for 30 minutes. After washing, 0.25 µg of 7-AAD (7-aminoactinomycin D, FUJIFILM Wako Pure Chemical Corp.) was added thereto, and a mean of PE signals of a 7-AAD-negative cell population was measured as an index for a receptor level on cell membranes using a flow cytometry system (Becton, Dickinson and Company). In each experiment run, a mean of PE signals was determined in the same manner as above by using cells expressing a FLAG-M2 acetylcholine receptor as a positive control (PC) and cells expressing no receptor as a negative control (NC). Normalization was performed with the NC PE signal defined as 0% and the PC PE signal defined as 100%. The PE signals (%) of various olfactory receptors were illustrated as membrane expression levels (Cell surface expression).

### 4) Luciferase assay

The medium was removed from the cultures prepared in 2), and 75 µL of a test substance solution with a predetermined concentration prepared with a fresh medium was added thereto. The cells were cultured for 4 hours in a CO₂ incubator and the luciferase gene was sufficiently expresses in the cells. Luciferase activity was measured using Dual-Glo(TM) luciferase assay system (Promega Corp.) in accordance with the operation manual of the product. A luminescence value derived from firefly luciferase induced by test substance stimulation in each well of the 96-well plate was divided by a luminescence value derived from Renilla luciferase to calculate a signal, which was then used in analysis. The signal under each transfection condition was normalized with the signal under a stimulation-free condition defined as 0% and the signal by stimulation with 30 µM forskolin defined as 100%, and used as a response (%) in analysis.

### 5) Results

### (i) Consensus design of olfactory receptor

First, 9 types of olfactory receptors (OR2A25, OR2T11, OR2W1, OR4Q3, OR4S2, OR6Y1, OR7D4, OR7A17, and OR11H4) already associated with ligands were selected and tested for their consensus design. The selection of receptors whose ligands are known is important because an assessment approach using ligand responsiveness as an index can be adopted even if a membrane expression level is improved in a range that cannot be experimentally detected.

Next, the following 3 types were selected as to evolutionary ranges where homologous genes are targeted to determine consensus amino acids for the selected olfactory receptors: (a) Primate ortholog = primate orthologs, (b) Primate ortholog/paralog = primate orthologs and the most related paralog, and (c) Ortholog = orthologs.

Table 6 shows the number of amino acid substitutions introduced for preparing a consensus olfactory receptor as to each olfactory receptor, the number of reference genes (the total number of the original olfactory receptor gene and the identified primate orthologs, primate orthologs and most related paralog, or orthologs), and the minimum homology thereamong. The numbers within the parentheses in the number of reference genes in (c) represent the number of primate orthologs among the reference genes. As a result of designing consensus olfactory receptors for the 9 olfactory receptors, the number of substitutions introduced to the original human olfactory receptor (307 amino acids on average) was 9.3 amino acids on average in the method (a), whereas the number of substitutions was increased to 27.1 and 19.9 amino acids on average in (b) and (c), respectively. Thus, consensus among more diverse genes was reflected. In all the methods, a large number of homologous genes were used for determining consensus amino acids in comparison with 10 genes in Non Patent Literature 2 except for 7 genes of primate orthologs of OR7A17 found to have no increase in membrane expression.

**[Table 6]**

| | (a) | | | (b) | | | (c) | | |
|---|---|---|---|---|---|---|---|---|---|
| | The number of amino acid substitutions | The number of reference genes | Minimum homology | The number of amino acid substitutions | The number of reference genes | Minimum homology | The number of amino acid substitutions | The number of reference genes | Minimum homology |
| 2A25 | 10 | 18 | 82 | 33 | 38 | 79 | 25 | 70 (17) | 74 |
| 2T11 | 10 | 16 | 78 | 75 | 45 | 69 | 14 | 95 (13) | 83 |
| 2W1 | 11 | 19 | 87 | 39 | 50 | 61 | 19 | 192 (18) | 61 |
| 4Q3 | 7 | 24 | 87 | 10 | 44 | 58 | 22 | 133 (24) | 58 |
| 4S2 | 7 | 23 | 96 | 26 | 47 | 61 | 14 | 250 (22) | 66 |
| 6Y1 | 10 | 26 | 87 | 7 | 48 | 58 | 13 | 177 (25) | 66 |
| 7A17 | 4 | 8 | 77 | 20 | 50 | 77 | 25 | 146 (43) | 77 |
| 7D4 | 13 | 36 | 89 | 20 | 50 | 70 | 27 | 242 (32) | 74 |
| 11H4 | 12 | 24 | 88 | 14 | 44 | 68 | 20 | 127 (23) | 78 |
| Average | 9.3 | 21.6 | 85.7 | 27.1 | 46.2 | 66.8 | 19.9 | 170.7 (24.1) | 70.8 |

### (ii) Increase in membrane expression level by consensus design

As a result of analysis using flow cytometry, all the 9 types of olfactory receptors markedly increased a membrane expression level by any of the 3 types of consensus design methods (Figure 1 and Table 7). In other words, the possibility was indicated that the application of all the 3 types of consensus design methods can increase the membrane expression levels of 100% olfactory receptors in terms of probability. The number of olfactory receptors having increased average membrane expression level in comparison with the original human olfactory receptor was 7 (approximately 78%) in the method (a) Primate ortholog, 8 (approximately 89%) in the method (b) Primate ortholog/paralog, and 9 (100%) in the method (c) Ortholog. Thus, the possibility was found that these methods are more versatile than the approach of Non Patent Literature 2 with a success rate of one out of 3 (approximately 33%) in response analysis. In the consensus olfactory receptor group which exhibited increase in membrane expression level, the consensus design was not necessarily introduced at a common amino acid site, revealing that an amino acid site for the consensus design differs depending on the type of a receptor.

**[Table 7]**

| | Human | | (a) Primate ortholog | | (b) Primate ortholog/paralog | | (c) Ortholog | |
|---|---|---|---|---|---|---|---|---|
| | Membrane expression level (%) | SEM | Membrane expression level (%) | SEM | Membrane expression level (%) | SEM | Membrane expression level (%) | SEM |
| 2A25 | 0.33 | 0.38 | 6.00 | 0.00 | 1.67 | 0.19 | 32.00 | 2.03 |
| 2T11 | -0.33 | 0.19 | 16.67 | 0.69 | 1.67 | 0.19 | 6.67 | 0.38 |
| 2W1 | 9.00 | 0.00 | 15.00 | 0.67 | 4.33 | 1.92 | 33.67 | 1.26 |
| 4Q3 | 0.67 | 0.19 | 11.33 | 0.19 | 8.00 | 0.33 | 13.33 | 0.51 |
| 4S2 | 2.33 | 0.38 | 1.00 | 0.33 | 46.00 | 0.33 | 3.33 | 0.19 |
| 6Y1 | 2.67 | 0.19 | 21.67 | 1.07 | 5.67 | 0.38 | 37.67 | 0.69 |
| 7A17 | 0.66 | 0.19 | 0.56 | 0.33 | 1.05 | 0.00 | 2.64 | 0.19 |
| 7D4 | -0.33 | 0.18 | 1.00 | 0.17 | 7.00 | 0.19 | 0.67 | 0.27 |
| 11H4 | 0.33 | 0.38 | 7.33 | 0.77 | 2.33 | 0.19 | 5.00 | 0.33 |

### (iii) Improvement in odor responsiveness by consensus design

Next, in order to test whether improvement in membrane expression level would lead to higher sensitivity as odor responsiveness, ligand responsiveness was measured as to each receptor (Figure 1). As a result, the following two points were found: when the membrane expression level is improved by the consensus design, the responsiveness is enhanced in almost all cases. The degree of improvement in membrane expression level does not bear a simple proportionate relationship with the degree of elevation in responsiveness. If the membrane expression level is improved even slightly, the responsiveness may be sufficiently improved. In the latter case, elevation in ligand responsiveness was found in association with increase in membrane expression level by the consensus design, as in 2T11, 6Y1, and 11H4, for example. On the other hand, no elevation in ligand responsiveness was found in some cases even if the membrane expression level was increased by the consensus design in comparison with the original human receptor, as in 2W1. The original human OR2W1 may exhibit a sufficient membrane expression level in itself and have no need for intracellular signal amplification even if the membrane expression level is further increased. Furthermore, the ligand responsiveness was sufficiently elevated in some cases even if the membrane expression level was slightly increased in comparison with the original human receptor, as in the consensus receptor of (b) Primate ortholog/paralog of 2A25 and the consensus receptor of (c) Ortholog of 7D4.

### (iv) Change in ligand selectivity by consensus design

An approach of introducing amino acid alteration, such as consensus design, might change a ligand binding site of the original olfactory receptor and consequently cause inherent odor responsiveness to be no longer observed.

Accordingly, among the 9 types of olfactory receptors selected this time, 5 types of receptors, 2A25, 2T11, 2W1, 11H4, and 7D4, found to have a plurality of agonists were examined for whether or not the consensus design of (a), (b), or (c) would change the ranking of affinity for 2 types of agonists (Figure 2). All the consensus design methods were found to maintain the same ranking of affinity as that of the original receptor. It was revealed that, for example, 2A25 responded to geraniol at a lower concentration in comparison with geranyl formate, whereas its response selectivity was maintained in 3 patterns of consensus receptors.

In order to further test the possibility that the consensus design would change ligand selectivity, the test was conducted using known agonists themselves and many structurally similar substances thereof (Table 8) to examine whether or not the consensus design would change the ranking of responsiveness (Figure 3). As a result, for all the olfactory receptors, an odorant which caused the highest response of the original human receptor caused the highest responses of the consensus receptors. In addition, no case was found where the consensus design canceled a response to an odorant to which the original human receptor responded. It can therefore be understood that the ligand selectivity is generally maintained. A plurality of cases, such as the case of 6Y1, were obtained where when the membrane expression level was increased by the consensus design, the consensus design was found to cause a response to an odorant to which the original human receptor did not respond. Although this result seems like a result brought about by change in ligand selectivity, it can be considered in reality that elevation in overall responsiveness enabled evaluation of a response to a ligand to which the original olfactory receptor was able to respond on olfactoryneurons. In fact, 6Y1 responds to diacetyl of odorant #5, as disclosed in Non Patent Literature 2, and this result is reasonable in well explaining an olfactory event measured by sensory evaluation (the gene differing between persons who strongly perceive odor of diacetyl and persons who weakly perceive odor of diacetyl is 6Y1). This demonstrated that an odorant for which response measurement was attained for the first time by the consensus design did not reflect a mere background and rather correctly reflected a human sense of smell. This will also be further supported by Examples mentioned below.

On the other hand, as for 7A17, odorant #6 caused a larger response of the original human receptor in comparison with odorant #4. However, when the consensus design was applied by the method (c) Ortholog, a plurality of cases, such as a tendency that reversed the ranking, were found where the response selectivity was not completely identical. However, such change in ligand selectivity in association with increase in membrane expression level is probably reasonable, as reported in preceding studies. For example, the report of Zhuang et al. (J Biol Chem. 2007 May 18; 282 (20): 15284-93) discloses that as a result of analyzing change in ligand selectivity at varying expression levels of the same olfactory receptor on cultured cell membranes, the change was found. The reasonability of the change has been discussed by quoting the preceding finding that in G protein-coupled receptors including olfactory receptors, their responses to, particularly, ligands having weak agonistic activity, can be detected with markedly high sensitivity when intracellular signals are amplified in association with increase in membrane expression level. Importantly, it has been considered that since olfactory receptors are highly expressed on olfactory neurons, ligand selectivity exhibited by the olfactory receptors placed under a high-expression condition on cultured cell membranes also reflects the physiological properties of the olfactory receptors.

**[Table 8]**

| | 4Q3 | 7D4 | 6Y1 | 11H4 | 7A17 |
|---|---|---|---|---|---|
| No | Odorant | Odorant | Odorant | Odorant | Odorant |
| 1 | geranyl propionate | 8-drimanol | acetone | geranyl propionate | okoumal |
| 2 | phenyl ethyl formate | ambrinol | butyric acid | *l*-citronellyl acetate | ysamber K |
| 3 | skatole | muscone | butanol | phenoxy ethanol iso-butyrate | ambrinol |
| 4 | benzophenone | amberketal | 2-peotanone | phenyl ethyl acetate | 8-drimanol |
| 5 | phenylethylacetate | androstadienone | diacetyl | phenyl ethyl alcohol | ambroxan |
| 6 | phenoxy ethanol i-butyrate | androstenone | acetoin | phenyl ethyl formate | iso E super |
| 7 | *l*-citronellyl acetate | okoumal | acetic acid | phenyl ethyl salicylate | amberketal |
| 8 | isovaleric acid | civettone | 2-butanone | skatole | civettone |
| 9 | *l*-citronellol | ysamber K | 1 -propanol | isovaleric acid | muscone |
| 10 | | Iso E super | | *l*-citronellol | |

| | 4S2 | 2W1 | 2T11 | 2A25 | |
|---|---|---|---|---|---|
| No | Odorant | Odorant | Odorant | Odorant | |
| 1 | dibutyl sulfide | lyral | furfuryl methylsulfide | isovaleric acid | |
| 2 | cis-3-hexenol | euqenol | dibutyl sulfide | phenoxy ethanol iso-butyrate | |
| 3 | cis-2-pentenol | benzyl saficilate | methyl mercaptan | geranyl formate | |
| 4 | sulfide allyl methyl | citronellol | furfuryl mercaptan | phenyl ethyl salicylate | |
| 5 | furfuryl methylsulfide | 1-hexanol | tert butyl mercaptan | geraniol | |
| 6 | methyl mercaptan | carvone | dimethyl disulfide | *l*-citronellol | |
| 7 | dimethyl trisulfide | octanol | sulfide allyl methyl | geranyl propionate | |
| 8 | trans-3-hexenol | benzyl acetate | dimethyl trisulfide | *l*-citronellyl acetate | |
| 9 | dimethyl sulfide | citral | dimethyl sulfide | phenyl ethyl formate | |
| 10 | furfuryl mercaptan | cinnamic aldehyde | | skatole | |
| 11 | | limonene | | | |
| 12 | | geraniol | | | |
| 13 | | linalool | | | |
| 14 | | benzyl alcohol | | | |
| 15 | | lilial | | | |
| 16 | | diacetyl | | | |

Example 2: Effectiveness of consensus design for olfactory receptor never before possible to functionally analyze - 1

The studies described above were carried out as to olfactory receptors which can be expressed on membranes of cultured cells and are possible to functionally analyze, for example, olfactory receptors whose ligands have been identified. The consensus design according to the present invention was tested for the actual degree of its effectiveness for yet-to-be analyzed olfactory receptors.

Non Patent Literature 2 and the report of Eriksson et al. (Flavour 1: 22 (2012)) disclose that comparative genome analysis was carried out as to a population containing individuals differing in perception of each individual odor, thereby selecting candidates of an olfactory receptor responsible for sensitivity to the odorant. In other words, although these reports have identified ligand candidate substances for olfactory receptors, responsiveness to the ligand candidate substances has not yet been demonstrated as to 34 types of olfactory receptors (human olfactory receptors in Table 10). As reasons for the unsuccessful response measurement, the possibilities have been pointed out that these 34 types of olfactory receptors were false positive i.e., correct ligand substances could not be identified, in the first place; and although correct ligand substances could be identified, responses thereto could not be detected due to nonefficient membrane expression in cultured cells. Accordingly, the consensus design was applied to these 34 types of olfactory receptors by the method (c) Ortholog for the olfactory receptors other than 11H1 and by the method (d) for 11H1 among the 3 patterns, (a) to (c), described above.

In the consensus design by the method of (d), paralogs were identified which had higher homology to an olfactory receptor of interest and permitted identification of at least 11 types of orthologs. An ortholog group of the paralogs was selected. When the olfactory receptor of interest was human OR11H1, the identification of the ortholog group was attempted in the same manner as in the case of Example 1 (c) except that the amino acid sequence of human OR11H1 (NP_001005239) was set to a query sequence. However, only 6 genes were selected as the ortholog group. Accordingly, human OR11H12 (NP_001013372.1) was focused on as a paralog having high homology to human OR11H1 from among top 250 homologous genes searched for by setting the amino acid sequence of human OR11H1 to a query sequence. An ortholog group of OR11H12 having high homology to human OR11H1 was identified, and 90 genes were selected as orthologs. The amino acid sequences of a total of 98 genes consisting of these 90 genes plus human OR11H1, 6 genes of orthologs of human OR11H1, and human OR11H12 (Table 9) were subjected to alignment analysis and consensus amino acid identification in the same manner as in Example 1 for the consensus design of the olfactory receptors.

**[Table 9]**

| | | | | | | | |
|---|---|---|---|---|---|---|---|
| 1 | NP_001005239.1 | 26 | XP_004694755.1 | 51 | XP_030176066.1 | 76 | XP_025295049.1 |
| 2 | NP_001013372.1 | 27 | XP_004865164.2 | 52 | XP_004010989.4 | 77 | XP_022283407.1 |
| 3 | XP_016783170.1 | 28 | XP_019286005.1 | 53 | XP_004609780.1 | 78 | XP_006218179.1 |
| 4 | XP_018866080.2 | 29 | XP_004584687.1 | 54 | XP_026346423.1 | 79 | XP_025848063.1 |
| 5 | XP_002833829.1 | 30 | XP_010807442.3 | 55 | XP_004781801.1 | 80 | XP_027975244.1 |
| 6 | XP_027687761.1 | 31 | XP_020955667.1 | 56 | XP_010604553.1 | 81 | XP_027426141.1 |
| 7 | XP_001141680.2 | 32 | XP_006877203.1 | 57 | XP_021551384.1 | 82 | XP_011216635.3 |
| 8 | XP_014199111.1 | 33 | XP_010831734.1 | 58 | XP_007538130.1 | 83 | XP_020011212.1 |
| 9 | XP_012608311.2 | 34 | XP_027408653.1 | 59 | XP_032273398.1 | 84 | XP_006877225.1 |
| 10 | XP_012504714.1 | 35 | XP_005908917.2 | 60 | XP_001927243.3 | 85 | XP_025739510.1 |
| 11 | XP_001504682.4 | 36 | XP_005908916.1 | 61 | XP_008708659.1 | 86 | XP_019060578.2 |
| 12 | XP_007956255.1 | 37 | XP_023111945.1 | 62 | XP_010948004.1 | 87 | XP_017524110.1 |
| 13 | XP_008531921.1 | 38 | XP_022377345.1 | 63 | XP_012906255.1 | 88 | XP_004865143.2 |
| 14 | XP_003423385.1 | 39 | XP_007091476.2 | 64 | XP_006191275.2 | 89 | XP_006149489.2 |
| 15 | XP_014700051.1 | 40 | XP_008837990.2 | 65 | XP_010995612.1 | 90 | XP_006877223.1 |
| 16 | XP_020756322.1 | 41 | XP_008682196.1 | 66 | XP_030881029.1 | 91 | XP_012892248.1 |
| 17 | XP_029808074.1 | 42 | XP_027408592.1 | 67 | XP_019823488.1 | 92 | XP_006877222.1 |
| 18 | XP_004456757.3 | 43 | XP_025775053.1 | 68 | XP_032197647.1 | 93 | XP_030740791.1 |
| 19 | XP_027785489.1 | 44 | XP_004391087.1 | 69 | XP_023581563.1 | 94 | XP_023398216.1 |
| 20 | XP_005685482.2 | 45 | XP_014921853.1 | 70 | XP_006052449.2 | 95 | XP_019060577.1 |
| 21 | XP_026267801.1 | 46 | XP_020756331.1 | 71 | XP_013363999.1 | 96 | XP_003128838.2 |
| 22 | XP_015336369.1 | 47 | XP_032712983.1 | 72 | XP_006075641.2 | 97 | XP_023398176.1 |
| 23 | XP_004421406.1 | 48 | XP_034850470.1 | 73 | XP_004670211.1 | 98 | XP_002690724.2 |
| 24 | XP_013221191.1 | 49 | XP_019823487.1 | 74 | XP_017909295.1 | | |
| 25 | XP_006883328.1 | 50 | XP_002690799.1 | 75 | XP_006877224.1 | | |

Table 10 shows the number of reference genes (the total number of the original olfactory receptor gene and the identified orthologs) used for preparing the consensus olfactory receptors. Among the olfactory receptors, 2W3, 2Y1, 3A2, 4C15, 4D5, 4D6, 4F15, 10D3, 10Q1, 11H1, and 13G1 were tested at n = 4 while the other olfactory receptors were tested at n = 3. As a result of measuring expression levels of these receptors on cell membranes, increase in average value was found in 29 (approximately 85%) receptors (Figure 4 and Table 11). This suggested that the consensus design enables functional analysis on many receptors never before possible to analyze. As for the remaining 5 receptors having no increase in membrane expression level, their expression levels may be increased by applying the consensus design (a) or (b).

**[Table 10]**

| | The number of reference genes | | The number of reference genes | | The number of reference genes | | The number of reference genes |
|---|---|---|---|---|---|---|---|
| 2AG1 | 130 | 4C15 | 245 | 6B2 | 182 | 11H1 | 98 |
| 2AG2 | 135 | 4D5 | 133 | 6C1 | 245 | 13C8 | 136 |
| 2D2 | 196 | 4D6 | 135 | 10A2 | 97 | 13G1 | 205 |
| 2D3 | 248 | 4F15 | 155 | 10A4 | 130 | 14J1 | 243 |
| 2T10 | 36 | 4K15 | 186 | 10A5 | 133 | 52A4 | 19 |
| 2W3 | 227 | 5A2 | 112 | 10D3 | 89 | 56A1 | 102 |
| 2Y1 | 103 | 5C1 | 133 | 10Q1 | 246 | 56A4 | 197 |
| 3A2 | 134 | 5W2 | 233 | 10R2 | 250 | | |
| 4A16 | 76 | 6A2 | 174 | 10Z1 | 131 | | |

**[Table 11]**

| | Original | | Consensus | | | Original | | Consensus | |
|---|---|---|---|---|---|---|---|---|---|
| | Membrane expression level (%) | SEM | Membrane expression level (%) | SEM | | Membrane expression level (%) | SEM | Membrane expression level (%) | SEM |
| 2AG1 | 0.18 | 0.05 | 3.83 | 0.24 | 5W2 | -0.08 | 0.08 | 10.59 | 0.80 |
| 2AG2 | 0.44 | 0.08 | 2.89 | 0.17 | 6A2 | 0.09 | 0.05 | 9.64 | 0.34 |
| 2D2 | 0.75 | 0.25 | 1.74 | 0.19 | 6B2 | 1.81 | 0.39 | 21.19 | 0.94 |
| 2D3 | -0.09 | 0.05 | 3.80 | 0.19 | 6C1 | -0.07 | 0.06 | 0.20 | 0.14 |
| 2W3 | 2.73 | 0.12 | 5.28 | 0.20 | 10A2 | -0.06 | 0.06 | 7.08 | 0.14 |
| 2Y1 | 2.19 | 0.21 | 16.42 | 0.74 | 10A4 | 0.94 | 0.04 | 2.21 | 0.18 |
| 3A2 | -0.02 | 0.03 | 12.41 | 0.73 | 10D3 | 0.33 | 0.08 | 1.10 | 0.40 |
| 4A16 | 0.88 | 0.25 | 16.98 | 0.94 | 10Q1 | 0.28 | 0.07 | 19.84 | 0.27 |
| 4C15 | 0.62 | 0.19 | 17.02 | 1.86 | 10Z1 | 0.41 | 0.15 | 38.16 | 0.79 |
| 4D5 | 1.61 | 0.18 | 2.65 | 0.50 | 11H1 | 0.22 | 0.05 | 25.34 | 1.87 |
| 4D6 | 0.70 | 0.04 | 1.37 | 0.11 | 13C8 | 0.43 | 0.11 | 2.00 | 0.31 |
| 4F15 | 0.38 | 0.08 | 5.92 | 0.56 | 13G1 | 0.44 | 0.07 | 17.96 | 1.13 |
| 4K15 | 0.74 | 0.35 | 24.14 | 1.07 | 52A4 | 0.31 | 0.20 | 0.54 | 0.21 |
| 5A2 | 0.01 | 0.06 | 1.60 | 0.09 | 56A4 | 0.01 | 0.08 | 0.42 | 0.16 |
| 5C1 | 0.52 | 0.18 | 13.63 | 0.51 | | | | | |

Subsequently, whether increase in membrane expression level by the consensus design would actually bring about success in response measurement was tested. Non Patent Literature 2 has reported 6B2, 5C1, and 10D3 as olfactory receptors related to sensitivity to isobutyraldehyde, citral, and isoeugenol, respectively. The approach of Non Patent Literature 2 has failed to express these olfactory receptors in cultured cells and to demonstrate their responsiveness to the corresponding odorants. This time, the consensus design by the method of (c) Ortholog was applied to these olfactory receptors, and response analysis was carried out. As a result, the responses became possible to detect for the first time by the consensus design in some cases (Figure 5). Importantly, 6B2 is a receptor reported to fail response analysis to isobutyraldehyde in Non Patent Literature 2, though its consensus design was attempted in 10 mammals. The consensus design according to the present invention enabled analysis of 6B2 as a method having higher versatility than that of the previous report.

Likewise, the report of Eriksson et al. (Flavour 1: 22 (2012)) has identified 8 olfactory receptor candidates (2AG2, 2AG1, 6A2, 10A2, 10A5, 10A4, 2D2, and 2D3) which explain difference in perception of coriander-derived odor among individuals. However, the approach of this report has failed functional analysis to actually identify any receptor as a receptor for a coriander-derived odorant. Accordingly, in the present research, the consensus design of (c) Ortholog was applied to these 8 receptors, and response analysis was carried out on major aroma components ((E)-2-decenal and (E)-2-dodecenal) (Figure 6). The obtained results revealed for the first time that 10A2 and 10A4 can recognize these odorants by the application of the consensus design.

Example 3: Effectiveness of consensus design for olfactory receptor never before possible to functionally analyze - 2

Whether responsiveness coinciding with difference among individuals in sensory evaluation would be obtained was tested by applying the consensus design (c) to each human olfactory receptor newly identified in Figures 5 and 6, and then introducing an amino acid sequence reported to have difference among individuals (Figure 7).

For example, it has been reported for OR10A2 that the frequency of appearance of an amino acid sequence registered as NP_001004460.1 is 68% in a population, whereas an amino acid sequence having three mutations, H43R, H207R, and K258T, appears with a frequency of 32% (the frequencies of appearance are from the 1000 genomes project phase 3 allele frequencies). Among these three mutations, H207R is also a consensus amino acid identified by the method (c). These two types of amino acid sequences found in a population are each presumed to bring about change in responsiveness to major aroma components ((E)-2-decenal and (E)-2-dodecenal) of coriander and consequently bring about difference in human perception of coriander flavor as unpleasant soap-like one or not. As a result of an experiment, the variant of H43R, H207R, and K258T was found to have markedly low responsiveness to the aroma components, as expected. The olfactory receptor 10A4 having lower responsiveness to the aroma components than that of 10A2 has also been reported to have a R262Q variant which appears with a frequency of 31% in a population. However, this variant brought about no change in responsiveness to the aroma components. Thus, 10A2 became possible to identify as a receptor which explains difference in perception of coriander flavor among individuals, for the first time by the application of the consensus design method.

Likewise, 6B2 is found to have a variant of R122C and C179R with a frequency of 1% in a population (test participants in Non Patent Literature 2), and according to Non Patent Literature 2, this population cannot strongly perceive odor of isobutyraldehyde having a low concentration. When the R122C and C179R mutations were introduced to the consensus olfactory receptor 6B2 so as to coincide with this difference among individuals in sensory evaluation, it was confirmed that the responsiveness to isobutyraldehyde was markedly reduced.

5C1 is found to have a N5K variant with a frequency of 1% in a population (1000 genomes project phase 3 allele frequencies), and according to Non Patent Literature 2, this population cannot strongly perceive odor of citral having a low concentration. When the N5K mutation was introduced to the consensus olfactory receptor 5C1 so as to coincide with this difference among individuals in sensory evaluation, it was confirmed that the responsiveness to citral was markedly reduced.

These results revealed that the consensus design method of the present invention is very effective for assessing a human sense of smell.

### Example 4: Applicable range of consensus design method - 1

Olfactory receptor gene families are a very highly diverse gene group, and the homology of olfactory receptor between different families falls below 40%. Thus, individual olfactory receptors can also be considered as totally different G protein-coupled receptors. Accordingly, a further test was conducted in order to clarify the general effectiveness of the consensus design method of the present invention for olfactory receptors, i.e., the applicable range of the consensus design method of the present invention. The test was conducted as to an olfactory receptor group other than the 9 types of Example 1 and the 34 types of Example 2 described above. Specifically, the consensus design by the method (c) Ortholog or (d) was applied to target olfactory receptors (58 types of human olfactory receptors in Figure 8 and Table 12). The consensus design (c) was applied to the olfactory receptors other than OR2T7 and OR4F5, and the consensus design (d) was applied to OR2T7 and OR4F5.

As for OR7E24 among the olfactory receptors to which the consensus design (c) was applied, a consensus residue nearest to the N terminus was a consensus residue at a position corresponding to a C-terminal side of the N terminus of the original human OR7E24 serving as a reference, and was not a methionine residue. Therefore, if a consensus residue on an N-terminal side of a consensus residue consisting of a methionine residue positioned nearest to the N terminus is changed to the absence of a consensus residue, the full-length of the amino acid sequence of the resulting consensus receptor is shorter by 10% or more than the full-length of the amino acid sequence of the original human OR7E24. Hence, instead of this method, the consensus residue nearest to the N terminus was substituted by methionine.

In the consensus design by the method (d), when the olfactory receptor of interest was human OR4F5, the identification of an ortholog group was attempted in the same manner as in the case of Example 1 (c) except that the amino acid sequence of human OR4F5 (NP_001005484.2) was set to a query sequence. However, no ortholog of OR4F5 was identified from other organism species. Accordingly, human OR4F4 (NP_001004195.2) was focused on as a paralog having high homology to human OR4F5 from among top 250 homologous genes searched for by setting the amino acid sequence of human OR4F5 to a query sequence. An ortholog group of OR4F4 having high homology to human OR4F5 was identified, and 99 genes were selected as orthologs. The amino acid sequences of a total of 101 genes consisting of these 99 genes plus human OR4F5 and human OR4F4 were subjected to alignment analysis and consensus amino acid identification in the same manner as in Example 1 for the consensus design of the olfactory receptors.

When the olfactory receptor of interest was human OR2T7, the identification of an ortholog group was attempted in the same manner as in the case of Example 1 (c) except that the amino acid sequence of human OR2T7 (NP_001372981.1) was set to a query sequence. However, only 5 genes were selected as the ortholog group. Accordingly, human OR2T27 was focused on as a paralog having the highest homology to human OR2T7 from among top 250 homologous genes searched for by setting the amino acid sequence of human OR2T7 to a query sequence. As a result of identifying an ortholog group, 100 genes were selected as the ortholog group. The amino acid sequences of a total of 107 genes consisting of these 105 genes plus human OR2T7 and human OR2T27 were subjected to alignment analysis and consensus amino acid identification in the same manner as in Example 1 for the consensus design of the olfactory receptors.

Figure 8 and Table 12 show cases where the membrane expression level was increased by the method (c) or (d). Table 13 shows the number of reference genes (the total number of the original olfactory receptor gene and the identified orthologs or orthologs and paralogs) used for preparing the consensus olfactory receptors. The results of Figure 8 and Table 12 revealed that the consensus design method of the present invention can increase membrane expression levels of an exceedingly large number of olfactory receptors.

**[Table 12]**

| | Original | | Consensus | | | Original | | Consensus | |
|---|---|---|---|---|---|---|---|---|---|
| | Membrane expression level (%) | SEM | Membrane expression level (%) | SEM | | Membrane expression level (%) | SEM | Membrane expression level (%) | SEM |
| 2B11 | 1.22 | 0.19 | 8.86 | 0.23 | 4K17 | 0.13 | 0.01 | 9.09 | 0.04 |
| 2C3 | 0.60 | 0.02 | 6.62 | 0.15 | 4L1 | 0.11 | 0.03 | 10.44 | 0.17 |
| 2G2 | -0.08 | 0.01 | 3.39 | 0.08 | 4M1 | 0.57 | 0.03 | 2.25 | 0.01 |
| 2G3 | 0.14 | 0.03 | 1.22 | 0.05 | 4N2 | 0.05 | 0.07 | 2.61 | 0.03 |
| 2L2 | 0.18 | 0.05 | 10.16 | 0.58 | 4N5 | 0.87 | 0.02 | 1.69 | 0.08 |
| 2L3 | 5.61 | 0.35 | 7.42 | 0.43 | 5A1 | 1.06 | 0.08 | 9.77 | 0.38 |
| 2L5 | 0.13 | 0.05 | 2.34 | 0.11 | 6F1 | 0.10 | 0.04 | 5.52 | 0.09 |
| 2L8 | 0.57 | 0.09 | 7.55 | 0.40 | 6J1 | 0.05 | 0.05 | 0.24 | 0.03 |
| 2L13 | 0.21 | 0.04 | 16.71 | 0.50 | 7A10 | 0.82 | 0.12 | 1.15 | 0.13 |
| 2M2 | 0.07 | 0.02 | 0.34 | 0.04 | 7C2 | 1.67 | 0.55 | 2.98 | 0.37 |
| 2M4 | -0.01 | 0.02 | 4.33 | 0.05 | 7G2 | 0.34 | 0.14 | 2.06 | 0.40 |
| 2T1 | 0.13 | 0.04 | 7.09 | 0.24 | 7G3 | 0.92 | 0.30 | 3.82 | 0.86 |
| 2T4 | 0.11 | 0.02 | 3.05 | 0.24 | 10G2 | 0.50 | 0.03 | 25.73 | 1.21 |
| 2T5 | 1.60 | 0.69 | 19.52 | 0.59 | 10J1 | 0.49 | 0.04 | 5.94 | 0.33 |
| 2T6 | 0.54 | 0.09 | 3.10 | 0.20 | 10J3 | 0.04 | 0.03 | 5.96 | 0.36 |
| 2T7 | 0.04 | 0.01 | 10.57 | 0.78 | 10K1 | 2.85 | 0.14 | 9.85 | 0.64 |
| 2T8 | 0.24 | 0.07 | 6.16 | 0.31 | 10K2 | 0.15 | 0.12 | 1.34 | 0.06 |
| 2T12 | 0.79 | 0.05 | 2.79 | 0.22 | 10T2 | 0.08 | 0.07 | 9.83 | 0.77 |
| 2T27 | 0.44 | 0.14 | 24.05 | 1.00 | 10X1 | 0.18 | 0.04 | 1.23 | 0.18 |
| 2T29 | 1.45 | 0.65 | 14.34 | 0.22 | 11G2 | 0.02 | 0.05 | 25.19 | 2.95 |
| 2T33 | 0.46 | 0.05 | 15.32 | 0.41 | 11H6 | 0.08 | 0.05 | 0.36 | 0.09 |
| 2T34 | 3.74 | 1.21 | 6.44 | 1.26 | 11H7 | 0.25 | 0.05 | 15.18 | 0.80 |
| 2T35 | -0.19 | 0.02 | 1.63 | 0.36 | 11L1 | 1.06 | 0.24 | 4.26 | 1.20 |
| 2AK2 | 0.89 | 0.06 | 7.87 | 0.63 | 14A2 | -0.01 | 0.01 | 0.27 | 0.07 |
| 4D10 | 0.07 | 0.06 | 2.73 | 0.21 | 14A16 | -0.13 | 0.02 | 1.33 | 0.37 |
| 4E2 | 1.01 | 0.22 | 21.22 | 1.67 | 14C36 | 0.05 | 0.01 | 5.35 | 0.22 |
| 4F5 | 0.27 | 0.06 | 11.99 | 0.60 | 14K1 | 0.48 | 0.02 | 1.84 | 0.09 |
| 4K1 | 0.32 | 0.02 | 1.27 | 0.11 | 7E24 | 0.48 | 0.17 | 1.47 | 0.50 |
| 4K2 | 0.69 | 0.09 | 12.06 | 0.35 | | | | | |
| 4K5 | 0.11 | 0.01 | 0.44 | 0.03 | | | | | |

**[Table 13]**

| | The number of reference genes | | The number of reference genes | | The number of reference genes | | The number of reference genes |
|---|---|---|---|---|---|---|---|
| 2B11 | 201 | 2T7 | 107 | 4K17 | 56 | 10K1 | 147 |
| 2C3 | 115 | 2T8 | 127 | 4L1 | 226 | 10K2 | 117 |
| 2G2 | 138 | 2T12 | 23 | 4M1 | 157 | 10T2 | 188 |
| 2G3 | 69 | 2T27 | 103 | 4N2 | 118 | 10X1 | 214 |
| 2L2 | 45 | 2T29 | 179 | 4N5 | 116 | 11G2 | 249 |
| 2L3 | 38 | 2T33 | 98 | 5A1 | 197 | 11H6 | 154 |
| 2L5 | 56 | 2T34 | 87 | 6F1 | 107 | 11H7 | 130 |
| 2L8 | 113 | 2T35 | 134 | 6J1 | 111 | 11L1 | 210 |
| 2L13 | 215 | 2AK2 | 185 | 7A10 | 86 | 14A2 | 223 |
| 2M2 | 12 | 4D10 | 103 | 7C2 | 141 | 14A16 | 107 |
| 2M4 | 77 | 4E2 | 99 | 7G2 | 144 | 14C36 | 250 |
| 2T1 | 127 | 4F5 | 101 | 7G3 | 134 | 14K1 | 120 |
| 2T4 | 60 | 4K1 | 209 | 10G2 | 230 | 7E24 | 106 |
| 2T5 | 192 | 4K2 | 213 | 10J1 | 241 | | |
| 2T6 | 121 | 4K5 | 128 | 10J3 | 148 | | |

### Example 5: Applicable range of consensus design method - 2

The applicability of the consensus design method was tested as to an olfactory receptor group other than the 9 types of Example 1, the 34 types of Example 2, and the 58 types of Example 4 described above. Specifically, the consensus design by the method (c) Ortholog or (d) was applied to target olfactory receptors (156 types of human olfactory receptors in Tables 14 and 15). The consensus design (c) was applied to the olfactory receptors other than OR1D4, OR3A3, OR11H2, OR51A2, and OR52E6, and the consensus design (d) was applied to OR1D4, OR3A3, OR11H2, OR51A2, and OR52E6.

As for OR9K2 among the olfactory receptors to which the consensus design (c) was applied, the full-length of the amino acid sequence of the consensus receptor was increased by 10% or more on an N-terminal side than the full-length of the amino acid sequence of the original human olfactory receptor. Therefore, an N-terminal structure of the original human olfactory receptor was maintained as it was in the amino acid sequence of the consensus receptor.

Tables 14 and 15 show cases where the membrane expression level was increased by the method (c) or (d) (n = 3). Table 16 shows the number of reference genes (the total number of the original olfactory receptor gene and the identified orthologs or orthologs and paralogs) used for preparing the consensus olfactory receptors. The results of Tables 14 and 15 revealed that the consensus design method of the present invention can increase membrane expression levels of an exceedingly large number of olfactory receptors.

**[Table 14]**

| | Original | | Consensus | | | Original | | Consensus | |
|---|---|---|---|---|---|---|---|---|---|
| | Membrane expression level (%) | SEM | Membrane expression level (%) | SEM | | Membrane expression level (%) | SEM | Membrane expression level (%) | SEM |
| 1B1 | -0.01 | 003 | 0.34 | 0.04 | 3A3 | 0.09 | 0.04 | 38.28 | 1.01 |
| 1D2 | 1.91 | 0.11 | 7.29 | 0.28 | 4A5 | -0.13 | 0.03 | 11.42 | 0.16 |
| 1D4 | 0.11 | 0.03 | 0.76 | 0.05 | 4A15 | -0.14 | 0.02 | 0.49 | 0.01 |
| 1E2 | 0.04 | 0.05 | 0.31 | 0.07 | 4A47 | -0.06 | 0.01 | 0.42 | 0.00 |
| 1F1 | 0.19 | 0.03 | 4.81 | 0.18 | 4B1 | -0.08 | 0.03 | 1.69 | 0.04 |
| 1G1 | 0.17 | 0.03 | 2.56 | 0.13 | 4C3 | 0.48 | 0.09 | 37.94 | 0.54 |
| 1I1 | 2.61 | 1.20 | 22.72 | 0.57 | 4C6 | 0.19 | 0.03 | 27.20 | 0.25 |
| 1J1 | 0.23 | 0.03 | 13.30 | 0.81 | 4C11 | 0.05 | 0.03 | 6.08 | 0.22 |
| 1J4 | 0.15 | 0.03 | 22.78 | 2.92 | 4C12 | 0.40 | 0.10 | 7.42 | 0.16 |
| 1K1 | 2.21 | 0.06 | 11.94 | 0.37 | 4C16 | 0.15 | 0.03 | 2.48 | 0.16 |
| 1L1 | 0.33 | 0.04 | 13.38 | 0.29 | 4C46 | 0.46 | 0.03 | 8.06 | 0.27 |
| 1L3 | 0.59 | 0.03 | 0.80 | 0.05 | 4D1 | 0.35 | 0.04 | 4.41 | 0.13 |
| 1L4 | 0.42 | 0.02 | 15.66 | 0.63 | 4D2 | 0.17 | 0.03 | 2.69 | 0.12 |
| 1L6 | -0.18 | 0.07 | 43.43 | 1.69 | 4D9 | 0.00 | 0.01 | 12.89 | 0.18 |
| 1L8 | 0.02 | 0.03 | 3.02 | 0.22 | 4D11 | -0.01 | 0.01 | 20.11 | 0.23 |
| 1M1 | 2.01 | 0.06 | 4.66 | 0.05 | 4F17 | 0.07 | 0.01 | 0.25 | 0.02 |
| 1Q1 | 0.35 | 0.03 | 18.47 | 0.05 | 4F21 | 0.28 | 0.02 | 1.78 | 0.11 |
| 1S1 | -0.07 | 0.02 | 3.99 | 0.12 | 4P4 | -0.02 | 0.01 | 0.99 | 0.02 |
| 1S2 | -0.10 | 0.02 | 0.02 | 0.03 | 4X1 | 0.03 | 0.01 | 2.47 | 0.06 |
| 2A1 | 0.32 | 0.08 | 59.91 | 0.63 | 4X2 | 0.34 | 0.01 | 20.63 | 0.25 |
| 2A5 | 0.30 | 0.03 | 22.72 | 0.53 | 5B2 | 0.06 | 0.01 | 0.70 | 0.01 |
| 2A7 | 0.39 | 0.04 | 0.96 | 0.06 | 5B12 | 0.25 | 0.05 | 9.64 | 0.22 |
| 2A14 | 1.32 | 0.34 | 5.87 | 0.26 | 5B17 | 0.05 | 0.00 | 0.55 | 0.03 |
| 2B2 | 0.22 | 0.12 | 1.18 | 0.14 | 5H1 | 0.22 | 0.09 | 1.68 | 0.03 |
| 2B3 | 0.54 | 0.15 | 0.70 | 0.14 | 5H2 | 1.90 | 0.00 | 24.30 | 0.52 |
| 2B6 | 0.18 | 0.07 | 1.20 | 0.14 | 5H6 | 1.78 | 0.05 | 3.56 | 0.12 |
| 2C1 | 1.92 | 0.10 | 29.81 | 0.77 | 5H14 | 0.61 | 0.03 | 1.53 | 0.11 |
| 2F1 | 0.16 | 0.05 | 1.03 | 0.09 | 5K3 | 0.39 | 0.09 | 2.32 | 0.19 |
| 2F2 | 0.27 | 0.03 | 0.39 | 0.02 | 5AK2 | 0.03 | 0.01 | 1.87 | 0.08 |
| 2H1 | 0.24 | 0.05 | 11.71 | 0.16 | 5AR1 | 0.72 | 0.29 | 21.76 | 0.25 |
| 2H2 | 0.30 | 0.02 | 1.66 | 0.10 | 5AS1 | 0.19 | 0.04 | 0.64 | 0.05 |
| 2K2 | 0.04 | 0.04 | 0.54 | 0.04 | 6B1 | 20.51 | 1.37 | 55.75 | 3.93 |
| 2S2 | 0.50 | 0.05 | 9.22 | 0.30 | 6B3 | 0.41 | 0.06 | 18.20 | 0.20 |
| 2V1 | 0.33 | 0.05 | 2.45 | 0.10 | 6C2 | 0.46 | 0.02 | 0.68 | 0.02 |
| 2V2 | 0.38 | 0.07 | 3.25 | 0.38 | 6C3 | 0.36 | 0.02 | 1.31 | 0.04 |
| 2Z1 | -0.01 | 0.01 | 0.60 | 0.01 | 6C4 | 0.07 | 0.01 | 5.68 | 0.10 |
| 2AE1 | 0.47 | 0.02 | 15.68 | 1.10 | 6C6 | 0.08 | 0.06 | 5.36 | 0.15 |
| 2AP1 | 0.38 | 0.02 | 17.61 | 0.19 | 6C65 | 0.02 | 0.06 | 2.75 | 0.07 |
| 2AT4 | 0.06 | 0.01 | 0.41 | 0.03 | 6C68 | 0.50 | 0.05 | 1.19 | 0.06 |
| 3A1 | 0.12 | 0.01 | 42.79 | 0.32 | 6C70 | 0.14 | 0.04 | 0.82 | 0.06 |

**[Table 15]**

| | Original | | Consensus | | | Original | | Consensus | |
|---|---|---|---|---|---|---|---|---|---|
| | Membrane expression level (%) | SEM | Membrane expression level (%) | SEM | | Membrane expression level (%) | SEM | Membrane expression level (%) | SEM |
| 6C74 | 0.02 | 0.05 | 3.73 | 0.12 | 51B4 | 0.18 | 0.04 | 55.86 | 0.13 |
| 6C75 | -0.07 | 0.04 | 2.61 | 0.07 | 51B5 | 0.53 | 0.04 | 5.59 | 0.01 |
| 6C76 | -0.05 | 0.03 | 1.92 | 0.09 | 51B6 | 0.35 | 0.13 | 15.21 | 0.71 |
| 6K3 | 0.10 | 0.06 | 20.73 | 0.51 | 51D1 | 2.22 | 0.14 | 19.27 | 0.77 |
| 6K6 | 0.06 | 0.03 | 7.09 | 0.31 | 51E2 | 29.92 | 1.09 | 32.72 | 1.44 |
| 6N2 | 0.75 | 0.06 | 2.37 | 0.18 | 51F1 | -0.02 | 0.06 | 15.26 | 0.82 |
| 8S1 | 0.05 | 0.06 | 1.50 | 0.10 | 51 F2 | -0.01 | 0.08 | 0.16 | 0.08 |
| 9A2 | 0.24 | 0.04 | 0.40 | 0.05 | 51G1 | 0.21 | 0.07 | 0.67 | 0.06 |
| 9A4 | 0.22 | 0.05 | 0.70 | 0.06 | 51G2 | 0.14 | 0.08 | 4.01 | 0.28 |
| 10A3 | -0.02 | 0.03 | 11.16 | 0.33 | 51I1 | -0.05 | 0.07 | 3.71 | 0.23 |
| 10A7 | 0.11 | 0.05 | 0.73 | 0.11 | 51I2 | 0.65 | 0.10 | 4.22 | 0.10 |
| 10G6 | -0.05 | 0.03 | 2.50 | 0.20 | 51J1 | -0.12 | 0.07 | 0.01 | 0.06 |
| 10G9 | 0.19 | 0.03 | 1.60 | 0.04 | 51L1 | 0.62 | 0.03 | 17.83 | 1.37 |
| 10H1 | 2.03 | 0.02 | 20.05 | 0.24 | 51M1 | -0.05 | 0.02 | 3.26 | 0.29 |
| 10H2 | 2.77 | 0.09 | 23.43 | 0.40 | 51Q1 | 0.09 | 0.04 | 7.74 | 0.53 |
| 10H3 | 0.15 | 0.03 | 1.68 | 0.05 | 51S1 | 4.24 | 0.37 | 67.95 | 4.75 |
| 10H4 | 0.13 | 0.02 | 0.52 | 0.06 | 51T1 | 0.30 | 0.01 | 29.44 | 1.41 |
| 10H5 | 1.40 | 0.06 | 7.87 | 0.13 | 51V1 | 0.08 | 0.00 | 14.15 | 1.28 |
| 10P1 | 0.44 | 0.05 | 9.57 | 0.07 | 52A1 | -0.01 | 0.02 | 8.46 | 0.67 |
| 10S1 | 0.06 | 0.03 | 19.03 | 0.36 | 52A5 | -0.06 | 0.02 | 2.41 | 0.22 |
| 10V1 | 0.00 | 0.04 | 5.83 | 0.12 | 52B2 | 0.06 | 0.01 | 15.35 | 1.15 |
| 10W1 | 0.01 | 0.02 | 14.67 | 0.54 | 52B4 | 0.29 | 0.02 | 24.02 | 1.75 |
| 10AD1 | 0.76 | 0.03 | 25.11 | 0.36 | 52B6 | -0.09 | 0.01 | 0.63 | 0.04 |
| 10AG1 | 0.51 | 0.02 | 5.40 | 0.10 | 52D1 | 0.16 | 0.01 | 0.97 | 0.03 |
| 11A1 | 1.04 | 0.14 | 19.24 | 0.54 | 52E5 | 0.05 | 0.01 | 16.11 | 1.37 |
| 11H2 | 0.18 | 0.07 | 50.95 | 2.61 | 52E6 | 0.34 | 0.04 | 1.70 | 0.14 |
| 12D2 | 0.08 | 0.03 | 13.51 | 0.40 | 52H1 | 0.22 | 0.01 | 1.93 | 0.16 |
| 12D3 | 0.23 | 0.06 | 17.42 | 0.36 | 52I1 | 0.09 | 0.02 | 15.09 | 1.35 |
| 13A1 | -0.09 | 0.06 | 21.10 | 0.51 | 5212 | 0.14 | 0.01 | 26.13 | 2.28 |
| 13C2 | 0.22 | 0.04 | 6.21 | 0.51 | 52J3 | 0.07 | 0.01 | 6.24 | 0.54 |
| 13C3 | 0.06 | 0.04 | 9.10 | 0.55 | 52K1 | 0.02 | 0.01 | 0.17 | 0.01 |
| 13C4 | 0.02 | 0.03 | 0.31 | 0.03 | 52K2 | 0.00 | 0.02 | 0.35 | 0.02 |
| 13C9 | 3.75 | 0.23 | 8.49 | 0.70 | 52N1 | 0.31 | 0.07 | 3.48 | 0.17 |
| 13F1 | 0.36 | 0.04 | 6.67 | 0.49 | 52R1 | 0.29 | 0.06 | 5.08 | 0.20 |
| 51A2 | 0.01 | 0.01 | 0.32 | 0.04 | 52W1 | 0.21 | 0.06 | 2.46 | 0.15 |
| 51A4 | 0.08 | 0.02 | 0.23 | 0.03 | 56A5 | 0.39 | 0.05 | 1.38 | 0.08 |
| 51A7 | 0.25 | 0.04 | 15.74 | 0.09 | 56B4 | 0.30 | 0.07 | 1.13 | 0.04 |
| 51B2 | 0.63 | 0.04 | 29.44 | 0.36 | 9K2 | 0.10 | 0.06 | 0.28 | 0.05 |

**[Table 16]**

| | The number of reference genes | | The number of reference genes | | The number of reference genes | | The number of reference genes |
|---|---|---|---|---|---|---|---|
| 1B1 | 144 | 3A3 | 153 | 6C74 | 134 | 51B6 | 164 |
| 1D2 | 219 | 4A5 | 160 | 6C75 | 170 | 51D1 | 152 |
| 1D4 | 49 | 4A15 | 211 | 6C76 | 195 | 51E2 | 222 |
| 1E2 | 48 | 4A47 | 247 | 6K3 | 250 | 51F1 | 215 |
| 1F1 | 180 | 4B1 | 221 | 6K6 | 137 | 51F2 | 233 |
| 1G1 | 87 | 4C3 | 122 | 6N2 | 164 | 51G1 | 143 |
| 1I1 | 151 | 4C6 | 240 | 8S1 | 242 | 51G2 | 244 |
| 1J1 | 104 | 4C11 | 245 | 9A2 | 27 | 51I1 | 158 |
| 1J4 | 183 | 4C12 | 243 | 9A4 | 211 | 51I2 | 169 |
| 1K1 | 118 | 4C16 | 233 | 10A3 | 165 | 51J1 | 107 |
| 1L1 | 89 | 4C46 | 221 | 10A7 | 124 | 51L1 | 149 |
| 1L3 | 119 | 4D1 | 162 | 10G6 | 98 | 51M1 | 132 |
| 1L4 | 171 | 4D2 | 90 | 10G9 | 137 | 51Q1 | 227 |
| 1L6 | 75 | 4D9 | 138 | 10H1 | 189 | 51S1 | 152 |
| 1L8 | 235 | 4D11 | 132 | 10H2 | 86 | 51T1 | 146 |
| 1M1 | 29 | 4F17 | 31 | 10H3 | 151 | 51V1 | 244 |
| 1Q1 | 124 | 4F21 | 16 | 10H4 | 96 | 52A1 | 247 |
| 1S1 | 150 | 4P4 | 244 | 10H5 | 26 | 52A5 | 240 |
| 1S2 | 22 | 4X1 | 106 | 10P1 | 124 | 52B2 | 245 |
| 2A1 | 160 | 4X2 | 221 | 10S1 | 152 | 52B4 | 248 |
| 2A5 | 128 | 5B2 | 150 | 10V1 | 242 | 52B6 | 135 |
| 2A7 | 28 | 5B12 | 193 | 10W1 | 178 | 52D1 | 232 |
| 2A14 | 108 | 5B17 | 53 | 10AD1 | 241 | 52E5 | 157 |
| 2B2 | 157 | 5H1 | 25 | 10AG1 | 241 | 52E6 | 207 |
| 2B3 | 112 | 5H2 | 129 | 11A1 | 227 | 52H1 | 242 |
| 2B6 | 74 | 5H6 | 16 | 11H2 | 98 | 52I1 | 93 |
| 2C1 | 90 | 5H14 | 36 | 12D2 | 232 | 52I2 | 155 |
| 2F1 | 51 | 5K3 | 171 | 12D3 | 136 | 52J3 | 244 |
| 2F2 | 23 | 5AK2 | 91 | 13A1 | 233 | 52K1 | 192 |
| 2H1 | 103 | 5AR1 | 28 | 13C2 | 108 | 52K2 | 59 |
| 2H2 | 138 | 5AS1 | 225 | 13C3 | 140 | 52N1 | 104 |
| 2K2 | 162 | 6B1 | 244 | 13C4 | 105 | 52R1 | 244 |
| 2S2 | 121 | 6B3 | 27 | 13C9 | 114 | 52W1 | 152 |
| 2V1 | 28 | 6C2 | 244 | 13F1 | 205 | 56A5 | 17 |
| 2V2 | 114 | 6C3 | 244 | 51A2 | 138 | 56B4 | 86 |
| 2Z1 | 220 | 6C4 | 210 | 51A4 | 137 | 9K2 | 246 |
| 2AE1 | 172 | 6C6 | 226 | 51A7 | 223 | | |
| 2AP1 | 102 | 6C65 | 95 | 51B2 | 145 | | |
| 2AT4 | 198 | 6C68 | 94 | 51B4 | 75 | | |
| 3A1 | 195 | 6C70 | 139 | 51B5 | 83 | | |

### Example 6: Applicable range of consensus design method - 3

The applicability of the consensus design method was tested as to an olfactory receptor group other than the 9 types of Example 1, the 34 types of Example 2, the 58 types of Example 4, and the 156 types of Example 5 described above. Specifically, the consensus design by the method (c) Ortholog or (d) was applied to target olfactory receptors (50 types of human olfactory receptors in Table 17). The consensus design (c) was applied to the olfactory receptors other than OR8H2, and the consensus design (d) was applied to OR8H2.

As for OR5I1 among the olfactory receptors to which the consensus design (c) was applied, a consensus residue nearest to the N terminus was a consensus residue at a position corresponding to a C-terminal side of the N terminus of the original human OR5I1 serving as a reference, and was not a methionine residue. Therefore, if a consensus residue on an N-terminal side of a consensus residue consisting of a methionine residue positioned nearest to the N terminus is changed to the absence of a consensus residue, the full-length of the amino acid sequence of the resulting consensus receptor is shorter by 10% or more than the full-length of the amino acid sequence of the original human OR5I1. Hence, instead of this method, the consensus residue nearest to the N terminus was substituted by methionine.

Table 17 shows cases where the membrane expression level was increased by the method (c) or (d) (n = 3). Table 18 shows the number of reference genes (the total number of the original olfactory receptor gene and the identified orthologs or orthologs and paralogs) used for preparing the consensus olfactory receptors. The results of Table 17 revealed that the consensus design method of the present invention can increase membrane expression levels of an exceedingly large number of olfactory receptors.

**[Table 17]**

| | Original | | Consensus | | | Original | | Consensus | |
|---|---|---|---|---|---|---|---|---|---|
| | Membrane expression level (%) | SEM | Membrane expression level (%) | SEM | | Membrane expression level (%) | SEM | Membrane expression level (%) | SEM |
| 2A2 | 0.35 | 0.08 | 0.76 | 0.12 | 6P1 | 1.26 | 0.07 | 9.83 | 0.69 |
| 2A12 | 0.79 | 0.19 | 10.47 | 2.45 | 6Q1 | 0.16 | 0.08 | 15.54 | 0.23 |
| 4S1 | 0.23 | 0.01 | 25.74 | 4.69 | 6T1 | -0.05 | 0.03 | 1.73 | 0.07 |
| 5AC2 | 0.86 | 0.06 | 1.75 | 0.35 | 6X1 | 0.30 | 0.17 | 4.58 | 0.17 |
| 5B3 | 0.45 | 0.04 | 7.05 | 0.30 | 8B4 | 0.15 | 0.05 | 2.29 | 0.08 |
| 5D13 | 0.29 | 0.02 | 35.61 | 2.00 | 8B8 | -0.08 | 0.03 | 0.26 | 0.05 |
| 5D14 | 0.20 | 0.00 | 1.11 | 0.02 | 8B12 | 0.22 | 0.11 | 15.66 | 0.27 |
| 5D16 | 0.14 | 0.02 | 7.04 | 0.18 | 8D1 | 8.41 | 0.24 | 24.38 | 0.33 |
| 5D18 | 0.11 | 0.02 | 34.39 | 2.27 | 8G1 | 0.85 | 0.05 | 3.44 | 0.07 |
| 5J2 | 1.38 | 0.05 | 13.92 | 0.73 | 8G5 | -0.02 | 0.03 | 0.40 | 0.03 |
| 5K4 | 0.19 | 0.04 | 1.17 | 0.23 | 8H2 | 0.05 | 0.04 | 4.86 | 0.14 |
| 5L1 | 0.22 | 0.03 | 7.42 | 0.29 | 8I2 | 1.57 | 0.04 | 25.51 | 0.30 |
| 5L2 | 0.23 | 0.03 | 9.99 | 0.36 | 8J1 | 2.63 | 0.09 | 8.18 | 0.13 |
| 5M1 | 0.33 | 0.03 | 0.99 | 0.05 | 8K1 | 1.84 | 0.08 | 2.24 | 0.10 |
| 5M3 | 0.18 | 0.01 | 2.95 | 0.14 | 8K3 | 0.61 | 0.05 | 9.84 | 0.74 |
| 5M8 | 0.13 | 0.01 | 1.31 | 0.07 | 8K5 | 0.19 | 0.02 | 7.08 | 0.54 |
| 5M9 | 0.25 | 0.02 | 9.91 | 0.44 | 8U1 | 0.50 | 0.05 | 0.72 | 0.02 |
| 5M10 | 0.90 | 0.03 | 1.21 | 0.04 | 9G1 | 0.16 | 0.01 | 10.71 | 0.80 |
| 5M11 | 0.06 | 0.02 | 4.54 | 0.44 | 9G4 | 0.15 | 0.02 | 3.59 | 0.27 |
| 5P2 | 0.09 | 0.01 | 17.01 | 0.61 | 9I1 | 0.26 | 0.01 | 9.37 | 0.67 |
| 5T1 | 1.19 | 0.03 | 0.71 | 0.03 | 11H12 | 0.10 | 0.03 | 34.26 | 2.14 |
| 5T2 | 0.08 | 0.01 | 0.52 | 0.03 | 52M1 | 0.17 | 0.08 | 0.39 | 0.11 |
| 5T3 | 0.10 | 0.05 | 6.56 | 0.09 | 52N5 | 0.14 | 0.01 | 0.50 | 0.07 |
| 6M1 | 0.02 | 0.02 | 3.06 | 0.03 | 56A3 | 0.50 | 0.08 | 0.78 | 0.13 |
| 6N1 | 0.41 | 0.01 | 14.69 | 0.97 | 5I1 | 0.04 | 0.02 | 3.54 | 0.11 |

**[Table 18]**

| | The number of reference genes | | The number of reference genes | | The number of reference genes | | The number of reference genes |
|---|---|---|---|---|---|---|---|
| 2A2 | 137 | 5M1 | 39 | 6Q1 | 160 | 8K3 | 246 |
| 2A12 | 98 | 5M3 | 180 | 6T1 | 106 | 8K5 | 232 |
| 4S1 | 89 | 5M8 | 141 | 6X1 | 216 | 8U1 | 22 |
| 5AC2 | 48 | 5M9 | 147 | 8B4 | 146 | 9G1 | 88 |
| 5B3 | 176 | 5M10 | 200 | 8B8 | 28 | 9G4 | 231 |
| 5D13 | 239 | 5M11 | 155 | 8B12 | 145 | 9I1 | 242 |
| 5D14 | 133 | 5P2 | 23 | 8D1 | 122 | 11H12 | 97 |
| 5D16 | 45 | 5T1 | 119 | 8G1 | 119 | 52M1 | 243 |
| 5D18 | 210 | 5T2 | 125 | 8G5 | 46 | 52N5 | 104 |
| 5J2 | 47 | 5T3 | 49 | 8H2 | 233 | 56A3 | 244 |
| 5K4 | 72 | 6M1 | 242 | 8I2 | 119 | 5I1 | 109 |
| 5L1 | 215 | 6N1 | 83 | 8J1 | 55 | | |
| 5L2 | 40 | 6P1 | 137 | 8K1 | 146 | | |

### Example 7: Applicable range of consensus design method - 4

The consensus design by the method (c) Ortholog was applied to OR5AN1 analyzed in detail as a musk receptor. However, the consensus design was not found to increase its membrane expression level (Figures 9A and 9B). On the other hand, when responsiveness to a known agonist was compared between the original OR5AN1 and consensus OR5AN1, the consensus design was found to elevate the responsiveness (odorant #1 to #8 in Figures 9C and 9D). The odorants used are shown in Table 19. Odorant #7 and #8 caused no response of the original OR5AN1 but caused a response of consensus OR5AN1. Although this result seems to suggest the possibility that the consensus design changed ligand selectivity from the original one, the possibility is presumably low. This is because odorant #7 and #8 have been shown to cause a response, albeit weak, of the original OR5AN1 according to the report of Sato-Akuhara et al. (J Neurosci. 36 (16): 4482-4491 (2016)). Thus, the original OR5AN1 exhibited no response to odorant #7 and #8 presumably because the present evaluation system had lower sensitivity than that in preceding studies. By contrast, the results showing a response by the consensus design is considered not to indicate abnormality in ligand selectivity, and probably indicate elevation in response sensitivity to an inherent agonist. Odorants #9 to #21 include a substance similar in chemical structure or odor quality to an OR5AN1 agonist, and further include a substance which does not activate OR5AN1 in the report of Sato-Akuhara et al. As a result of performing response measurement therefor, consensus OR5AN1 was shown to not respond to a substance which was not recognized by the original OR5AN1, and further supported no large change in ligand selectivity by the consensus design. The consensus design which elevates responsiveness, despite no increase in membrane expression level, is not limited by OR5AN1, and is also found in OR4S2 (Primate ortholog/paralog) and OR7A17 (Primate ortholog) in Figure 1. The possibility is suggested that such consensus design enhances the efficiency of shift of the receptor protein to an active structure, the efficiency of coupling to G protein, or the like. These results showed that the consensus design technique of the present invention may be effective for analyzing functions of olfactory receptors with high sensitivity, even if not increasing their membrane expression levels.

**[Table 19]**

| No. | Odorant |
|---|---|
| 1 | musk ketone |
| 2 | civettone |
| 3 | globanone ^{™} |
| 4 | cyclopentadecanone/exaltone |
| 5 | muscenone ^{™} delta |
| 6 | muscone |
| 7 | cyclopentadecanol |
| 8 | ethylene brassylate |
| 9 | ambrettolide |
| 10 | cyclododecanone |
| 11 | habanolide ^{™} |
| 12 | pentalide |
| 13 | galaxolide |
| 14 | tentarome/tonalid ^{™} |
| 15 | celestolide |
| 16 | helvetolide |
| 17 | romandolide ^{™} |
| 18 | ω-dodecanolactam |
| 19 | amber xtreme TM |
| 20 | beta-ionone |
| 21 | ambrinol |

### Example 8: Applicable range of consensus design method - 5

The consensus design was confirmed to be also effective for the analysis of mouse olfactory receptors. M71 (Olfr151, MOR171-2) was focused on as a typical mouse olfactory receptor analyzed from long ago, and the consensus design by the method (c) Ortholog was applied thereto. Specifically, 249 genes having high homology to the amino acid sequence of M71 were selected, and consensus amino acid sequences were determined. M71 obtained by the consensus design was inserted downstream of a FLAG tag and a Rho tag in a pME18S vector, as in the human olfactory receptors, and HEK293 cells were transfected with the resulting vector. Then, expression levels of proteins on cell membranes were measured 24 hours later (n = 3). As a result, the membrane expression level (Cell surface expression (%)) of the original M71 was 0.78 ± 0.07 (mean ± SEM), whereas the membrane expression level was increased to 3.20 ± 0.19 for M71 obtained by the consensus design. Thus, the consensus design was shown to be also effective for olfactory receptors of non-human organism species.

### Example 9: Identification of olfactory receptor which responds to sulfur compound

### 1) Preparation of olfactory receptor-expressing cell

A reaction solution as to each consensus olfactory receptor prepared in Examples described above was prepared according to the composition shown in Table 4, left standing for 20 minutes in a clean bench, and then added to each well of a 96-well plate (Becton, Dickinson and Company). The original olfactory receptor was used as to OR4S2. Composition shown in Table 20 was used as to OR11H2 and OR4E2. Subsequently, 100 µL of HEK293 cells suspended in DMEM (Nacalai Tesque, Inc.) was inoculated at 2 × 10⁵ cells/cm² to each well, and cultured for 24 hours in an incubator kept at 37°C and 5% CO₂. Cells expressing no olfactory receptor (Mock) were provided as a control.

**[Table 20]**

| Reaction solution composition: per well of 96-well plate | |
|---|---|
| DMEM (Nacalai) | 10 µL |
| Olfactory receptor gene (incorporated in pME18S vector) | 0.0075 µg |
| pGL4.29 (fluc2P-CRE-hygro, Promega) | 0.03 µg |
| pGL4.75 (hRluc2P-CMV-hygro, Promega) | 0.003 µg |
| pME18S-RTP1S | 0.03 µg |
| POLYETHYLENIMINE-MAX (0.1% Aqueous solution, pH7.4, COSMO BIO) | 0.18 µL |

### 2) Luciferase assay

The medium was removed from the cultures prepared in 1), and 75 µL of a test substance solution (methyl mercaptan (MeSH) (CAS: 74-93-1, Tokyo Chemical Industry Co., Ltd.), dimethyl sulfide (DMS) (CAS: 75-18-3, FUJIFILM Wako Pure Chemical Corp.), dimethyl disulfide (DMDS) (CAS: 624-92-0, Tokyo Chemical Industry Co., Ltd.), concentration range: 0 µM, 1 µM, 3 pM, 10 pM, 30 µM, 100 pM, and 300 pM, or dimethyl trisulfide (DMTS) (CAS: 3658-80-8, Tokyo Chemical Industry Co., Ltd.), concentration range: 0 pM, 0.1 pM, 0.3 pM, 1 µM, 3 µM, 10 µM, and 30 µM) prepared with a fresh medium was added thereto. Under a copper ion addition condition, the test substance solution was adjusted to contain 30 µM copper(II) chloride (FUJIFILM Wako Pure Chemical Corp.) in addition to the odorant. The cells were cultured for 4 hours in a CO₂ incubator and the luciferase gene was expressed sufficiently in the cells. Luciferase activity was measured using Dual-Glo(TM) luciferase assay system (Promega Corp.) in accordance with the operation manual of the product. A luminescence value derived from firefly luciferase induced by test substance stimulation in each well of the 96-well plate was divided by a luminescence value derived from Renilla luciferase to calculate a value (fluc/hRluc) as a signal, which was then used in analysis. The signal under each transfection condition was normalized with the signal value (fluc/hRluc) under an odorant stimulation-free condition defined as 0% and the signal value (fluc/hRluc) by stimulation with 10 µM forskolin defined as 100%, and used as a response (%) in analysis.

### 3) Results

Figure 10 shows olfactory receptors which responded to methyl mercaptan, dimethyl sulfide, dimethyl disulfide, or dimethyl trisulfide in the presence of a copper ion among the tested olfactory receptors. In Figure 10, response values to each concentration of methyl mercaptan, dimethyl sulfide, dimethyl disulfide, or dimethyl trisulfide are shown as to the individual olfactory receptors. Each olfactory receptor is a consensus olfactory receptor except for OR4S2. Statistically significant difference was found between the response value (Response (%)) of each olfactory receptor to the highest concentration of 300 µM (30 µM for DMTS) tested and the response value (Response (%)) in cells expressing no receptor under a Mock condition (Student's t-test, P < 0.05). For each olfactory receptor, significant difference was also found in the same statistical approach as above when the signal value (fluc/hRluc) under an odorant stimulation-free condition was compared with the signal value (fluc/hRluc) under a condition of stimulation at the highest concentration of 300 µM (30 µM for DMTS). The numeric values in Tables 21 to 24 are EC50 (µM) calculated by the regression of the value of Response (%) to a sigmoid curve. As listed in Figure 10, the olfactory receptors without the description of EC50 (µM) in Tables 21 to 24 mean that although the receptors can respond to methyl mercaptan, dimethyl sulfide, dimethyl disulfide, or dimethyl trisulfide, data sufficient for regression to a sigmoid curve was not obtained in the concentration range tested this time and no EC50 was calculated. Thus, consensus OR2T29, consensus OR4K17, consensus OR2T27, consensus OR2T5, consensus OR2T4, consensus OR11H2, consensus OR6B3, consensus OR12D3, consensus OR2T11, OR4S2, consensus OR2T1, consensus OR4C15, consensus OR4E2, and consensus OR2L13 responded to methyl mercaptan. Among them, consensus OR2T29, consensus OR2T5, consensus OR2T4, consensus OR2T11, consensus OR2T1, and consensus OR4E2 were also confirmed to have a dose-dependent response to methyl mercaptan. Consensus OR4K17, consensus OR11H2, OR4S2, consensus OR4C15, and consensus OR4E2 responded to dimethyl sulfide in a dose-dependent manner. Consensus OR2T29, consensus OR4K17, consensus OR2T27, consensus OR2T5, consensus OR2T4, consensus OR11H2, consensus OR6B3, consensus OR12D3, consensus OR2T11, OR4S2, consensus OR2T1, consensus OR4C15, consensus OR4E2, and consensus OR2L13 responded to dimethyl disulfide. Among them, consensus OR4K17, consensus OR6B3, consensus OR2T11, OR4S2, consensus OR4C15, and consensus OR4E2 were also confirmed to have a dose-dependent response to dimethyl disulfide. Consensus OR2T29, consensus OR2T4, consensus OR11H2, consensus OR6B3, consensus OR12D3, consensus OR6B1, consensus OR2T11, OR4S2, consensus OR2T1, consensus OR4C15, consensus OR4E2, and consensus OR2L13 responded to dimethyl trisulfide. Among them, consensus OR2T11, OR4S2, and consensus OR4E2 were also confirmed to have a dose-dependent response to dimethyl trisulfide. For example, OR2T11 and OR2T1 are known to respond to methyl mercaptan in the presence of a metal ion (JP-B-6122181). The results about consensus OR2T11 and consensus OR2T1 which responded to methyl mercaptan are consistent with the finding. Accordingly, consensus OR2T29, consensus OR4K17, consensus OR2T27, consensus OR2T5, consensus OR2T4, consensus OR11H2, consensus OR6B3, consensus OR12D3, consensus OR6B1, consensus OR2T11, OR4S2, consensus OR2T1, consensus OR4C15, consensus OR4E2, and consensus OR2L13 were found to respond to at least one sulfur compound selected from the group consisting of methyl mercaptan, dimethyl sulfide, dimethyl disulfide, and dimethyl trisulfide.

**[Table 21]**

| Consensus receptor | EC₅₀(µM) with 30 µM CuCl₂ |
|---|---|
| | MeSH |
| OR2T29 | 85 |
| OR2T5 | 217 |
| OR2T4 | 340 |
| OR2T11 | 5 |
| OR2T1 | 33 |
| OR4E2 | 250 |

**[Table 22]**

| Consensus receptor | EC₅₀(µM) with 30 µM CuCl₂ |
|---|---|
| | DMS |
| OR4K17 | 188 |
| OR11H2 | 22 |
| OR4S2 (original) | 22 |
| OR4C15 | 414 |
| OR4E2 | 22 |

**[Table 23]**

| Consensus receptor | EC₅₀ (pM) with 30µM CuCl₂ |
|---|---|
| | DMDS |
| OR4K17 | 568 |
| OR6B3 | 1597 |
| OR2T11 | 246 |
| OR4S2 (original) | 530 |
| OR4C15 | 615 |
| OR4E2 | 397 |

**[Table 24]**

| Consensus receptor | EC₅₀ (pM) with 30 µM CuCl₂ |
|---|---|
| | DMTS |
| OR2T11 | 26 |
| OR4S2 (original) | 28 |
| OR4E2 | 33 |

Figure 11 shows olfactory receptors improved in responsiveness to methyl mercaptan, dimethyl sulfide, dimethyl disulfide, or dimethyl trisulfide in the presence of a copper ion in comparison with that in the absence of the copper ion among the tested olfactory receptors. In Figure 11, signal values to 100 µM methyl mercaptan, dimethyl sulfide, dimethyl disulfide, or dimethyl trisulfide are shown as to the individual olfactory receptors. Each olfactory receptor is a consensus olfactory receptor except for OR4S2. Consensus OR2T29, consensus OR4K17, consensus OR2T27, consensus OR2T5, consensus OR2T4, consensus OR2T11, OR4S2, consensus OR2T1, consensus OR4C15, and consensus OR4E2 were improved in responsiveness to methyl mercaptan, dimethyl sulfide, dimethyl disulfide, or dimethyl trisulfide in the presence of a copper ion in comparison with that in the absence of the copper ion.

The response of a consensus olfactory receptor to an odorant reflects a response of the original olfactory receptor to olfactory cells. Accordingly, from the results described above, the original OR2T29, OR4K17, OR2T27, OR2T5, OR2T4, OR11H2, OR6B3, OR12D3, OR6B1, OR2T11, OR4S2, OR2T1, OR4C15, OR4E2, and OR2L13 were identified as receptors for a sulfur compound.

Tables 25-1 to 25-5 below show the amino acid sequences of the original olfactory receptors, the amino acid sequences of the consensus olfactory receptors, and the consensus design method used in Examples 1 to 9 described above.

**[Table 25-1]**

| No. | Olfactory receptor | Accession No. | SEQ ID NO of original amino acid sequence | SEQ ID NO of consensus amino acid sequence | Consensus design method | No. | Olfactory receptor | Accession No. | SEQ ID NO of original amino acid sequence | SEQ ID NO of consensus amino acid sequence | Consensus design method |
|---|---|---|---|---|---|---|---|---|---|---|---|
| 1 | OR2A25 | NP_001004488.1 | 1 | 97 | a | 31 | OR5W2 | NP_001001960.1 | 21 | 127 | c |
| 2 | OR2A25 | NP_001004488.1 | 1 | 98 | b | 32 | OR6A2 | NP_003687.2 | 22 | 128 | c |
| 3 | OR2A25 | NP_001004488.1 | 1 | 99 | c | 33 | OR6B2 | NP_001005853.1 | 23 | 129 | c |
| 4 | OR2AG1 | NP_001004489.1 | 2 | 100 | c | 34 | OR6C1 | NP_001005182.1 | 24 | 130 | c |
| 5 | OR2AG2 | NP_001004490.1 | 3 | 101 | c | 35 | OR6Y1 | NP_001005189.1 | 25 | 131 | a |
| 6 | OR2D2 | ALI87480.1 | 4 | 102 | c | 36 | OR6Y1 | NP_001005189.1 | 25 | 132 | b |
| 7 | OR2D3 | NP_001004684.1 | 5 | 103 | c | 37 | OR6Y1 | NP_001005189.1 | 25 | 133 | c |
| 8 | OR2T11 | NP_001001964.1 | 6 | 104 | a | 38 | OR7A17 | NP_112163.1 | 26 | 134 | a |
| 9 | OR2T11 | NP_001001964.1 | 6 | 105 | b | 39 | OR7A17 | NP_112163.1 | 26 | 135 | b |
| 10 | OR2T11 | NP_001001964.1 | 6 | 106 | c | 40 | OR7A17 | NP_112163.1 | 26 | 136 | c |
| 11 | OR2W1 | NP_112165.1 | 7 | 107 | a | 41 | OR7D4 | NP_001005191.1 | 27 | 137 | a |
| 12 | OR2W1 | NP_112165.1 | 7 | 108 | b | 42 | OR7D4 | NP_001005191.1 | 27 | 138 | b |
| 13 | OR2W1 | NP_112165.1 | 7 | 109 | c | 43 | OR7D4 | NP_001005191.1 | 27 | 139 | c |
| 14 | OR2W3 | NP_001001957.2 | 8 | 110 | c | 44 | OR10A2 | NP_001004460.1 | 28 | 140 | c |
| 15 | OR2Y1 | NP_001001657.1 | 9 | 111 | c | 45 | OR10A4 | ALI87721.1 | 29 | 141 | c |
| 16 | OR3A2 | NP_002542.3 | 10 | 112 | c | 46 | OR10D3 | NP_001342142.1 | 30 | 142 | c |
| 17 | OR4A16 | NP_001005274.1 | 11 | 113 | c | 47 | OR10Q1 | NP_001004471.1 | 31 | 143 | c |
| 18 | OR4C15 | NP_001001920.2 | 12 | 114 | c | 48 | OR10Z1 | NP_001004478.1 | 32 | 144 | c |
| 19 | OR4D5 | NP_001001965.1 | 13 | 115 | c | 49 | OR11H1 | NP_001005239 | 33 | 145 | d |
| 20 | OR4D6 | NP_001004708.1 | 14 | 116 | c | 50 | OR11H4 | NP_001004479.1 | 34 | 146 | a |
| 21 | OR4F15 | NP_001001674.1 | 15 | 117 | c | 51 | OR11H4 | NP_001004479.1 | 34 | 147 | b |
| 22 | OR4K15 | NP_001005486.1 | 16 | 118 | c | 52 | OR11H4 | NP_001004479.1 | 34 | 148 | c |
| 23 | OR4Q3 | NP_751944.1 | 17 | 119 | a | 53 | OR13C8 | NP_ 001004483.1 | 35 | 148 | c |
| 24 | OR4Q3 | NP_751944.1 | 17 | 120 | b | 54 | OR13G1 | NP_001005487.1 | 36 | 150 | c |
| 25 | OR4Q3 | NP_751944.1 | 17 | 121 | c | 55 | OR52A4 | AAI40753.1 | 37 | 151 | c |
| 26 | OR4S2 | NP_001004059.2 | 18 | 122 | a | 56 | OR56A4 | NP_001005179.3 | 38 | 152 | c |
| 27 | OR4S2 | NP_001004059.2 | 18 | 123 | b | | | | | | |
| 28 | OR4S2 | NP_001004059.2 | 18 | 124 | c | | | | | | |
| 29 | OR5A2 | NP_001001954.1 | 19 | 125 | c | | | | | | |
| 30 | OR5C1 | NP_001001923.1 | 20 | 126 | c | | | | | | |

**[Table 25-2]**

| No. | Olfactory receptor | Accession No. | SEQ ID NO of original amino acid sequence | SEQ ID NO of consensus amino acid sequence | Consensus design method | No. | Olfactory receptor | Accession No. | SEQ ID NO of original amino acid sequence | SEQ ID NO of consensus amino acid sequence | Consensus design method |
|---|---|---|---|---|---|---|---|---|---|---|---|
| 57 | OR2B11 | NP_001004492.1 | 39 | 153 | c | 87 | OR4K17 | NP_001004715.3 | 69 | 183 | c |
| 58 | OR2C3 | NP_932340.4 | 40 | 154 | c | 88 | OR4L1 | NP_001004717.1 | 70 | 184 | c |
| 59 | OR2G2 | NP_001001915.1 | 41 | 155 | c | 89 | OR4M1 | NP_001005500.1 | 71 | 185 | c |
| 60 | OR2G3 | NP_001001914.1 | 42 | 156 | c | 90 | OR4N2 | NP_001004723.1 | 72 | 186 | c |
| 61 | OR2L2 | NP_001004686.1 | 43 | 157 | c | 91 | OR4N5 | NP_01004724.1 | 73 | 187 | c |
| 62 | OR2L3 | NP_001004687.1 | 44 | 158 | c | 92 | OR5A1 | NP_001004728 | 74 | 188 | c |
| 63 | OR2L5 | NP_001245213.1 | 45 | 159 | c | 93 | OR6F1 | NP_001005286.1 | 75 | 189 | c |
| 64 | OR2L8 | NP_001001963.1 | 46 | 160 | c | 94 | OR6J1 | NP_001335162.1 | 76 | 190 | c |
| 65 | OR2L13 | NP_001291464.1 | 47 | 161 | c | 95 | OR7A10 | NP_001005190.1 | 77 | 191 | c |
| 66 | OR2M2 | NP_001004688.1 | 48 | 162 | c | 96 | OR7C2 | NP_036509.1 | 78 | 192 | c |
| 67 | OR2M4 | NP_059974.1 | 49 | 163 | c | 97 | OR7G2 | NP_001005193.2 | 79 | 193 | c |
| 68 | OR2T1 | NP_112166.2 | 50 | 164 | c | 98 | OR7G3 | NP_001001958.1 | 80 | 194 | c |
| 69 | OR2T4 | NP_001004696.2 | 51 | 165 | c | 99 | OR10G2 | NP_001005466.2 | 81 | 195 | c |
| 70 | OR2T5 | NP_001004697.1 | 52 | 166 | c | 100 | OR10J1 | NP_036483.3 | 82 | 196 | c |
| 71 | OR2T6 | NP_001005471.1 | 53 | 167 | c | 101 | OR10J3 | NP_01004467.1 | 83 | 197 | c |
| 72 | OR2T7 | NP_001372981.1 | 54 | 168 | d | 102 | OR10K1 | NP_001004473.1 | 84 | 198 | c |
| 73 | OR2T8 | NP_001005522.1 | 55 | 169 | c | 103 | OR10K2 | NP_001004476.1 | 85 | 199 | c |
| 74 | OR2T12 | NP_001004692.1 | 56 | 170 | c | 104 | OR10T2 | NP_001004475.1 | 86 | 200 | c |
| 75 | OR2T27 | NP_001001824.1 | 57 | 171 | c | 105 | OR10X1 | NP_001004477.1 | 87 | 201 | c |
| 76 | OR2T29 | NP_001004694.2 | 58 | 172 | c | 106 | OR11G2 | NP_001372962.1 | 88 | 202 | c |
| 77 | OR2T33 | NP_001004695.1 | 59 | 173 | c | 107 | OR11H6 | NP_001004480.1 | 89 | 203 | c |
| 78 | OR2T34 | NP_001001821.1 | 60 | 174 | c | 108 | OR11H7 | NP_001335202.1 | 90 | 204 | c |
| 79 | OR2T35 | NP_001001827.1 | 61 | 175 | c | 109 | OR11L1 | NP_001001959.1 | 91 | 205 | c |
| 80 | OR2AK2 | NP_001004491.1 | 62 | 176 | c | 110 | OR14A2 | NP_001342221.1 | 92 | 206 | c |
| 81 | OR4D10 | NP_001004705.1 | 63 | 177 | c | 111 | OR14A16 | NP_001001966.1 | 93 | 207 | c |
| 82 | OR4E2 | NP_001001912.2 | 64 | 178 | c | 112 | OR14C36 | NP_001001918.1 | 94 | 208 | c |
| 83 | OR4F5 | NP_001005484.2 | 65 | 179 | d | 113 | OR14K1 | NP_001004732.2 | 95 | 209 | c |
| 84 | OR4K1 | XP_011535455.1 | 66 | 180 | c | 114 | OR7E24 | NP_001373037.1 | 96 | 210 | c |
| 85 | OR4K2 | NP_001005501.1 | 67 | 181 | c | | | | | | |
| 86 | OR4K5 | NP_001005483.1 | 68 | 182 | c | | | | | | |

**[Table 25-3]**

| No. | Olfactory receptor | Accession No. | SEQ ID NO of original amino acid sequence | SEQ ID NO of consensus amino acid sequence | Consensus design method | No. | Olfactory receptor | Accession No. | SEQ ID NO of original amino acid sequence | SEQ ID NO of consensus amino acid sequence | Consensus design method |
|---|---|---|---|---|---|---|---|---|---|---|---|
| 115 | OR1B1 | NP_001004450.2 | 327 | 485 | c | 155 | OR3A3 | NP_036505.3 | 367 | 525 | d |
| 116 | OR1D2 | NP_002539.2 | 328 | 486 | c | 156 | OR4A5 | NP_001005272.3 | 368 | 526 | c |
| 117 | OR1D4 | NP_003543.3 | 329 | 487 | d | 157 | OR4A15 | NP_001005275.1 | 369 | 527 | c |
| 118 | OR1E2 | NP_003545.1 | 330 | 488 | c | 158 | OR4A47 | NP_001005512.2 | 370 | 528 | c |
| 119 | OR1F1 | NP_036492.1 | 331 | 489 | c | 159 | OR4B1 | NP_001005470.1 | 371 | 529 | c |
| 120 | OR1G1 | NP_003546.1 | 332 | 490 | c | 160 | OR4C3 | NP_001004702.2 | 372 | 530 | c |
| 121 | OR1I1 | NP_001004713.1 | 333 | 491 | c | 161 | OR4C6 | NP_001004704.1 | 373 | 531 | c |
| 122 | OR1J1 | NP_001004451.1 | 334 | 492 | c | 162 | OR4C11 | NP_001004700.2 | 374 | 532 | c |
| 123 | OR1J4 | NP_001004452.1 | 335 | 493 | c | 163 | OR4C12 | NP_001005270.3 | 375 | 533 | c |
| 124 | OR1K1 | NP_543135.1 | 336 | 494 | c | 164 | OR4C16 | NP_001004701.2 | 376 | 534 | c |
| 125 | OR1L1 | NP_001005236.3 | 337 | 495 | c | 165 | OR4C46 | NP_001004703.1 | 377 | 535 | c |
| 126 | OR1L3 | NP_001005234.1 | 338 | 496 | c | 166 | OR4D1 | NP_036506.1 | 378 | 536 | c |
| 127 | OR1L4 | NP_001005235.1 | 339 | 497 | c | 167 | OR4D2 | NP_001004707.1 | 379 | 537 | c |
| 128 | OR1L6 | NP_001004453.2 | 340 | 498 | c | 168 | OR4D9 | NP_001004711.1 | 380 | 538 | c |
| 129 | OR1L8 | NP_001004454.1 | 341 | 499 | c | 169 | OR4D11 | NP_001004706.1 | 381 | 539 | c |
| 130 | OR1M1 | NP_001004456.1 | 342 | 500 | c | 170 | OR4F17 | NP_001005240.1 | 382 | 540 | c |
| 131 | OR1Q1 | NP_036496.1 | 343 | 501 | c | 171 | OR4F21 | NP_001005504.1 | 383 | 541 | c |
| 132 | OR1S1 | NP_001004458.1 | 344 | 502 | c | 172 | OR4P4 | NP_001004124.1 | 384 | 542 | c |
| 133 | OR1S2 | NP_001004459.1 | 345 | 503 | c | 173 | OR4X1 | NP_001004726.1 | 385 | 543 | c |
| 134 | OR2A1 | NP_001005287.1 | 346 | 504 | c | 174 | OR4X2 | NP_001004727.1 | 386 | 544 | c |
| 135 | OR2A5 | NP_036497.1 | 347 | 505 | c | 175 | OR5B2 | NP_001005566.1 | 387 | 545 | c |
| 136 | OR2A7 | NP_001005328.1 | 348 | 506 | c | 176 | OR5B12 | NP_001004733.1 | 388 | 546 | c |
| 137 | OR2A14 | NP_001001659.1 | 349 | 507 | c | 177 | OR5B17 | NP_001005489.1 | 389 | 547 | c |
| 138 | OR2B2 | NP_149046.2 | 350 | 508 | c | 178 | OR5H1 | NP_001005338.1 | 390 | 548 | c |
| 139 | OR2B3 | NP_001005226.1 | 351 | 509 | c | 179 | OR5H2 | NP_001005482.2 | 391 | 549 | c |
| 140 | OR2B6 | NP_036499.1 | 352 | 510 | c | 180 | OR5H6 | NP_001005479.2 | 392 | 550 | c |
| 141 | OR2C1 | NP_036500.2 | 353 | 511 | c | 181 | OR5H14 | NP_001005514.1 | 393 | 551 | c |
| 142 | OR2F1 | NP_036501.2 | 354 | 512 | c | 182 | OR5K3 | NP_001005516.1 | 394 | 552 | c |
| 143 | OR2F2 | NP_001004685.1 | 355 | 513 | c | 183 | OR5AK2 | NP_001005323.1 | 395 | 553 | c |
| 144 | OR2H1 | NP_001304943.1 | 356 | 514 | c | 184 | OR5AR1 | NP_001004730.1 | 396 | 554 | c |
| 145 | OR2H2 | NP_009091.3 | 357 | 515 | c | 185 | OR5AS1 | NP_001001921.1 | 397 | 555 | c |
| 146 | OR2K2 | NP_995581.1 | 358 | 516 | c | 186 | OR6B1 | NP_001005281.1 | 398 | 556 | c |
| 147 | OR2S2 | NP_063950.2 | 359 | 517 | c | 187 | OR6B3 | NP_775486.1 | 399 | 557 | c |
| 148 | OR2V1 | NP_001245212.1 | 360 | 518 | c | 188 | OR6C2 | NP_473446.1 | 400 | 558 | c |
| 149 | OR2V2 | NP_996763.1 | 361 | 519 | c | 189 | OR6C3 | NP_473445.1 | 401 | 559 | c |
| 150 | OR2Z1 | NP_001004699.1 | 362 | 520 | c | 190 | OR6C4 | NP_001372904.1 | 402 | 560 | c |
| 151 | OR2AE1 | NP_001005276.1 | 363 | 521 | c | 191 | OR6C6 | NP_001005493.1 | 403 | 561 | c |
| 152 | OR2AP1 | NP_001245214.1 | 364 | 522 | c | 192 | OR6C65 | NP_001005518.1 | 404 | 562 | c |
| 153 | OR2AT4 | NP_001005285.1 | 365 | 523 | c | 193 | OR6C68 | NP_001005519.2 | 405 | 563 | c |
| 154 | OR3A1 | NP_002541.2 | 366 | 524 | c | 194 | OR6C70 | NP_001005499.1 | 406 | 564 | c |

**[Table 25-4]**

| No. | Olfactory receptor | Accession No. | SEQ ID NO of original amino acid sequence | SEQ ID NO of consensus amino acid sequence | Consensus design method | No. | Olfactory receptor | Accession No. | SEQ ID NO of original amino acid sequence | SEQ ID NO of consensus amino acid sequence | Consensus design method |
|---|---|---|---|---|---|---|---|---|---|---|---|
| 195 | OR6C74 | NP_001005490.1 | 407 | 565 | c | 234 | OR51B5 | NP_001005567.2 | 446 | 604 | c |
| 196 | OR6C75 | NP_001005497.1 | 408 | 566 | c | 235 | OR51B6 | NP_001004750.1 | 447 | 605 | c |
| 197 | OR6C76 | NP_001005183.1 | 409 | 567 | c | 236 | OR51D1 | NP_001004751.1 | 448 | 606 | c |
| 198 | OR6K3 | NP_001005327.2 | 410 | 568 | c | 237 | OR51E2 | NP_110401.1 | 449 | 607 | c |
| 199 | OR6K6 | NP_001005184.1 | 411 | 569 | c | 238 | OR51F1 | NP_001004752.2 | 450 | 608 | c |
| 200 | OR6N2 | NP_001005278.1 | 412 | 570 | c | 239 | OR51F2 | NP_001004753.2 | 451 | 609 | c |
| 201 | OR8S1 | NP_001377778.1 | 413 | 571 | c | 240 | OR51G1 | NP_001005237.1 | 452 | 610 | c |
| 202 | OR9A2 | NP_001001658.1 | 414 | 572 | c | 241 | OR51G2 | NP_001005238.1 | 453 | 611 | c |
| 203 | OR9A4 | NP_001001656.1 | 415 | 573 | c | 242 | OR51I1 | NP_001005288.1 | 454 | 612 | c |
| 204 | OR10A3 | NP_001003745.1 | 416 | 574 | c | 243 | OR51I2 | NP_001004754.1 | 455 | 613 | c |
| 205 | OR10A7 | NP_001005280.1 | 417 | 575 | c | 244 | OR51J1 | NP_001335153.1 | 456 | 614 | c |
| 206 | OR10G6 | NP_001342148.1 | 418 | 576 | c | 245 | OR51L1 | NP_001004755.1 | 457 | 615 | c |
| 207 | OR10G9 | NP_001001953.1 | 419 | 577 | c | 246 | OR51M1 | NP_001004756.2 | 458 | 616 | c |
| 208 | QR10H1 | NP_039228.1 | 420 | 578 | c | 247 | OR51Q1 | NP_001004757.1 | 459 | 617 | c |
| 209 | OR10H2 | NP_039227.1 | 421 | 579 | c | 248 | OR51S1 | NP_001004758.1 | 460 | 618 | c |
| 210 | OR10H3 | NP_039226.1 | 422 | 580 | c | 249 | OR51T1 | NP_001004759.2 | 461 | 619 | c |
| 211 | OR10H4 | NP_001004465.1 | 423 | 581 | c | 250 | OR51V1 | NP_001004760.3 | 462 | 620 | c |
| 212 | OR10H5 | NP_001004466.1 | 424 | 582 | c | 251 | OR52A1 | NP_036507.2 | 463 | 621 | c |
| 213 | OR10P1 | NP_996782.1 | 425 | 583 | c | 252 | OR52A5 | NP_001005160.1 | 464 | 622 | c |
| 214 | OR10S1 | NP_001004474.1 | 426 | 584 | c | 253 | OR52B2 | NP_001004052.1 | 465 | 623 | c |
| 215 | OR10V1 | NP_001005324.1 | 427 | 585 | c | 254 | OR52B4 | NP_301005161.2 | 466 | 624 | c |
| 216 | OR10W1 | NP_997257.2 | 428 | 586 | c | 255 | OR52B6 | NP_001005162.2 | 467 | 625 | c |
| 217 | OR10AD1 | NP_001004134.1 | 429 | 587 | c | 256 | OR52D1 | NP_001005163.1 | 468 | 626 | c |
| 218 | OR10AG1 | NP_001005491.2 | 430 | 588 | c | 257 | OR52E5 | NP_001005166.3 | 469 | 627 | c |
| 219 | OR11A1 | NP_039225.1 | 431 | 589 | c | 258 | OR52E6 | NP_001005167.1 | 470 | 628 | d |
| 220 | OR11H2 | NP_001184216.2 | 432 | 590 | d | 259 | OR52H1 | NP_001005289.2 | 471 | 629 | c |
| 221 | OR12D2 | NP_039224.2 | 433 | 591 | c | 260 | OR52I1 | NP_001005169.1 | 472 | 630 | c |
| 222 | OR12D3 | NP_112221.1 | 434 | 592 | c | 261 | OR52I2 | NP_001005170.1 | 473 | 631 | c |
| 223 | OR13A1 | NP_001004297.2 | 435 | 593 | c | 262 | OR52J3 | NP_001001916.2 | 474 | 632 | c |
| 224 | OR13C2 | NP_001004481.1 | 436 | 594 | c | 263 | OR52K1 | NP_001005171.2 | 475 | 633 | c |
| 225 | OR13C3 | NP_001001961.2 | 437 | 595 | c | 264 | OR52K2 | NP_001005172.2 | 476 | 634 | c |
| 226 | OR13C4 | NP_001001919.1 | 438 | 596 | c | 265 | OR52N1 | NP_001001913.1 | 477 | 635 | c |
| 227 | OR13C9 | NP_001001956.1 | 439 | 597 | c | 266 | OR52R1 | NP_001005177.3 | 478 | 636 | c |
| 228 | OR13F1 | NP_001004485.1 | 440 | 598 | c | 267 | OR52W1 | NP_001005178.1 | 479 | 637 | c |
| 229 | OR51A2 | NP_001004748.1 | 441 | 599 | d | 268 | OR56A5 | NP_001139505.1 | 480 | 638 | c |
| 230 | OR51A4 | NP_001005329.1 | 442 | 600 | c | 269 | OR56B4 | NP_001005181.1 | 481 | 639 | c |
| 231 | OR51A7 | NP_001004749.1 | 443 | 601 | c | 270 | OR5AN1 | NP_001004729.1 | 482 | 640 | c |
| 232 | OR51B2 | NP_1494204 | 444 | 602 | c | 271 | M71 | NP_997547.1 | 483 | 641 | c |
| 233 | OR51B4 | NP_149419.2 | 445 | 603 | c | 272 | OR9K2 | NP_001005243.2 | 484 | 642 | c |

**[Table 25-5]**

| No. | Olfactory receptor | Accession No. | SEQ ID NO of original amino acid sequence | SEQ ID NO of consensus amino acid sequence | Consensus design method | No. | Olfactory receptor | Accession No. | SEQ ID NO of original amino acid sequence | SEQ ID NO of consensus amino acid sequence | Consensus design method |
|---|---|---|---|---|---|---|---|---|---|---|---|
| 273 | OR2A2 | NP_001005480.2 | 801 | 851 | c | 298 | OR6P1 | NP_001153797.1 | 826 | 876 | c |
| 274 | OR2A12 | NP_001004135.1 | 802 | 852 | c | 299 | OR6Q1 | NP_001005186.2 | 827 | 877 | c |
| 275 | OR4S1 | NP_001004725.1 | 803 | 853 | c | 300 | OR6T1 | NP_001005187.1 | 828 | 878 | c |
| 276 | OR5AC2 | NP_473447.1 | 804 | 854 | c | 301 | OR6X1 | NP_001005188.1 | 829 | 879 | c |
| 277 | OR5B3 | NP_001005469.1 | 805 | 855 | c | 302 | OR8B4 | NP_001005196.1 | 830 | 880 | c |
| 278 | OR5D13 | NP_001001967.1 | 806 | 856 | c | 303 | OR8B8 | NP_036510.1 | 831 | 881 | c |
| 279 | OR5D14 | NP_001004735.1 | 807 | 857 | c | 304 | OR8B12 | NP_001005195.1 | 832 | 882 | c |
| 280 | OR5D16 | NP_001005496.1 | 808 | 858 | c | 305 | OR8D1 | NP_001002917.1 | 833 | 883 | c |
| 281 | OR5D18 | NP_001001952.1 | 809 | 859 | c | 306 | OR8G1 | NP_001002905.1 | 834 | 884 | c |
| 282 | OR5J2 | NP_001005492.1 | 810 | 860 | c | 307 | OR8G5 | NP_001005198.2 | 835 | 885 | c |
| 283 | OR5K4 | NP_001005517.1 | 811 | 861 | c | 308 | OR8H2 | NP_001372993.1 | 836 | 886 | d |
| 284 | OR5L1 | NP_001004738.1 | 812 | 862 | c | 309 | OR8I2 | NP_001003750.1 | 837 | 887 | c |
| 285 | OR5L2 | NP_001004739.1 | 813 | 863 | c | 310 | OR8J1 | NP_001005205.2 | 838 | 888 | c |
| 286 | OR5M1 | NP_001004740.1 | 814 | 864 | c | 311 | OR8K1 | NP_001002907.1 | 839 | 889 | c |
| 287 | OR5M3 | NP_001004742.2 | 815 | 865 | c | 312 | OR8K3 | NP_001005202.1 | 840 | 890 | c |
| 288 | OR5M8 | NP_001005282.1 | 816 | 866 | c | 313 | OR8K5 | NP_001004058.2 | 841 | 891 | c |
| 289 | OR5M9 | NP_001004743.1 | 817 | 867 | c | 314 | OR8U1 | NP_001005204.1 | 842 | 892 | c |
| 290 | OR5M10 | NP_001004741.1 | 818 | 868 | c | 315 | OR9G1 | NP_001005213.1 | 843 | 893 | c |
| 291 | OR5M11 | NP_001005245.1 | 819 | 869 | c | 316 | OR9G4 | NP_001377761.1 | 844 | 894 | c |
| 292 | OR5P2 | NP_703145.1 | 820 | 870 | c | 317 | OR9I1 | NP_001005211.1 | 845 | 895 | c |
| 293 | OR5T1 | NP_001004745.1 | 821 | 871 | c | 318 | OR11H12 | NP_001013372.1 | 846 | 896 | c |
| 294 | OR5T2 | NP_001004746.2 | 822 | 872 | c | 319 | OR52M1 | NP_001004137.1 | 847 | 897 | c |
| 295 | OR5T3 | NP_001004747.2 | 823 | 873 | c | 320 | OR52N5 | NP_001372591.1 | 848 | 898 | c |
| 296 | OR6M1 | NP_001005325.1 | 824 | 874 | c | 321 | OR56A3 | NP_001003443.2 | 849 | 899 | c |
| 297 | OR6N1 | NP_001005185.1 | 825 | 875 | c | 322 | OR5I1 | NP_006628.1 | 850 | 900 | c |

Thus, the present consensus design method enables functional analysis by actually increasing the cell membrane expression level of an olfactory receptor thereby improving the responsiveness of the olfactory receptor, or by improving the responsiveness of the olfactory receptor even if its cell membrane expression level is not increased. Also, the finding obtained by this method properly explains, for example, difference in sense of smell among individuals obtained in the preceding finding. Thus, the present method is useful, as mentioned in the beginning, for assessing the properties of a human sense of smell or providing a method for searching for a substance which can control a sense of smell.

## Claims

1. A method for expressing an olfactory receptor polypeptide, comprising
expressing, in a cell, an olfactory receptor polypeptide consisting of an amino acid sequence obtained by, in an amino acid sequence of an olfactory receptor of interest, altering at least one amino acid residue different from that in a consensus amino acid sequence to the amino acid residue at a position corresponding thereto in the consensus amino acid sequence, wherein
the consensus amino acid sequence is an amino acid sequence derived by alignment of the amino acid sequence of the olfactory receptor of interest and amino acid sequences of any of the following olfactory receptors (a) to (d):
(a) at least 11 olfactory receptors selected from the group consisting of olfactory receptors encoded by orthologs of the olfactory receptor of interest in the same order as that of an organism species from which the olfactory receptor of interest is derived;
(b) at least 11 olfactory receptors selected from the group consisting of the olfactory receptors encoded by orthologs of the olfactory receptor of interest in the same order as that of an organism species from which the olfactory receptor of interest is derived and olfactory receptors encoded by paralogs of the olfactory receptor of interest, the 11 olfactory receptors including at least one of the olfactory receptor encoded by the paralog of the olfactory receptor of interest;
(c) at least 11 olfactory receptors selected from the group consisting of olfactory receptors encoded by orthologs of the olfactory receptor of interest in vertebrates, the 11 olfactory receptors including at least one olfactory receptor encoded by a vertebrate ortholog in an order different from the order of an organism species from which the olfactory receptor of interest is derived; and
(d) at least 11 olfactory receptors selected from the group consisting of olfactory receptors encoded by orthologs of the olfactory receptor of interest in vertebrates and olfactory receptors encoded by orthologs in a vertebrate with a paralog having the highest homology to the olfactory receptor of interest among paralogs of the olfactory receptor of interest having 11 or more vertebrate orthologs, the 11 olfactory receptors including at least one olfactory receptor encoded by a vertebrate ortholog in an order different from the order of an organism species from which the olfactory receptor of interest is derived, and
an olfactory receptor encoded by the paralog.

2. The method according to claim 1, wherein the consensus amino acid sequence is an amino acid sequence consisting of consensus residues identified in accordance with the following criteria (i) to (iii) from the alignment of the amino acid sequence of the olfactory receptor of interest and the amino acid sequences of any of the olfactory receptors (a) to (d):
(i) at each amino acid position of the alignment,
(i-i) when there exists one amino acid residue which is different from the amino acid residue of the olfactory receptor of interest and has a frequency of appearance of 50% or more, the amino acid residue is identified as a consensus residue,
(i-ii) when there exist two amino acid residues having a frequency of appearance of 50%, the amino acid residues of the olfactory receptor of interest are identified as a consensus residue,
(i-iii) when there exists an amino acid residue in the olfactory receptor of interest and there exists no amino acid residue having a frequency of appearance of 40% or more, the absence of a consensus residue is identified,
(i-iv) when there exists no amino acid residue in the olfactory receptor of interest and there exists an amino acid residue having a frequency of appearance of 60% or more, an amino acid residue having the highest frequency of appearance is identified as a consensus residue, and when there exist two or more amino acid residues having the highest frequency of appearance, an amino acid residue having the smallest molecular weight among the amino acid residues is identified as a consensus residue, and
(i-v) if none of the above (i-i) to (i-iv) is appropriate, the amino acid residue of the olfactory receptor of interest is identified as a consensus residue;
(ii) when the consensus residues are identified in accordance with the criterion (i) and when a consensus residue nearest to the N terminus is a consensus residue at a position corresponding to the N terminus of the olfactory receptor of interest or a C-terminal side thereof and is not a methionine residue, a consensus residue on an N-terminal side of a consensus residue consisting of a methionine residue positioned nearest to the N terminus is changed to the absence of a consensus residue; and
(iii) when the consensus residues are identified in accordance with the criterion (i) and when a consensus residue nearest to the N terminus is a consensus residue at a position corresponding to an N-terminal side of the N terminus of the olfactory receptor of interest and is not a methionine residue, an amino acid residue having the highest frequency of appearance is identified as a consensus residue until a methionine residue appears by tracing back one by one amino acid positions on an N-terminal side of the position of the consensus residue of the alignment, and when there exist two or more amino acid residues having the highest frequency of appearance, an amino acid residue having the smallest molecular weight among the amino acid residues is identified as a consensus residue.

3. The method according to claim 1 or 2, wherein the alignment is alignment of the amino acid sequence of the olfactory receptor of interest and the olfactory receptors (c).

4. The method according to any one of claims 1 to 3, wherein the olfactory receptor of interest is a human olfactory receptor.

5. The method according to claim 1 or 2, wherein the olfactory receptor polypeptide consists of an amino acid sequence obtained by, in an amino acid sequence of sequence identification number (2) of an olfactory receptor (1) in the following Tables 1-1 to 1-5 altering at least one amino acid residue different from that in a consensus amino acid sequence of sequence identification number (3) to the amino acid residue at a position corresponding thereto in the consensus amino acid sequence:
**[Table 1-1]**
| No. | (1) Olfactory receptor | (2) SEQ ID NO of amino acid sequence | (3) SEQ ID NO of consensus amino acid sequence | No. | (1) Olfactory receptor | (2) SEQ ID NO of amino acid sequence | (3) SEQ ID NO of consensus amino acid sequence |
|---|---|---|---|---|---|---|---|
| 1 | OR2A25 | 1 | 97 | 31 | OR5W2 | 21 | 127 |
| 2 | OR2A25 | 1 | 98 | 32 | OR6A2 | 22 | 128 |
| 3 | OR2A25 | 1 | 99 | 33 | OR6B2 | 23 | 129 |
| 4 | OR2AG1 | 2 | 100 | 34 | OR6C1 | 24 | 130 |
| 5 | OR2AG2 | 3 | 101 | 35 | OR6Y1 | 25 | 131 |
| 6 | OR2D2 | 4 | 102 | 36 | OR6Y1 | 25 | 132 |
| 7 | OR2D3 | 5 | 103 | 37 | OR6Y1 | 25 | 133 |
| 8 | OR2T11 | 6 | 104 | 38 | OR7A17 | 26 | 134 |
| 9 | OR2T11 | 6 | 105 | 39 | OR7A17 | 26 | 135 |
| 10 | OR2T11 | 6 | 106 | 40 | OR7A17 | 26 | 136 |
| 11 | OR2W1 | 7 | 107 | 41 | OR7D4 | 27 | 137 |
| 12 | OR2W1 | 7 | 108 | 42 | OR7D4 | 27 | 138 |
| 13 | OR2W1 | 7 | 109 | 43 | OR7D4 | 27 | 139 |
| 14 | OR2W3 | 8 | 110 | 44 | OR10A2 | 28 | 140 |
| 15 | OR2Y1 | 9 | 111 | 45 | OR10A4 | 29 | 141 |
| 16 | OR3A2 | 10 | 112 | 46 | OR10D3 | 30 | 142 |
| 17 | OR4A16 | 11 | 113 | 47 | OR10Q1 | 31 | 143 |
| 18 | OR4C15 | 12 | 114 | 48 | OR10Z1 | 32 | 144 |
| 19 | OR4D5 | 13 | 115 | 49 | OR11H1 | 33 | 145 |
| 20 | OR4D6 | 14 | 116 | 50 | OR11H4 | 34 | 146 |
| 21 | OR4F15 | 15 | 117 | 51 | OR11H4 | 34 | 147 |
| 22 | OR4K15 | 16 | 118 | 52 | OR11H4 | 34 | 148 |
| 23 | OR4Q3 | 17 | 119 | 53 | OR13C8 | 35 | 149 |
| 24 | OR4Q3 | 17 | 120 | 54 | OR13G1 | 36 | 150 |
| 25 | OR4G3 | 17 | 121 | 55 | OR52A4 | 37 | 151 |
| 26 | OR4S2 | 18 | 122 | 56 | OR56A4 | 38 | 152 |
| 27 | OR4S2 | 18 | 123 | | | | |
| 28 | OR4S2 | 18 | 124 | | | | |
| 29 | OR5A2 | 19 | 125 | | | | |
| 30 | OR5C1 | 20 | 126 | | | | |
**[Table 1-2]**
| No. | (1) Olfactory receptor | (2) SEQ ID NO of amino acid sequence | (3) SEQ ID NO amino acid sequence | No. | (1) Olfactory receptor | (2) SEQ ID NO of amino acid sequence | (3) SEQ ID NO of consensus amino acid sequence |
|---|---|---|---|---|---|---|---|
| 57 | OR2B11 | 39 | 153 | 87 | OR4K17 | 69 | 183 |
| 58 | OR2C3 | 40 | 154 | 88 | OR4L1 | 70 | 184 |
| 59 | OR2G2 | 41 | 155 | 89 | OR4M1 | 71 | 185 |
| 60 | OR2G3 | 42 | 156 | 90 | OR4N2 | 72 | 186 |
| 61 | OR2L2 | 43 | 157 | 91 | OR4N5 | 73 | 187 |
| 62 | OR2L3 | 44 | 158 | 92 | OR5A1 | 74 | 188 |
| 63 | OR2L5 | 45 | 159 | 93 | OR6F1 | 75 | 189 |
| 64 | OR2L8 | 46 | 160 | 94 | OR6J1 | 76 | 190 |
| 65 | OR2L13 | 47 | 161 | 95 | OR7A10 | 77 | 191 |
| 66 | OR2M2 | 48 | 162 | 96 | OR7C2 | 78 | 192 |
| 67 | OR2M4 | 49 | 163 | 97 | OR7G2 | 79 | 193 |
| 68 | OR2T1 | 50 | 164 | 98 | OR7G3 | 80 | 194 |
| 69 | OR2T4 | 51 | 165 | 99 | OR10G2 | 81 | 195 |
| 70 | OR2T5 | 52 | 166 | 100 | OR10J1 | 82 | 196 |
| 71 | OR2T6 | 53 | 167 | 101 | OR10J3 | 83 | 197 |
| 72 | OR2T7 | 54 | 168 | 102 | OR10K1 | 84 | 198 |
| 73 | OR2T8 | 55 | 169 | 103 | OR10K2 | 85 | 199 |
| 74 | OR2T12 | 56 | 170 | 104 | OR10T2 | 86 | 200 |
| 75 | OR2T27 | 57 | 171 | 105 | OR10X1 | 87 | 201 |
| 76 | OR2T29 | 58 | 172 | 106 | OR11G2 | 88 | 202 |
| 77 | OR2T33 | 59 | 173 | 107 | OR11H6 | 89 | 203 |
| 78 | OR2T34 | 60 | 174 | 108 | OR11H7 | 90 | 204 |
| 79 | OR2T35 | 61 | 175 | 109 | OR11L1 | 91 | 205 |
| 80 | OR2AK2 | 62 | 176 | 110 | OR14A2 | 92 | 206 |
| 81 | OR4D10 | 63 | 177 | 111 | OR14A16 | 93 | 207 |
| 82 | OR4E2 | 64 | 178 | 112 | OR14C36 | 94 | 208 |
| 83 | OR4F5 | 65 | 179 | 113 | OR14K1 | 95 | 209 |
| 84 | OR4K1 | 66 | 180 | | | | |
| 85 | OR4K2 | 67 | 181 | | | | |
| 86 | OR4K5 | 68 | 182 | | | | |
**[Table 1-3]**
| No. | (1) Olfactory receptor | (2) SEQ ID NO of amino acid sequence | (3) SEQ ID NO of consensus amino acid sequence | No. | (1) Olfactory receptor | (2) SEQ ID NO of amino acid sequence | (3) SEQ ID NO of consensus amino acid sequence |
|---|---|---|---|---|---|---|---|
| 115 | OR1B1 | 327 | 485 | 155 | OR3A3 | 367 | 525 |
| 116 | OR1D2 | 328 | 486 | 156 | OR4A5 | 368 | 526 |
| 117 | OR1D4 | 329 | 487 | 157 | OR4A15 | 369 | 527 |
| 118 | OR1E2 | 330 | 488 | 158 | OR4A47 | 370 | 528 |
| 119 | OR1F1 | 331 | 489 | 159 | OR4B1 | 371 | 529 |
| 120 | OR1G1 | 332 | 490 | 160 | OR4C3 | 372 | 530 |
| 121 | OR1I1 | 333 | 491 | 161 | OR4C6 | 373 | 531 |
| 122 | OR1J1 | 334 | 492 | 162 | OR4C11 | 374 | 532 |
| 123 | OR1J4 | 335 | 493 | 163 | OR4C12 | 375 | 533 |
| 124 | OR1K1 | 336 | 494 | 164 | OR4C16 | 376 | 534 |
| 125 | OR1L1 | 337 | 495 | 165 | OR4C46 | 377 | 535 |
| 126 | OR1L3 | 338 | 496 | 166 | OR4D1 | 378 | 536 |
| 127 | OR1L4 | 339 | 497 | 167 | OR4D2 | 379 | 537 |
| 128 | OR1L6 | 340 | 498 | 168 | OR4D9 | 380 | 538 |
| 129 | OR1L8 | 341 | 499 | 169 | OR4D11 | 381 | 539 |
| 130 | OR1M1 | 342 | 500 | 170 | OR4F17 | 382 | 540 |
| 131 | OR1Q1 | 343 | 501 | 171 | OR4F21 | 383 | 541 |
| 132 | OR1S1 | 344 | 502 | 172 | OR4P4 | 384 | 542 |
| 133 | OR1S2 | 345 | 503 | 173 | OR4X1 | 385 | 543 |
| 134 | OR2A1 | 346 | 504 | 174 | OR4X2 | 386 | 544 |
| 135 | OR2A5 | 347 | 505 | 175 | OR5B2 | 387 | 545 |
| 136 | OR2A7 | 348 | 506 | 176 | OR5B12 | 388 | 546 |
| 137 | OR2A14 | 349 | 507 | 177 | OR5B17 | 389 | 547 |
| 138 | OR2B2 | 350 | 508 | 178 | OR5H1 | 390 | 548 |
| 139 | OR2B3 | 351 | 509 | 179 | OR5H2 | 391 | 549 |
| 140 | OR2B6 | 352 | 510 | 180 | OR5H6 | 392 | 550 |
| 141 | OR2C1 | 353 | 511 | 181 | OR5H14 | 393 | 551 |
| 142 | OR2F1 | 354 | 512 | 182 | OR5K3 | 394 | 552 |
| 143 | OR2F2 | 355 | 513 | 183 | OR5AK2 | 395 | 553 |
| 144 | OR2H1 | 356 | 514 | 184 | OR5AR1 | 396 | 554 |
| 145 | OR2H2 | 357 | 515 | 185 | OR5AS1 | 397 | 555 |
| 146 | OR2K2 | 358 | 516 | 186 | OR6B1 | 398 | 556 |
| 147 | OR2S2 | 359 | 517 | 187 | OR6B3 | 399 | 557 |
| 148 | OR2V1 | 360 | 518 | 188 | OR6C2 | 400 | 558 |
| 149 | OR2V2 | 361 | 519 | 189 | OR6C3 | 401 | 559 |
| 150 | OR2Z1 | 362 | 520 | 190 | OR6C4 | 402 | 560 |
| 151 | OR2AE1 | 363 | 521 | 191 | OR6C6 | 403 | 561 |
| 152 | OR2AP1 | 364 | 522 | 192 | OR6C65 | 404 | 562 |
| 153 | OR2AT4 | 365 | 523 | 193 | OR6C68 | 405 | 563 |
| 154 | OR3A1 | 366 | 524 | 194 | OR6C70 | 406 | 564 |
**[Table 1-4]**
| No. | (1) Olfactory receptor | (2) SEQ ID NO of amino acid sequence | (3) SEQ ID NO of consensus amino acid sequence | No. | (1) Olfactory receptor | (2) SEQ ID NO of amino acid sequence | (3) SEQ ID NO of consensus amino acid sequence |
|---|---|---|---|---|---|---|---|
| 195 | OR6C74 | 407 | 565 | 234 | OR51B5 | 446 | 604 |
| 196 | OR6C75 | 408 | 566 | 235 | OR51B6 | 447 | 605 |
| 197 | OR6C76 | 409 | 567 | 236 | OR51D1 | 448 | 606 |
| 198 | OR6K3 | 410 | 568 | 237 | OR51E2 | 449 | 607 |
| 199 | OR6K6 | 411 | 569 | 238 | OR51F1 | 450 | 608 |
| 200 | OR6N2 | 412 | 570 | 239 | OR51F2 | 451 | 609 |
| 201 | OR8S1 | 413 | 571 | 240 | OR51G1 | 452 | 610 |
| 202 | OR9A2 | 414 | 572 | 241 | OR51G2 | 453 | 611 |
| 203 | OR9A4 | 415 | 573 | 242 | OR51I1 | 454 | 612 |
| 204 | OR10A3 | 416 | 574 | 243 | OR51I2 | 455 | 613 |
| 205 | OR10A7 | 417 | 575 | 244 | OR51J1 | 456 | 614 |
| 206 | OR10G6 | 418 | 576 | 245 | OR51L1 | 457 | 615 |
| 207 | OR10G9 | 419 | 577 | 246 | OR51M1 | 458 | 616 |
| 208 | OR10H1 | 420 | 578 | 247 | OR51Q1 | 459 | 617 |
| 209 | OR10H2 | 421 | 579 | 248 | OR51S1 | 460 | 618 |
| 210 | OR10H3 | 422 | 580 | 249 | OR51T1 | 461 | 619 |
| 211 | OR10H4 | 423 | 581 | 250 | OR51V1 | 462 | 620 |
| 212 | OR10H5 | 424 | 582 | 251 | OR52A1 | 463 | 621 |
| 213 | OR10P1 | 425 | 583 | 252 | OR52A5 | 464 | 622 |
| 214 | OR10S1 | 426 | 584 | 253 | OR52B2 | 465 | 623 |
| 215 | OR10V1 | 427 | 585 | 254 | OR52B4 | 466 | 624 |
| 216 | OR10W1 | 428 | 586 | 255 | OR52B6 | 467 | 625 |
| 217 | OR10AD1 | 429 | 587 | 256 | OR52D1 | 468 | 626 |
| 218 | OR10AG1 | 430 | 588 | 257 | OR52E5 | 469 | 627 |
| 219 | OR11A1 | 431 | 589 | 258 | OR52E6 | 470 | 628 |
| 220 | OR11H2 | 432 | 590 | 259 | OR52H1 | 471 | 629 |
| 221 | OR12D2 | 433 | 591 | 260 | OR52I1 | 472 | 630 |
| 222 | OR12D3 | 434 | 592 | 261 | OR52I2 | 473 | 631 |
| 223 | OR13A1 | 435 | 593 | 262 | OR52J3 | 474 | 632 |
| 224 | OR13C2 | 436 | 594 | 263 | OR52K1 | 475 | 633 |
| 225 | OR13C3 | 437 | 595 | 264 | OR52K2 | 476 | 634 |
| 226 | OR13C4 | 438 | 596 | 265 | OR52N1 | 477 | 635 |
| 227 | OR13C9 | 439 | 597 | 266 | OR52R1 | 478 | 636 |
| 228 | OR13F1 | 440 | 598 | 267 | OR52W1 | 479 | 637 |
| 229 | OR51A2 | 441 | 599 | 268 | OR56A5 | 480 | 638 |
| 230 | OR51A4 | 442 | 600 | 269 | OR56B4 | 481 | 639 |
| 231 | OR51A7 | 443 | 601 | 270 | OR5AN1 | 482 | 640 |
| 232 | OR51B2 | 444 | 602 | 271 | M71 | 483 | 641 |
| 233 | OR51B4 | 445 | 603 | | | | |
**[Table 1-5]**
| No. | (1) Olfactory receptor | (2) SEQ ID NO of amino acid sequence | (3) SEQ ID NO of consensus amino acid sequence | No. | (1) Olfactory receptor | (2) SEQ ID NO of amino acid sequence | (3) SEQ ID NO of consensus amino acid sequence |
|---|---|---|---|---|---|---|---|
| 273 | OR2A2 | 801 | 851 | 298 | OR6P1 | 826 | 876 |
| 274 | OR2A12 | 802 | 852 | 299 | OR6Q1 | 827 | 877 |
| 275 | OR4S1 | 803 | 853 | 300 | OR6T1 | 828 | 878 |
| 276 | OR5AC2 | 804 | 854 | 301 | OR6X1 | 829 | 879 |
| 277 | OR5B3 | 805 | 855 | 302 | OR8B4 | 830 | 880 |
| 278 | OR5D13 | 806 | 856 | 303 | OR8B8 | 831 | 881 |
| 279 | OR5D14 | 807 | 857 | 304 | OR8B12 | 832 | 882 |
| 280 | OR5D16 | 808 | 858 | 305 | OR8D1 | 833 | 883 |
| 281 | OR5D18 | 809 | 859 | 306 | OR8G1 | 834 | 884 |
| 282 | OR5J2 | 810 | 860 | 307 | OR8G5 | 835 | 885 |
| 283 | OR5K4 | 811 | 861 | 308 | OR8H2 | 836 | 886 |
| 284 | OR5L1 | 812 | 862 | 309 | OR8I2 | 837 | 887 |
| 285 | OR5L2 | 813 | 863 | 310 | OR8J1 | 838 | 888 |
| 286 | OR5M1 | 814 | 864 | 311 | OR8K1 | 839 | 889 |
| 287 | OR5M3 | 815 | 865 | 312 | OR8K3 | 840 | 890 |
| 288 | OR5M8 | 816 | 866 | 313 | OR8K5 | 841 | 891 |
| 289 | OR5M9 | 817 | 867 | 314 | OR8U1 | 842 | 892 |
| 290 | OR5M10 | 818 | 868 | 315 | OR9G1 | 843 | 893 |
| 291 | OR5M11 | 819 | 869 | 316 | OR9G4 | 844 | 894 |
| 292 | OR5P2 | 820 | 870 | 317 | OR9I1 | 845 | 895 |
| 293 | OR5T1 | 821 | 871 | 318 | OR11H12 | 846 | 896 |
| 294 | OR5T2 | 822 | 872 | 319 | OR52M1 | 847 | 897 |
| 295 | OR5T3 | 823 | 873 | 320 | OR52N5 | 848 | 898 |
| 296 | OR6M1 | 824 | 874 | 321 | OR56A3 | 849 | 899 |
| 297 | OR6N1 | 825 | 875 | | | | |

6. The method according to claim 5, wherein the olfactory receptor polypeptide consists of an amino acid sequence of any of SEQ ID NOs: 97 to 209, 485 to 641, and 851 to 899.

7. A method for expressing an olfactory receptor polypeptide, comprising
expressing, in a cell, an olfactory receptor polypeptide consisting of an amino acid sequence obtained by, in an amino acid sequence of an olfactory receptor of interest, altering at least one amino acid residue different from that in a consensus amino acid sequence to the amino acid residue at a position corresponding thereto in the consensus amino acid sequence, wherein
the olfactory receptor polypeptide consists of an amino acid sequence obtained by, in an amino acid sequence of SEQ ID NO: 96 of a human olfactory receptor OR7E24, altering at least one amino acid residue different from that in a consensus amino acid sequence of SEQ ID NO: 210 to the amino acid residue at a position corresponding thereto in the consensus amino acid sequence,
the olfactory receptor polypeptide consists of an amino acid sequence obtained by, in an amino acid sequence of SEQ ID NO: 484 of a human olfactory receptor OR9K2, altering at least one amino acid residue different from that in a consensus amino acid sequence of SEQ ID NO: 642 to the amino acid residue at a position corresponding thereto in the consensus amino acid sequence, or
the olfactory receptor polypeptide consists of an amino acid sequence obtained by, in an amino acid sequence of SEQ ID NO: 850 of a human olfactory receptor OR5I1, altering at least one amino acid residue different from that in a consensus amino acid sequence of SEQ ID NO: 900 to the amino acid residue at a position corresponding thereto in the consensus amino acid sequence.

8. The method according to claim 7, wherein the olfactory receptor polypeptide consists of an amino acid sequence of SEQ ID NO: 210, 642, or 900.

9. A method for measuring a response of an olfactory receptor of interest, comprising
measuring a response of an olfactory receptor polypeptide expressed by the method according to any one of claims 1 to 8.

10. A method for searching for a ligand for an olfactory receptor of interest, comprising:
measuring a response of an olfactory receptor polypeptide expressed by the method according to any one of claims 1 to 8 in the presence of a test substance; and
selecting a test substance to which the olfactory receptor polypeptide has responded.

11. A method for evaluating and/or selecting a suppressor of odor of a ligand for an olfactory receptor of interest, comprising:
adding a test substance and the ligand for an olfactory receptor of interest to an olfactory receptor polypeptide expressed by the method according to any one of claims 1 to 8; and
measuring a response of the olfactory receptor polypeptide to the ligand.

12. A method for evaluating and/or selecting a suppressor of odor of a ligand for an olfactory receptor of interest, comprising:
adding a test substance to an olfactory receptor polypeptide expressed by the method according to any one of claims 1 to 8; and
measuring a response of the olfactory receptor polypeptide to the test substance.

13. A method for evaluating and/or selecting an enhancer of odor of a ligand for an olfactory receptor of interest, comprising:
adding a test substance and the ligand for an olfactory receptor of interest to an olfactory receptor polypeptide expressed by the method according to any one of claims 1 to 8; and
measuring a response of the olfactory receptor polypeptide to the ligand.

14. The method according to any one of claims 9 to 13, wherein the response of the olfactory receptor polypeptide is measured through intracellular cAMP level measurement by ELISA or reporter gene assay, calcium ion level measurement by calcium imaging or TGFα shedding assay, or potential change measurement inside and outside cell membranes by a two-electrode voltage clamp technique using *Xenopus* oocytes.

15. An altered olfactory receptor polypeptide consisting of
an amino acid sequence obtained by, in an amino acid sequence of an olfactory receptor of interest, altering at least one amino acid residue different from that in a consensus amino acid sequence to the amino acid residue at a position corresponding thereto in the consensus amino acid sequence, wherein
the consensus amino acid sequence is an amino acid sequence obtained by alignment of the amino acid sequence of the olfactory receptor of interest and amino acid sequences of any of the following olfactory receptors (a) to (d):
(a) at least 11 olfactory receptors selected from the group consisting of olfactory receptors encoded by orthologs of the olfactory receptor of interest in the same order as that of an organism species from which the olfactory receptor of interest is derived;
(b) at least 11 olfactory receptors selected from the group consisting of the olfactory receptors encoded by orthologs of the olfactory receptor of interest in the same order as that of an organism species from which the olfactory receptor of interest is derived and olfactory receptors encoded by paralogs of the olfactory receptor of interest, the 11 olfactory receptors including at least one of the olfactory receptor encoded by the paralog of the olfactory receptor of interest;
(c) at least 11 olfactory receptors selected from the group consisting of olfactory receptors encoded by orthologs of the olfactory receptor of interest in vertebrates, the 11 olfactory receptors including at least one olfactory receptor encoded by a vertebrate ortholog in an order different from the order of an organism species from which the olfactory receptor of interest is derived; and
(d) at least 11 olfactory receptors selected from the group consisting of the olfactory receptors encoded by orthologs of the olfactory receptor of interest in vertebrates and olfactory receptors encoded by orthologs in a vertebrate with a paralog having the highest homology to the olfactory receptor of interest among paralogs of the olfactory receptor of interest having 11 or more vertebrate orthologs, the 11 olfactory receptors including at least one olfactory receptor encoded by a vertebrate orthologs in an order different from the order of an organism species from which the olfactory receptor of interest is derived, and
an olfactory receptor encoded by the paralog.

16. The altered olfactory receptor polypeptide according to claim 15, wherein the consensus amino acid sequence is an amino acid sequence consisting of consensus residues identified in accordance with the following criteria (i) to (iii) from the alignment of the amino acid sequence of the olfactory receptor of interest and the amino acid sequences of any of the olfactory receptors (a) to (d):
(i) at each amino acid position of the alignment,
(i-i) when there exists one amino acid residue which is different from the amino acid residue of the olfactory receptor of interest and has a frequency of appearance of 50% or more, the amino acid residue is identified as a consensus residue,
(i-ii) when there exist two amino acid residues having a frequency of appearance of 50%, the amino acid residues of the olfactory receptor of interest are identified as a consensus residue,
(i-iii) when there exists an amino acid residue in the olfactory receptor of interest and there exists no amino acid residue having a frequency of appearance of 40% or more, the absence of a consensus residue is identified,
(i-iv) when there exists no amino acid residue in the olfactory receptor of interest and there exists an amino acid residue having a frequency of appearance of 60% or more, an amino acid residue having the highest frequency of appearance is identified as a consensus residue, and when there exist two or more amino acid residues having the highest frequency of appearance, an amino acid residue having the smallest molecular weight among the amino acid residues is identified as a consensus residue, and
(i-v) if none of the above (i-i) to (i-iv) is appropriate, the amino acid residue of the olfactory receptor of interest is identified as a consensus residue;
(ii) when the consensus residues are identified in accordance with the criterion (i) and when a consensus residue nearest to the N terminus is a consensus residue at a position corresponding to the N terminus of the olfactory receptor of interest or a C-terminal side thereof and is not a methionine residue, a consensus residue on an N-terminal side of a consensus residue consisting of a methionine residue positioned nearest to the N terminus is changed to the absence of a consensus residue; and
(iii) when the consensus residues are identified in accordance with the criterion (i) and when a consensus residue nearest to the N terminus is a consensus residue at a position corresponding to an N-terminal side of the N terminus of the olfactory receptor of interest and is not a methionine residue, an amino acid residue having the highest frequency of appearance is identified as a consensus residue until a methionine residue appears by tracing back one by one amino acid positions on an N-terminal side of the position of the consensus residue of the alignment, and when there exist two or more amino acid residues having the highest frequency of appearance, an amino acid residue having the smallest molecular weight among the amino acid residues is identified as a consensus residue.

17. The altered olfactory receptor polypeptide according to claim 15 or 16, wherein the alignment is alignment of the amino acid sequence of the olfactory receptor of interest and the olfactory receptors (c).

18. The altered olfactory receptor polypeptide according to any one of claims 15 to 17, wherein the olfactory receptor of interest is a human olfactory receptor.

19. The altered olfactory receptor polypeptide according to claim 15 or 16, wherein the olfactory receptor polypeptide consists of an amino acid sequence obtained by, in an amino acid sequence of sequence identification number (2) of an olfactory receptor (1) in the above Tables 1-1 to 1-5, altering at least one amino acid residue different from that in a consensus amino acid sequence of sequence identification number (3) to the amino acid residue at a position corresponding thereto in the consensus amino acid sequence.

20. The altered olfactory receptor polypeptide according to claim 19 which consists of an amino acid sequence of any of SEQ ID NOs: 97 to 209, 485 to 641, and 851 to 899.

21. An altered olfactory receptor polypeptide
consisting of an amino acid sequence obtained by, in an amino acid sequence of SEQ ID NO: 96 of a human olfactory receptor OR7E24, altering at least one amino acid residue different from that in a consensus amino acid sequence of SEQ ID NO: 210 to the amino acid residue at a position corresponding thereto in the consensus amino acid sequence,
consisting of an amino acid sequence obtained by, in an amino acid sequence of SEQ ID NO: 484 of a human olfactory receptor OR9K2, altering at least one amino acid residue different from that in a consensus amino acid sequence of SEQ ID NO: 642 to the amino acid residue at a position corresponding thereto in the consensus amino acid sequence, or
consisting of an amino acid sequence obtained by, in an amino acid sequence of SEQ ID NO: 850 of a human olfactory receptor OR5I1, altering at least one amino acid residue different from that in a consensus amino acid sequence of SEQ ID NO: 900 to the amino acid residue at a position corresponding thereto in the consensus amino acid sequence.

22. The altered olfactory receptor polypeptide according to claim 21 which consists of an amino acid sequence of SEQ ID NO: 210, 642, or 900.

23. A polynucleotide encoding the altered olfactory receptor polypeptide according to any one of claims 15 to 22.

24. A vector or a DNA fragment comprising the polynucleotide according to claim 23.

25. A transformed cell comprising the vector or the DNA fragment according to claim 24.
